(19) Europäisches Patentamt
European Patent Office
Office européen des brevets

(11) EP 2 507 341 B1

(12) # EUROPÄISCHE PATENTSCHRIFT

(45) Veröffentlichungstag und Bekanntmachung des Hinweises auf die Patenterteilung:
**29.10.2014 Patentblatt 2014/44**

(21) Anmeldenummer: **10778576.8**

(22) Anmeldetag: **16.11.2010**

(51) Int Cl.:
***C09K 19/12*** (2006.01)

(86) Internationale Anmeldenummer:
**PCT/EP2010/006961**

(87) Internationale Veröffentlichungsnummer:
**WO 2011/066905 (09.06.2011 Gazette 2011/23)**

(54) **BAUTEILE FÜR DIE HOCHFREQUENZTECHNIK, FLÜSSIGKRISTALLINE MEDIEN UND VERBINDUNGEN**

COMPONENTS FOR HIGH FREQUENCY TECHNOLOGY, LIQUID CRYSTAL MEDIA AND COMPOUNDS

COMPOSANTS POUR LA TECHNIQUE DES HAUTES FRÉQUENCES, MILIEUX DE CRISTAUX LIQUIDES ET COMPOSÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.12.2009 DE 102009056560**

(43) Veröffentlichungstag der Anmeldung:
**10.10.2012 Patentblatt 2012/41**

(73) Patentinhaber: **Merck Patent GmbH**
**64293 Darmstadt (DE)**

(72) Erfinder:
- **MANABE, Atsutata**
  **64625 Bensheim (DE)**
- **JASPER, Christian**
  **64283 Darmstadt (DE)**
- **RIEFFENRATH, Volker**
  **64380 Roßdorf (DE)**
- **MONTENEGRO, Elvira**
  **69469 Weinheim (DE)**
- **PAULUTH, Detlef**
  **64372 Ober-Ramstadt (DE)**

(56) Entgegenhaltungen:
**EP-A2- 0 364 185 DE-A1-102004 029 429**
**JP-A- 2005 120 208**

- **GALDA P ET AL: "A versatile palladium-catalyzed synthesis of n-alkyl-substituted oligo-p-phenyls", SYNTHESIS, GEORG THIEME VERLAG, STUTTGART, DE, Nr. 5, 1. Mai 1996 (1996-05-01), Seiten 614-620, XP002013346, ISSN: 0039-7881, DOI: DOI:10.1055/S-1996-4260 in der Anmeldung erwähnt**
- **SUBRAMANIAM G ET AL: "MESOGENIC PROPERTIES OF 2',3'''-DIMETHYL-P-SEXIPHENYL", MOLECULAR CRYSTALS AND LIQUID CRYSTALS(INC. NONLINEAR OPTICS ), GORDON AND BREACH SCIENCE PUBLISHERS, READING, GB, Bd. 166, 1. Januar 1989 (1989-01-01), Seiten 173-179, XP000007410,**
- **LARIOS-LOPEZ L ET AL: "OLIGO(P-PHENYLENE)S SUBSTITUTED WITH LONG ALKOXY CHAINS I. THERMOTROPIC LIQUID CRYSTALLINE PROPERTIES AND UV ABSORPTION/EMISSION CHARACTERISTICS", LIQUID CRYSTALS: AN INTERNATIONAL JOURNAL OF SCIENCE AND TECHNOLOGY, TAYLOR & FRANCIS, GB, Bd. 30, Nr. 4, 1. April 2003 (2003-04-01), Seiten 423-433, XP001145195, ISSN: 0267-8292, DOI: DOI: 10.1080/0267829031000083768 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 507 341 B1

**Beschreibung**

Gebiet der Erfindung

Die vorliegende Erfindung betrifft neue Bauteile für die Hochfrequenztechnik, speziell Bauteile für Hochfrequenzvorrichtungen, insbesondere Antennen, speziell für den Gigahertzbereich, die im Mikrowellen- oder im Millimeterwellenbereich betrieben werden. Diese Bauteile verwenden besondere flüssigkristalline, chemische Verbindungen oder damit zusammengestellte flüssigkristalline Medien beispielsweise zur Phasenschiebung von Mikrowellen für abstimmbare "phased-array" Antennen oder für abstimmbare Zellen von Mikrowellenantennen basierend auf "reflectarrays".

Stand der Technik und zu lösendes Problem

Subramaniam, G. and Gilpin, R. K. "Mesogenic Properties or 2',3""-dimethyl-p-sexiphenyl", MCLC Band 166 (1989) Seiten 173-179 offenbart Sexiphenyle mit lateralen Substituenten und ohne Endgruppen mit ihren mesogenen Eigenschaften.

EP 0 364 185 A2 beschäftigt sich mit halbleitenden Flüssigkristallen, die Oligophenyle enthalten können.

Flüssigkristalline Medien werden seit längerem in elektrooptischen Anzeigen (Liquid Crystal Displays - LCDs) genutzt, um Informationen anzuzeigen.

In neuerer Zeit werden flüssigkristalline Medien auch für die Verwendung in Komponenten, bzw, in Bauteilen, für die Hochfrequenztechnik, insbesondere für die Mikrowellentechnik vorgeschlagen, wie z.B. in DE 10 2004 029 429 A und in JP 2005-120208 (A).

Eine technisch wertvolle Anwendung der flüssigkristallinen Medien in der Hochfrequenztechnik beruht auf ihrer Eigenschaft, dass sie sich durch eine variable Spannung in ihren dielektrischen Eigenschaften steuern lassen, besonders für den Gigahertzbereich. Somit lassen sich abstimmbare Antennen konstruieren, die keine beweglichen Teile beinhalten

(Gaebler, A., Moessinger, A., Goelden, F., et al., "Liquid Crystal-Reconfigurable Antenna Concepts for Space Applications at Microwave and Millimeter Waves", International Journal of Antennas and Propagation, Band 2009, Artikel ID 876989, (2009), Seiten 1 - 7, DOI: 10.1155/2009/876989.

Penirschke, A., Müller, S., Scheele, P., Weil, C., Wittek, M., Hock, C. und Jakoby, R.: "Cavity Perturbation Method for Characterization of Liquid Crystals up to 35 GHz", 34th European Microwave Conference-Amsterdam, S. 545-548 beschreibt unter anderem die Eigenschaften der bekannten, flüssigkristallinen Einzelsubstanz K15 (Merck KGaA, Deutschland) bei einer Frequenz von 9 GHz.

DE 10 2004 029 429 A beschreibt die Anwendung von Flüssigkristallmedien in der Mikrowellentechnik unter anderem in Phasenschiebern. In DE 10 2004 029 429 A werden bereits flüssigkristalline Medien bezüglich ihrer Eigenschaften im entsprechenden Frequenzbereich untersucht.

Die bisher bekannten Zusammensetzungen sind jedoch mit Nachteilen behaftet. Die meisten von ihnen führen, neben anderen Mängeln, zu unvorteilhaft hohen Verlusten und/oder unzureichenden Phasenverschiebungen bzw. zu geringer Materialgüte ($\eta$).

Für die Anwendung in der Hochfrequenztechnik werden flüssigkristalline Medien mit besonderen, bislang eher ungewöhnlichen, ungebräuchlichen Eigenschaften, bzw. Kombinationen von Eigenschaften benötigt.

Somit sind neue flüssigkristalline Medien mit verbesserten Eigenschaften erforderlich. Insbesondere müssen der Verlust im Mikrowellenbereich verringert und die Materialgüte verbessert werden.

Außerdem besteht der Bedarf das Tieftemperaturverhalten der Bauteile zu verbessern. Hier sind sowohl eine Verbesserung der Betriebseigenschaften, wie auch der Lagerfähigkeit nötig.

Es besteht daher ein erheblicher Bedarf an flüssigkristallinen Medien mit geeigneten Eigenschaften für entsprechende praktische Anwendungen.

Larios-Lopez, L., Navarro-Rodriguez, D., Arias-Marin, E. M., Moggio, I. und Reyes-Casteneda, C. V. Liquid Crystals, 2003 Band 30, Nr. 4, Seiten 423-433 beschreibt Oligo-(*p*-phenyle) mit fünf und mit sieben Phenylringen, die terminal und in einigen lateralen Positionen mit Alkoxyresten substituiert sind. Diese Verbindungen sind alle hoch schmelzend.

Banerjee M., Shukla, R. und Rathore, R., J. Am. Chem. Soc. 2009, Band 131, Seiten 1780-1786, sowie die zugehörige "supporting information", beschreibt terminal substituierte Hexa- und Hepta-p-phenylene, die ebenfalls alle hoch schmelzende Verbindungen sind.

Lateral substituierte Dekaphenyle der Formel

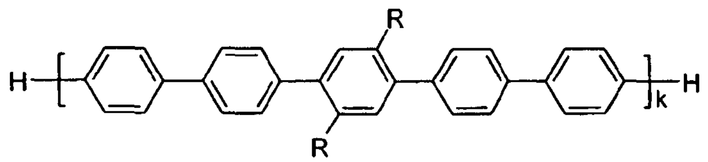

mit k = 1, 2 bzw. 3 und R = n-Hexyl bzw. n-Docdecyl werden in Rehahn, M. und Galda, P., Synthesis 1996, Seiten 614 bis 620 (DOI: 10.1055/s-1996-4260) offenbart.

Bistolanverbindungen, auch Triphenyldiacetylene genannt, mit einer zusätzlichen Alkylsubstitution am zentralen Phenylenring sind dem Fachmann hinlänglich bekannt.

Z.B. Wu, S.-T., Hsu, C.-S. and Shyu, K.-F., Appl. Phys. Lett., 74 (3), (1999), p. 344-346 offenbart verschiedene flüssigkristalline Bistolanverbindungen mit einer lateralen Methylgruppe der Formel

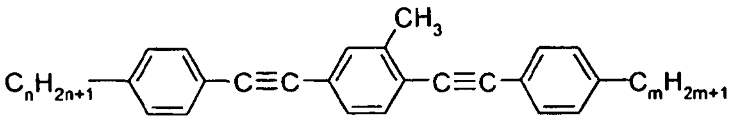

.

Hsu, C. S. Shyu, K. F., Chuang, Y. Y. and Wu, S.-T., Liq. Cryst., 27 (2), (2000), p. 283-287 offenbart, neben solchen flüssigkristallinen Bistolanverbindungen mit einer lateralen Methylgruppe, auch entsprechende Verbindungen mit einer lateralen Ethylgruppe und schlägt deren Verwendung u. a. in "liquid crystal optically phased arrays" vor.

In Dabrowski, R., Kula, P. Gauza, S., Dziadiszek, J. Urban, S. und Wu, S.-T., IDRC 08, (2008), Seiten 35- 38 werden dielektrisch neutrale Bistolanverbindungen mit und ohne lateraler Methylgruppe am mittleren Ring neben den stark dielektrisch positiven Isithiocyanatbistolanverbindungen der Formel

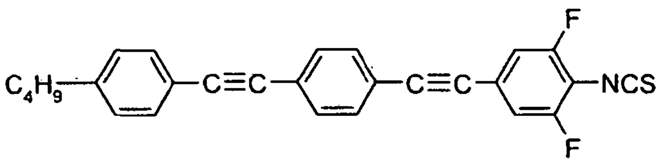

erwähnt.

A. Gaebler, F. Goelden, S. Müller, A. Penirschke und R. Jakoby "Direct Simulation of Material Permittivites using an Eigen-Susceptibility Formulation of the Vector Variational Approach", 12MTC 2009-International Instrumentation and Measurement Technology Conference, Singapur, 2009 (IEEE), S. 463-467 beschreibt die entsprechenden Eigenschaften der bekannten Flüssigkristallmischung E7 (ebenfalls Merck KGaA, Deutschland).

DE 10 2004 029 429 A beschreibt die Anwendung von Flüssigkristallmedien in der Mikrowellentechnik unter anderem in Phasenschiebern. In DE 10 2004 029 429 A werden bereits flüssigkristalline Medien bezüglich ihrer Eigenschaften im entsprechenden Frequenzbereich untersucht. Außerdem werden dort flüssigkristalline Medien, die Verbindungen der Formeln

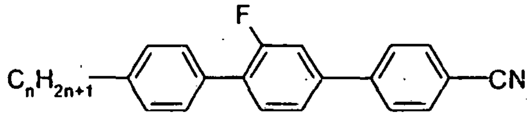

neben Verbindungen der Formeln

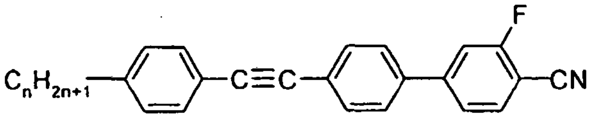

und

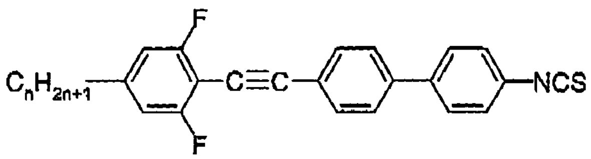

enthält.

Diese Zusammensetzungen sind jedoch mit gravierenden Nachteilen behaftet. Die meisten von ihnen führen, neben anderen Mängeln, zu unvorteilhaft hohen Verlusten und/oder zu geringer Materialgüte.

Für diese Anwendungen werden flüssigkristalline Medien mit besonderen, bislang eher ungewöhnlichen, ungebräuchlichen Eigenschaften, bzw. Kombinationen von Eigenschaften benötigt.

Somit sind neue flüssigkristalline Medien mit verbesserten Eigenschaften erforderlich. Insbesondere müssen der Verlust im Mikrowellenbereich verringert und die Materialgüte verbessert werden.

Außerdem besteht der Bedarf das Tieftemperaturverhalten der Bauteile zu verbessern. Hier sind sowohl eine Verbesserung der Betriebseigenschaften, wie auch der Lagerfähigkeit nötig.

Es besteht daher ein erheblicher Bedarf an flüssigkristallinen Medien mit geeigneten Eigenschaften für entsprechende praktische Anwendungen.

Vorliegende Erfindung

Überraschenderweise wurde nun gefunden, dass Bauteile für die Hochfrequenztechnik realisiert werden können, welche die Nachteile der Materialien des Standes der Technik nicht oder zumindest nur in erheblich geringerem Maße aufweisen, wenn ausgewählte, flüssigkristalline Verbindungen oder diese Verbindungen enthaltende Medien eingesetzt werden.

Gegenstand der vorliegenden Erfindung ist somit ein Bauteil für die Hochfrequenztechnik, bzw. für den Mikrowellenbereich und/oder den Millimeter-bereich des elektromagnetischen Spektrums, dadurch gekennzeichnet, dass es ein Flüssigkristallmedium enthält, das seinerseits eine *Komponente A,* enthält, die ihrerseits aus einer oder mehreren Verbindungen der Formel I

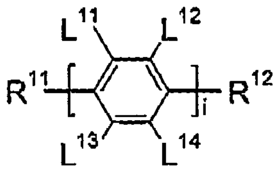

I

worin

$R^{11}$ und $R^{12}$ unabhängig voneinander Halogen, bevorzugt F oder Cl, unfluoriertes Alkyl oder fluoriertes Alkyl oder unfluoriertes Alkoxy oder fluoriertes Alkoxy jeweils mit 1 bis 15 C-Atomen oder unfluoriertes Alkenyl oder fluoriertes Alkenyl, unfluoriertes Alkenyloxy oder unfluoriertes Alkoxyalkyl oder fluoriertes Alkoxyalkyl jeweils mit 2 bis 15 C-Atomen, worin unabhängig voneinander auch eine oder mehrere "$-CH_2-$"-Gruppen durch Cycloalkyl mit 3 bis 6 C-Atomen, bevorzugt mit 4 oder 6 C-Atomen, ersetzt sein können, und alternativ auch einer von $R^{11}$ und $R^{12}$ oder $R^{11}$ und $R^{12}$ beide H,

bevorzugt

$R^{11}$ und $R^{12}$ unabhängig voneinander unfluoriertes Alkyl oder unfluoriertes Alkoxy jeweils mit 1 bis 7 C-Atomen oder unfluoriertes Alkenyl, unfluoriertes Alkenyloxy oder unfluoriertes Alkoxyalkyl jeweils mit 2 bis 7 C-Atomen,

besonders bevorzugt

$R^{11}$ unfluoriertes Alkyl mit 1 bis 7 C-Atomen oder unfluoriertes Alkenyl, unfluoriertes Alkenyloxy oder unfluoriertes Alkoxyalkyl jeweils mit 2 bis 7 C-Atomen, und

besonders bevorzugt

$R^{12}$ unfluoriertes Alkyl oder unfluoriertes Alkoxy jeweils mit 1 bis 7 C-Atomen, und

$L^{11}$ bis $L^{14}$ bei jedem Erscheinen, jeweils unabhängig voneinander H, Alkyl mit 1 bis 15 C-Atomen, F oder Cl und

i eine ganze Zahl im Bereich von 6 bis 15, bevorzugt von 6 oder 8 bis 12 und besonders bevorzugt von 6 oder 9 bis 10

bedeuten
und bevorzugt
mindestens zwei der vorhandenen Substituenten

$L^{11}$ bis $L^{14}$ eine von H verschiedene Bedeutung haben, und diese bevorzugt Alkyl bedeuten, und

$R^{11}$ $C_nH_{2n+1}$ oder $CH_2$=CH-$(CH_2)_z$, und

$R^{12}$ $C_mH_{2m+1}$ oder O-$C_mH_{2m+1}$ oder $(CH_2)_Z$-CH=$CH_2$,

bedeutet, und worin

n und m unabhängig voneinander eine ganze Zahl im Bereich von 0 bis 15, bevorzugt im Bereich von 1 bis 7 und besonders bevorzugt 1 bis 5 bedeuten, und

z 0, 1, 2, 3 oder 4, bevorzugt 0 oder 2, bedeutet, besteht.

Die Verbindungen der Formel I, bei denen $R^{11}$ und $R^{12}$ beide eine von H verschiedene Bedeutung haben, sind ebenfalls Gegenstand der vorliegenden Erfindung.
Bevorzugt sind Verbindungen der Formel I, bzw. werden Verbindungen der Formel I eingesetzt, bei denen im Fall i gleich 6 bis 8,

$L^{11}$ bis $L^{14}$ bei jedem Erscheinen, jeweils unabhängig voneinander H, Alkyl mit 1 bis 8 C-Atomen, besonders bevorzugt mit 2 bis 5 C-Atomen, F oder Cl und

bevorzugt mindestens zwei der vorhandenen Substituenten $L^{11}$ bis $L^{14}$ Alkyl bedeuten,
im Fall i gleich 9 bis 12,

$L^{11}$ bis $L^{14}$ bei jedem Erscheinen, jeweils unabhängig voneinander H, Alkyl mit 3 bis 10 C-Atomen, besonders bevorzugt mit 4 bis 8 C-Atomen, F oder Cl und

bevorzugt mindestens drei, besonders bevorzugt mindestens vier, der vorhandenen Substituenten $L^{11}$ bis $L^{14}$ Alkyl bedeuten,
im Fall i gleich 13 bis 15,

$L^{11}$ bis $L^{14}$ bei jedem Erscheinen, jeweils unabhängig voneinander H, Alkyl mit 5 bis 15 C-Atomen, besonders bevorzugt mit 6 bis 12 C-Atomen, F oder Cl und

i eine ganze Zahl im Bereich von 6 bis 15, bevorzugt von 6 oder 8 bis 12 und besonders bevorzugt von 6 oder 9 bis 10 und

bevorzugt mindestens vier, besonders bevorzugt mindestens sechs, der vorhandenen Substituenten $L^{11}$ bis $L^{14}$ Alkyl bedeuten.
Bevorzugt weist die *Komponente A* eine dielektrische Anisotropie im Bereich von mehr als -5,0 und von weniger als 10,0 aufweist und besteht aus Verbindungen mit acht oder mehr fünf- sechs- oder siebengliedrigen Ringen.
Besonders bevorzugt sind die Verbindungen der Formel I, die eine Flüssigkristallphase aufweisen.
In einer bevorzugten Ausführungsform der vorliegenden Erfindung enthält die *Komponente A* eine oder mehrere Verbindungen der Formel I, bevorzugt ausgewählt aus der Gruppe der Verbindungen der der Formeln IA und IB

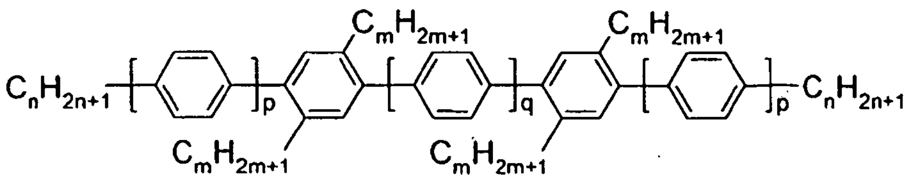

IA

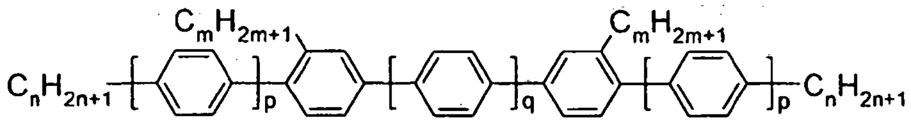

IB

worin

n und m voneinander unabhängig eine ganze Zahl von 1 bis 15, bevorzugt 3 bis 12,

p eine ganze Zahl von 1 bis 4, bevorzugt 2,

q eine ganze Zahl von 1 bis 6, bevorzugt 1 oder 4, und

(p + q) eine ganze Zahl von 4 bis 12, bevorzugt 4, 6 oder 8,

bedeuten.

In einer bevorzugten Ausführungsform der vorliegenden Erfindung enthält die *Komponente A* eine oder mehrere Verbindungen der Formel IA ausgewählt aus der Gruppe der Verbindungen der Formeln IA-1 bis IA-3

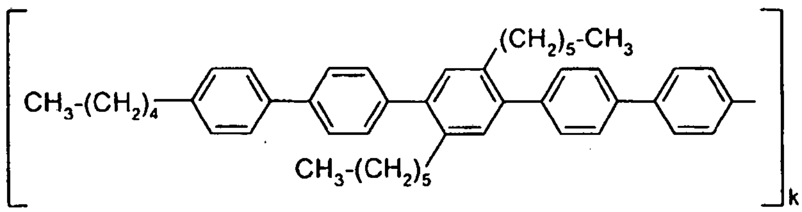

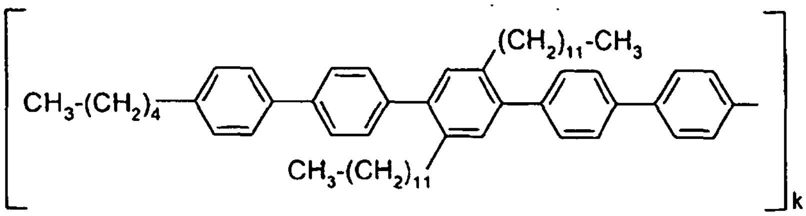

IA-2

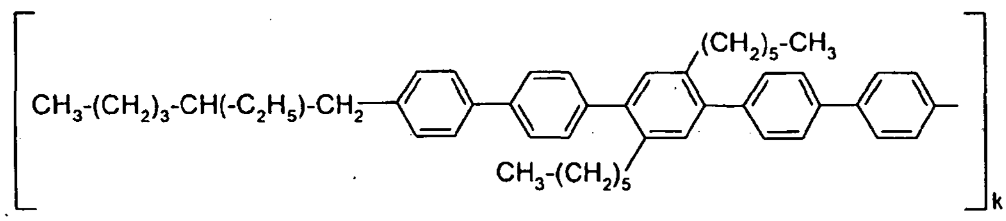

IA-3

worin

k 2

bedeutet.

In einer bevorzugten Ausführungsform der vorliegenden Erfindung enthält die *Komponente A* eine oder mehrere Verbindungen der Formel IB ausgewählt aus der Gruppe der Verbindungen der Formeln IB-1 und IB-2

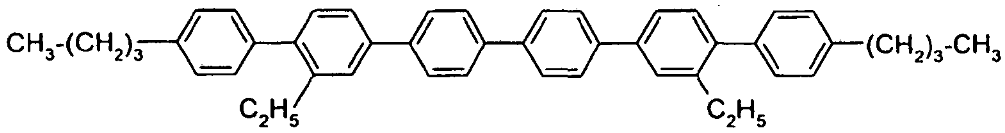

IB-1

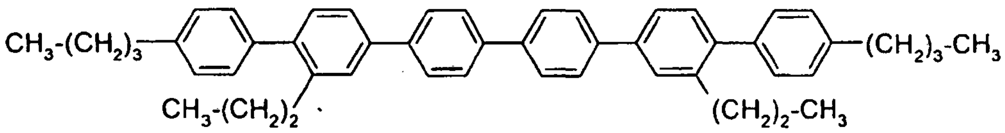

IB-2 .

In einer weiteren bevorzugten Ausführungsform der vorliegenden Erfindung enthält das Bauteil für die Hochfrequenztechnik ein flüssigkristallines Medium, das zusätzlich zu der *Komponente A* mindestens eine weitere Komponente, *Komponente B,* enthält, die ebenfalls bevorzugt eine dielektrische Anisotropie im Bereich von mehr als -5,0 und von weniger als 10,0 aufweist und aus einer oder mehreren Verbindungen der Formel IV

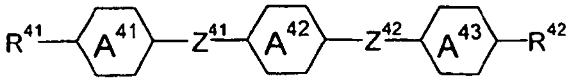

IV

worin

einer oder mehrere von

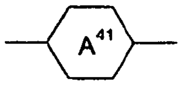 bis 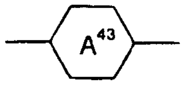

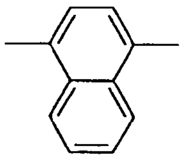 oder 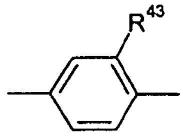 und die anderen

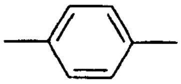 , und, bevorzugt

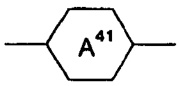 und 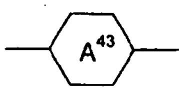 beide

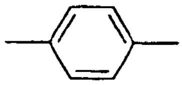 und

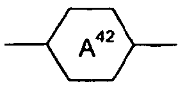 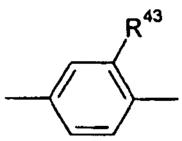 ,

$R^{41}$ bis $R^{43}$ unabhängig voneinander eine der für $R^{11}$ gegeben Bedeutungen haben, bevorzugt unfluoriertes Alkyl oder unfluoriertes Alkoxy jeweils mit 1 bis 15 C-Atomen oder unfluoriertes Alkenyl, unfluoriertes Alkenyloxy oder unfluoriertes Alkoxyalkyl jeweils mit 2 bis 15 C-Atomen,

bevorzugt

$R^{41}$ und $R^{42}$ unabhängig voneinander unfluoriertes Alkyl oder unfluoriertes Alkoxy jeweils mit 1 bis 7 C-Atomen oder unfluoriertes Alkenyl, unfluoriertes Alkenyloxy oder unfluoriertes Alkoxyalkyl jeweils mit 2 bis 7 C-Atomen,

besonders bevorzugt

$R^{41}$ unfluoriertes Alkyl mit 1 bis 7 C-Atomen oder unfluoriertes Alkenyl, unfluoriertes Alkenyloxy oder unfluoriertes Alkoxyalkyl jeweils mit 2 bis 7 C-Atomen, und

besonders bevorzugt

$R^{42}$ unfluoriertes Alkyl oder unfluoriertes Alkoxy jeweils mit 1 bis 7 C-Atomen, und

bevorzugt

$R^{43}$ unfluoriertes Alkyl mit 1 bis 5 C-Atomen, unfluoriertes Cyclohexyl mit 3 bis 7 C-Atomen, unfluoriertes Alkylcyclohexyl oder unfluoriertes Cyclohexylalkyl jeweils mit 4 bis 12 C-Atomen oder unfluoriertes Alkylcyclohexylalkyl mit 5 bis 15 C-Atomen, bevorzugt n-Alkyl, besonders bevorzugt Methyl, Ethyl oder n-Propyl,

$Z^{41}$ und $Z^{42}$ unabhängig voneinander -C≡C-, -CF=CF-, -CF=CH-, -CH=CF oder -CH=CH-, bevorzugt -C≡C- oder

-CF=CF-,

bevorzugt $Z^{41}$ und $Z^{42}$ beide -C≡C-,
bedeuten, besteht.

Gemäß einer weiteren bevorzugten Ausführungsform der vorliegenden Erfindung enthält das Bauteil für die Hochfrequenztechnik ein flüssigkristallines Medium, enthaltend

- eine erste Komponente, *Komponente A,* die aus einer oder mehreren Verbindungen der oben gegeben Formel I besteht und
- eine oder mehrere weitere Komponenten ausgewählt aus der Gruppe der im Folgenden definierten *Komponenten B* bis *E*:
  - *Komponente B*, die bevorzugt eine dielektrische Anisotropie im Bereich von mehr als -5,0 und von weniger als 10,0 aufweist und bevorzugt aus Verbindungen der oben gegebenen Formel IV besteht,
  - eine stark dielektrisch positive Komponente, *Komponente C*, die eine dielektrische Anisotropie von 10,0 oder mehr aufweist,
  - eine stark dielektrisch negative Komponente, *Komponente D*, die eine dielektrische Anisotropie von -5,0 oder weniger aufweist,
  - eine weitere Komponente, *Komponente E*, die ebenfalls eine dielektrische Anisotropie im Bereich von mehr als -5,0 und von weniger als 10,0 aufweist und aus Verbindungen mit bis zu fünf fünf-, sechs- oder siebengliedrigen Ringen besteht.

Typische Beispiele für fünfgliedrige Ringe sind

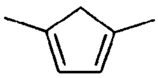 , 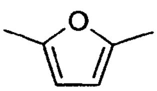 , 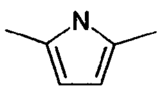 ,

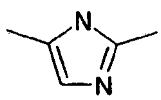

und andere.

Typische Beispiele für sechsgliedrige Ringe sind

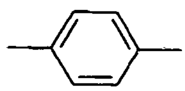 , 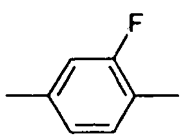 , 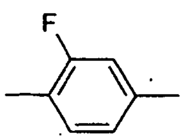 ,

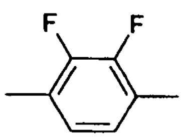 , 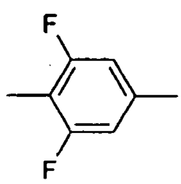 , 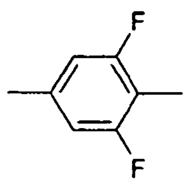 ,

und

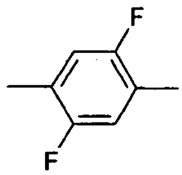

.

Typische Beispiele für siebengliedrige Ringe sind

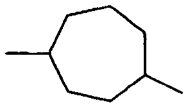 und 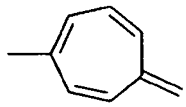 .

Die fünf-, sechs- und siebengliedrigen Ringe umfassen auch gesättigte, sowie teilgesättigte Ringe, ebenso wie heterocyclische Ringe.

Im Sinne der vorliegenden Anmeldung werden kondensierte Ringsysteme, die aus zwei dieser Ringe, das heißt z. B. aus zwei fünfgliedrigen, einem fünfgliedrigen Ring und einem sechsgliedrigen Ring, fünfgliedrigen Ring und einem siebengliedrigen Ring oder aus zwei sechsgliedrigen Ringen, bestehen, wie z. B.

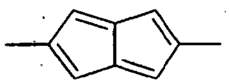 , 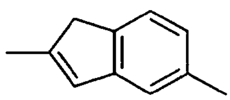 , 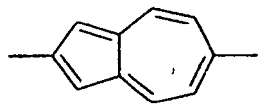 ,

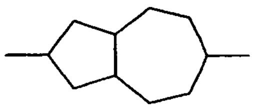 und 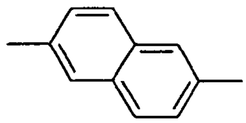

bei der Zuordnung der Verbindungen zu den *Komponenten A* bzw. *E* als einer dieser fünf- bzw. sechs- bzw. siebengliedrigen Ringe gezählt.

Entsprechend werden kondensierte Ringsysteme, die aus einer Kombination von dreien oder mehreren dieser Ringe bestehen, die in Längsrichtung im Molekül eingebaut sind, wie z. B.

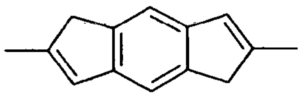 , 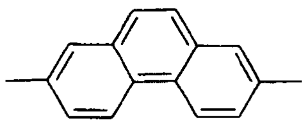 ,

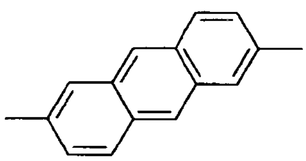 , 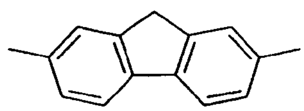

und

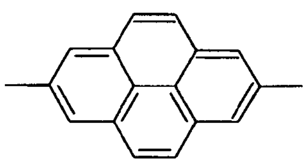

als zwei dieser fünf-, sechs- oder siebengliedrigen Ringe gezählt.

Im Gegensatz dazu, werden kondensierte Ringsysteme, die in Querrichtung im Molekül eingebaut sind, wie z. B.

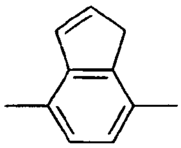,
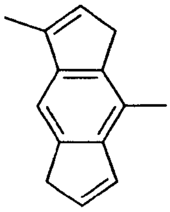,
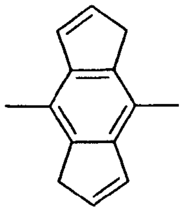,

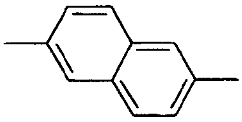
(s.o.),
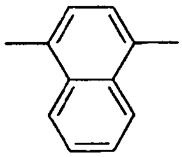,
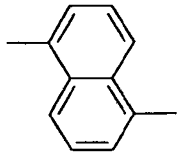,

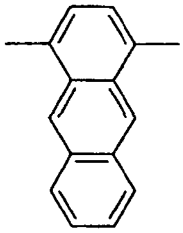,
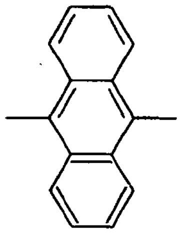,
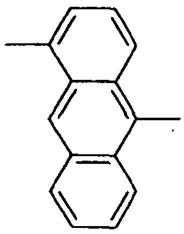,

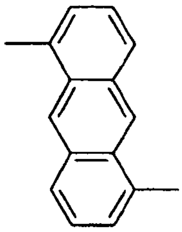,
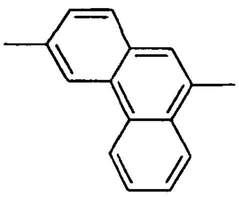,
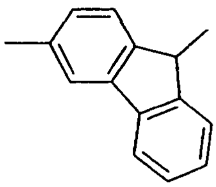,

und

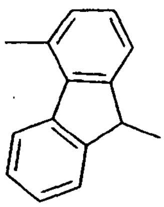

als einer dieser fünf-, sechs- oder siebengliedrigen Ringe gezählt.

Gegenstand der vorliegenden Erfindung sind ebenfalls die direkt vorhergehend, sowie weiter unten beschriebenen, flüssigkristallinen Medien, sowie deren Verwendung in elektro-optischen Anzeigen und in Bauteilen für die Hochfrequenztechnik.

In einer bevorzugten Ausführungsform der vorliegenden Erfindung enthält das Flüssigkristallmedium eine oder mehrere Verbindungen der Formeln IV, bevorzugt der Formel IVA

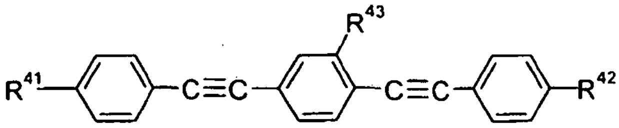

IVA

worin die Parameter die oben angegebene Bedeutung haben.

Besonders bevorzugt sind die Verbindungen der Formel IVA ausgewählt aus der Gruppe der Verbindungen der Formeln IVA-1 bis IVA-3, bevorzugt der Formeln IVA-1 und/oder IVA-2 und/oder IVA-3, bevorzugt der Formeln IVA-1 und IVA-2, stärker bevorzugt bestehen diese Verbindungen der Formel IV überwiegend, noch stärker bevorzugt bestehen sie im Wesentlichen und ganz besonders bevorzugt bestehen sie vollständig daraus:

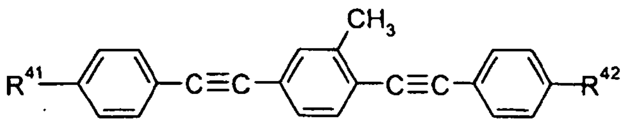

IVA-1

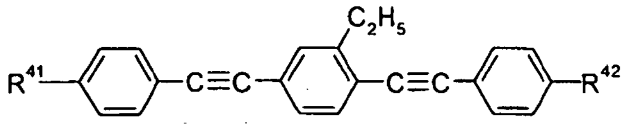

IVA-2

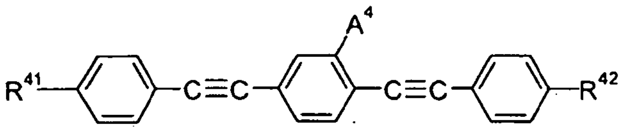

IVA-3

worin

$A^4$ Cycloalkyl mit 3 bis 6 C-Atomen, bevorzugt Cyclopropyl, Cyclobutyl oder Cyclohexyl, besonders bevorzugt Cyclopropyl oder Cyclohexyl und ganz besonders bevorzugt Cyclopropyl,

bedeutet und die anderen Parameter die jeweiligen oben bei Formel IV angegebenen Bedeutungen haben und bevorzugt

$R^{41}$ unfluoriertes Alkyl mit 1 bis 7 C-Atomen, und

$R^{42}$ unfluoriertes Alkyl mit 1 bis 7 C-Atomen oder unfluoriertes Alkoxy mit 1 bis 7 C-Atomen,

bedeutet.

Bevorzugt enthalten diese erfindungsgemäßen Medien eine *Komponente* C und keine *Komponente D* oder umgekehrt.

Bevorzugt enthalten diese erfindungsgemäßen Medien neben der *Komponente A* eine Komponente aus gewählt aus den beiden *Komponenten C* und *D* und gegebenenfalls zusätzlich die *Komponente B* und/oder die *Komponente E.*

Bevorzugt enthalten diese erfindungsgemäßen Medien zwei, drei oder vier, besonders bevorzugt zwei oder drei Komponenten ausgewählt aus der Gruppe der *Komponenten A* bis *E.* Bevorzugt enthalten diese Medien

- *Komponente A* und *Komponente B,* oder
- *Komponente A* und *Komponente C,* oder
- *Komponente A, Komponente B* und *Komponente C,* oder
- *Komponente A*, *Komponente B* und/oder *Komponente C* und *Komponente E*, oder
- *Komponente A* und *Komponente D*, oder
- *Komponente A, Komponente B* und *Komponente D,* oder
- *Komponente A*, *Komponente B* und/oder *Komponente D* und *Komponente E.*

Bevorzugt hat die stark dielektrisch positive Komponente, *Komponente C*, eine dielektrische Anisotropie von 20,0 oder mehr, stärker bevorzugt von 25,0 oder mehr, besonders bevorzugt von 30,0 oder mehr und ganz besonders

bevorzugt von 40,0 oder mehr.

Bevorzugt hat die stark dielektrisch negative Komponente, *Komponente D,* eine dielektrische Anisotropie von -7,0 oder weniger, stärker bevorzugt von -8,0 oder weniger, besonders bevorzugt von -10,0 oder weniger und ganz besonders bevorzugt von -15,0 oder weniger.

In einer bevorzugten Ausführungsform der vorliegenden Erfindung enthält die *Komponente C* eine oder mehrere Verbindungen ausgewählt aus der Gruppe der Verbindungen der Formeln IIA bis IID

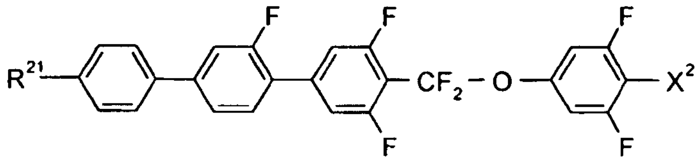

IIA

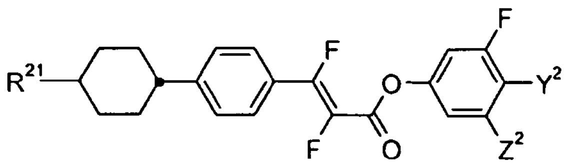

IIB

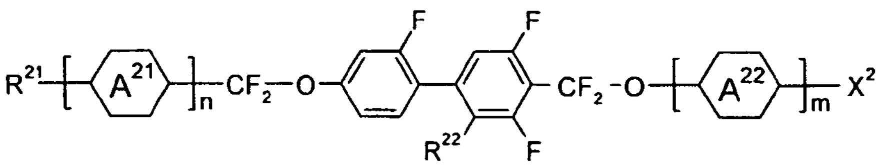

IIC

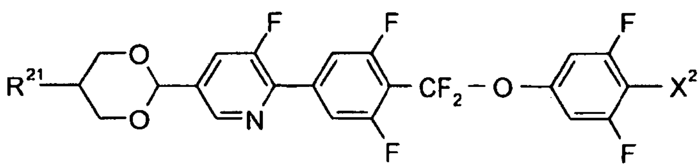

IID

$R^{21}$ unfluoriertes Alkyl oder unfluoriertes Alkoxy jeweils mit 1 bis 15 C-Atomen oder unfluoriertes Alkenyl, unfluoriertes Alkenyloxy oder unfluoriertes Alkoxyalkyl jeweils mit 2 bis 15 C-Atomen, bevorzugt Alkyl, besonders bevorzugt n-Alkyl,

$R^{22}$ H, unfluoriertes Alkyl oder unfluoriertes Alkoxy jeweils mit 1 bis 5, bevorzugt mit 1 bis 3, besonders bevorzugt mit 3 C-Atomen,

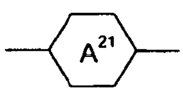 bis 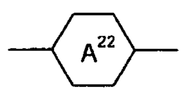

unabhängig voneinander und bei mehrfachem Auftreten auch diese untereinander jeweils unabhängig voneinander

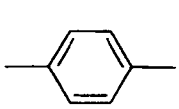 , 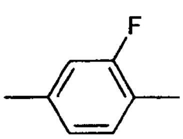 , 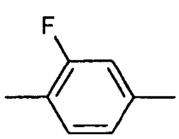 ,

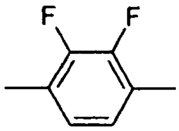 , 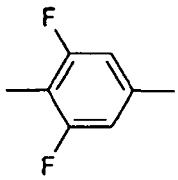 , 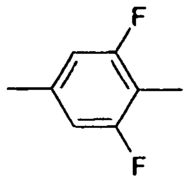 ,

oder 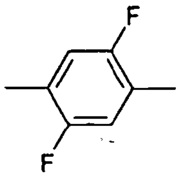 ,

vorzugsweise

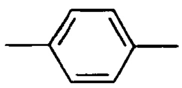 , 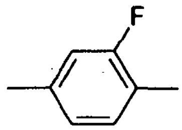

oder

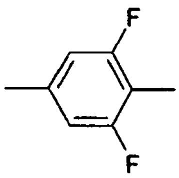 ,

bedeuten,

n und m unabhängig voneinander 1 oder 2, bevorzugt

(n + m) 3 oder 4 und, besonders bevorzugt

n 2,

$X^2$ F, Cl, $-CF_3$ oder $-OCF_3$, bevorzugt F oder Cl, besonders bevorzugt F,

$Y^2$ F, Cl, $-CF_3$, $-OCF_3$, oder CN, bevorzugt CN, und

$Z^2$ H oder F,

bedeuten.

Bevorzugte Verbindungen der Formel IIA sind die Verbindungen der entsprechenden Unterformel IIA-1

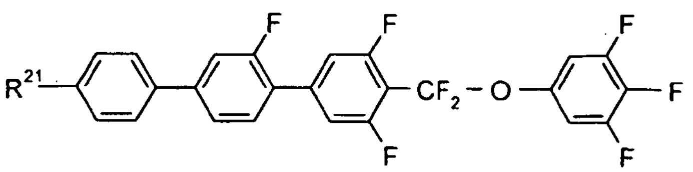

IIA-1

worin $R^{21}$ die oben gegebene Bedeutung hat.

Bevorzugte Verbindungen der Formet IIB sind die Verbindungen der entsprechenden Unterformeln IIB-1 und IIB-2

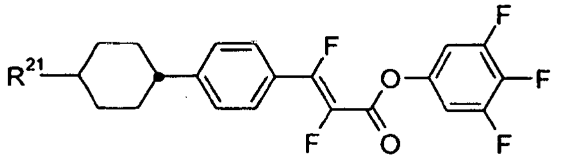

IIB-1

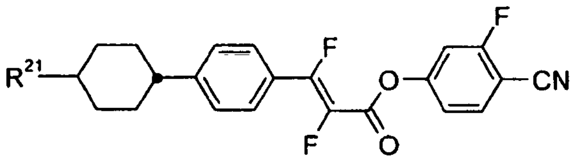

IIB-2

worin $R^{21}$ die oben gegebene Bedeutung hat.

Bevorzugte Verbindungen der Formel IIC sind die Verbindungen der entsprechenden Unterformeln IIC-1 und IIC-2

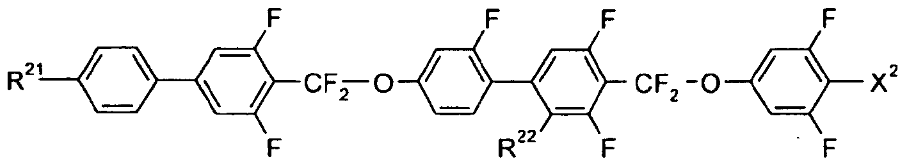

IIC-1

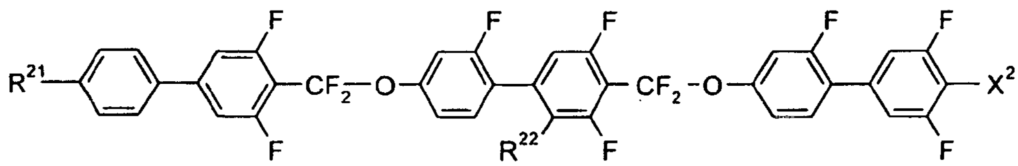

IIC-2

worin $R^{21}$ , $R^{22}$ und $X^2$ die jeweiligen oben gegebenen Bedeutungen haben.

Bevorzugte Verbindungen der Formel IID sind die Verbindungen der entsprechenden Unterformel IID-1

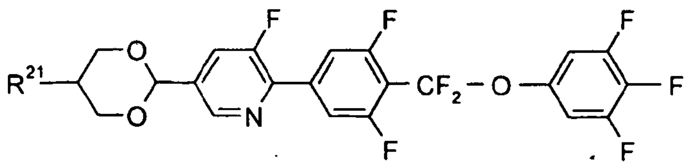

IID-1

worin $R^{21}$ die jeweilige oben gegebene Bedeutung hat.

In einer bevorzugten Ausführungsform der vorliegenden Erfindung enthält die *Komponente D* eine oder mehrere Verbindungen ausgewählt aus der Gruppe der Verbindungen der Formeln IIIA und IIIB:

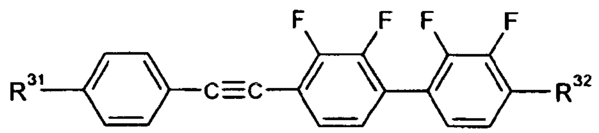

IIIA

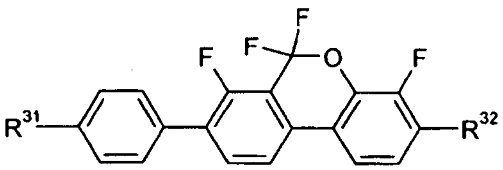

IIIB

worin

$R^{31}$ und $R^{32}$ unabhängig voneinander die oben bei Formel IIA für $R^{21}$ die oben angegebene Bedeutung haben

und bevorzugt

$R^{31}$ $C_nH_{2n+1}$ oder $CH_2$=CH-$(CH_2)_z$ und

$R^{32}$ $C_mH_{2m+1}$ oder O-$C_mH_{2m+1}$ oder $(CH_2)_Z$-CH=$CH_2$

bedeutet, und worin

n und m unabhängig voneinander eine ganze Zahl im Bereich von 0 bis 15, bevorzugt im Bereich von 1 bis 7 und besonders bevorzugt 1 bis 5 bedeuten, und

z 0, 1, 2, 3 oder 4, bevorzugt 0 oder 2, bedeutet.

Die bevorzugten Kombinationen von ($R^{31}$ und $R^{32}$) sind hier insbesondere ($C_nH_{2n+1}$ und $C_mH_{2m+1}$) und ($C_nH_{2n+1}$ und O-$C_mH_{2m+1}$).

Bevorzugte Verbindungen der Formel IIIB sind die Verbindungen der Unterformeln IIIB-1 und IIIB-2

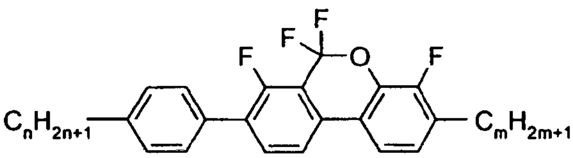

IIIB-1

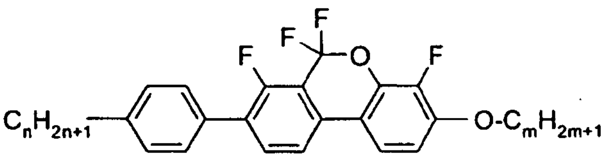

IIIB-2

worin

n und m jeweils die oben bei Formel IIIB gegebenen Bedeutungen haben und bevorzugt unabhängig voneinander eine ganze Zahl im Bereich von 1 bis 7,

bedeuten.

In einer bevorzugten Ausführungsform der vorliegenden Anmeldung enthält das Flüssigkristallmedium zusätzlich eine weitere Komponente, *Komponente E*, die bevorzugt aus einer oder mehreren Verbindungen ausgewählt aus der Gruppe der Verbindungen der Formeln V bis IX besteht

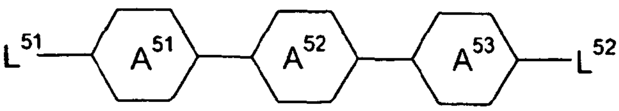

V

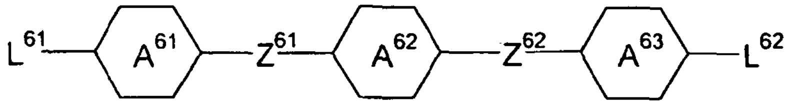 VI

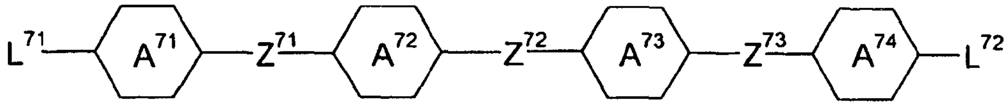 VII

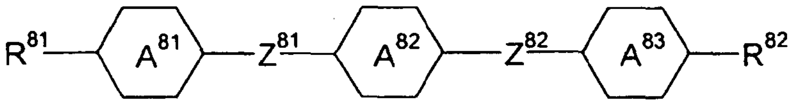 VIII

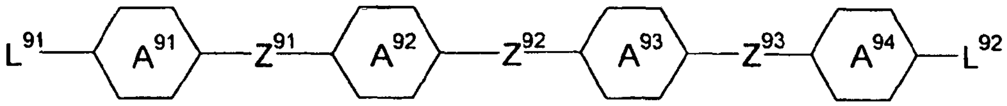 IX

worin

| | |
|---|---|
| $L^{51}$ | $R^{51}$ oder $X^{51}$, |
| $L^{52}$ | $R^{52}$ oder $X^{52}$, |
| $R^{51}$ und $R^{52}$ | unabhängig voneinander H, unfluoriertes Alkyl oder unfluoriertes Alkoxy mit 1 bis 17, bevorzugt mit 3 bis 10, C-Atomen oder unfluoriertes Alkenyl, unfluoriertes Alkenyloxy, oder unfluoriertes Alkoxyalkyl mit 2 bis 15, bevorzugt 3 bis 10, C-Atomen, vorzugsweise Alkyl oder unfluoriertes Alkenyl, bedeuten, |
| $X^{51}$ und $X^{52}$ | unabhängig voneinander H, F, Cl, -CN, -NCS, $-SF_5$, fluoriertes Alkyl oder fluoriertes Alkoxy mit 1 bis 7 C-Atomen oder fluoriertes Alkenyl, unfluoriertes oder Alkenyloxy, oder unfluoriertes oder Alkoxyalkyl mit 2 bis 7 C-Atomen, vorzugsweise fluoriertes Alkoxy, fluoriertes Alkenyloxy, F oder Cl bedeuten, und |

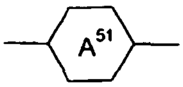 bis 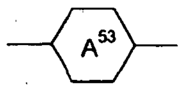

unabhängig voneinander

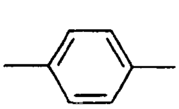 , 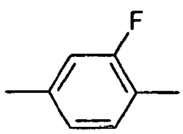 , 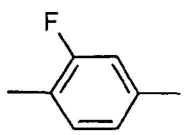

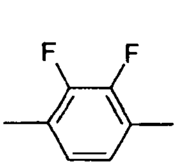 , 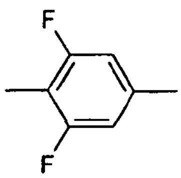 , 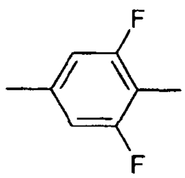 ,

oder 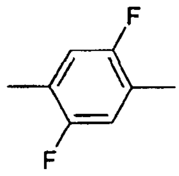 ,

vorzugsweise

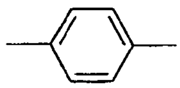 , 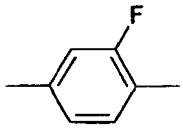 , 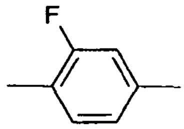 ,

oder 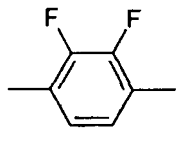 ,

bedeuten,

$L^{61}$ $R^{61}$ und, im Fall, dass $Z^{61}$ und/oder $Z^{62}$ *trans-* -CH=CH-oder *trans-* -CF=CF-, bedeutet, alternativ $X^{61}$, bedeutet,

$L^{62}$ $R^{62}$ und, im Fall, dass $Z^{61}$ und/oder $Z^{62}$ *trans-* -CH=CH-oder *trans-* -CF=CF-, bedeutet, alternativ $X^{62}$, bedeutet,

$R^{61}$ und $R^{62}$ unabhängig voneinander H, unfluoriertes Alkyl oder unfluoriertes Alkoxy mit 1 bis 17, bevorzugt mit 3 bis 10, C-Atomen oder unfluoriertes Alkenyl, unfluoriertes Alkenyloxy, oder unfluoriertes Alkoxyalkyl mit 2 bis 15, bevorzugt 3 bis 10, C-Atomen, vorzugsweise Alkyl oder unfluoriertes Alkenyl, bedeuten,

$X^{61}$ und $X^{62}$ unabhängig voneinander F oder Cl, -CN, -NCS, $-SF_5$, fluoriertes Alkyl oder Alkoxy mit 1 bis 7 C-Atomen oder fluoriertes Alkenyl, Alkenyloxy oder Alkoxyalkyl mit 2 bis 7 C-Atomen, oder -NCS, vorzugsweise -NCS, bedeuten,

einer von

$Z^{61}$ und $Z^{62}$ *trans-* -CH=CH-, *trans-* -CF=CF- oder -C≡C- bedeutet und der andere unabhängig davon *trans-* -CH=CH-, *trans-* -CF=CF- oder eine Einfachbindung bedeutet, vorzugsweise einer von ihnen -C≡C- öder *trans-* -CH=CH- und der andere eine Einfachbindung bedeutet, und

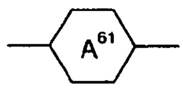 bis 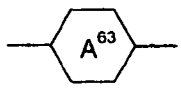

unabhängig voneinander

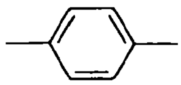, 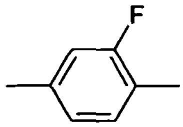, 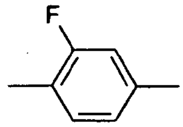,

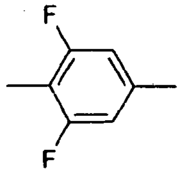, 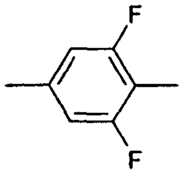, 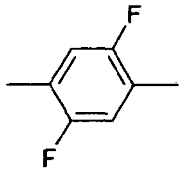,

oder 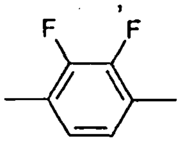, vorzugsweise

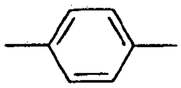, 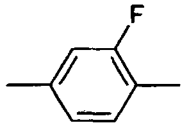, 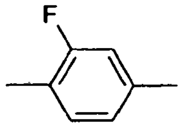,

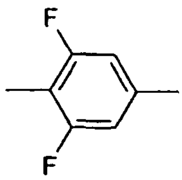, 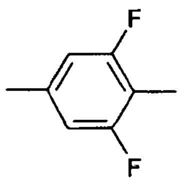,

oder

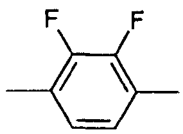

bedeuten,

$L^{71}$ $R^{71}$ oder $X^{71}$,

$L^{72}$ $R^{72}$ oder $X^{72}$,

$R^{71}$ und $R^{72}$ unabhängig voneinander H, unfluoriertes Alkyl oder unfluoriertes Alkoxy mit 1 bis 17, bevorzugt mit 3 bis 10, C-Atomen oder unfluoriertes Alkenyl, unfluoriertes Alkenyloxy, oder unfluoriertes Alkoxyalkyl mit 2 bis 15, bevorzugt 3 bis 10, C-Atomen, vorzugsweise Alkyl oder unfluoriertes Alkenyl bedeuten,

$X^{71}$ und $X^{72}$ unabhängig voneinander H, F, Cl, -CN, -NCS, $-SF_5$, fluoriertes Alkyl oder fluoriertes Alkoxy mit 1 bis 7 C-Atomen oder fluoriertes Alkenyl, unfluoriertes oder Alkenyloxy, oder unfluoriertes oder Alkoxyalkyl mit 2 bis 7 C-Atomen, vorzugsweise fluoriertes Alkoxy, fluoriertes Alkenyloxy, F oder Cl bedeuten, und

$Z^{71}$ bis $Z^{73}$ unabhängig voneinander *trans*- -CH=CH-, *trans*--CF=CF-, -C=C- oder eine Einfachbindung bedeuten, vorzugsweise eines oder mehrere von ihnen eine Einfachbindung bedeutet, besonders bevorzugt alle eine Einfachbindung bedeuten, und

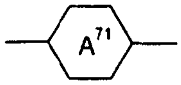 bis 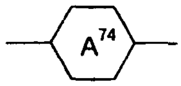

unabhängig voneinander

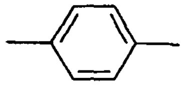, 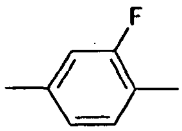, 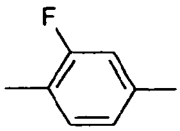,

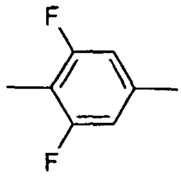, 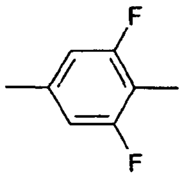, 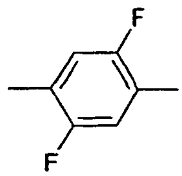,

oder 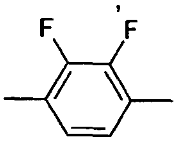, vorzugsweise

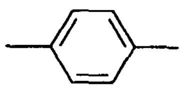, 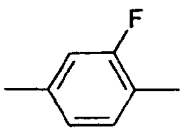, 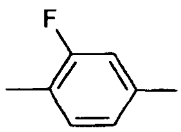,

oder 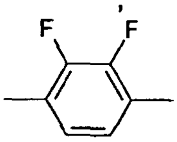, bedeuten,

$R^{81}$ und $R^{82}$ unabhängig voneinander H, unfluoriertes Alkyl oder Alkoxy mit 1 bis 15, bevorzugt 3 bis 10, C-Atomen oder unfluoriertes Alkenyl, Alkenyloxy oder Alkoxyalkyl mit 2 bis 15, bevorzugt 3 bis 10, C-Atomen, vorzugsweise unfluoriertes Alkyl oder Alkenyl bedeuten,

einer von

$Z^{81}$ und $Z^{82}$ *trans*--CH=CH-, *trans*--CF=CF- oder -C=C- bedeutet und der andere unabhängig davon *trans*--CH=CH-, *trans*- -CF=CF- oder eine Einfachbindung bedeutet, vorzugsweise einer von ihnen -C=C- oder *trans*--CH=CH- und der andere eine Einfachbindung bedeutet, und

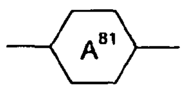 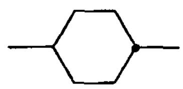

bedeutet,

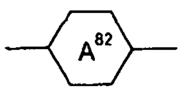 und 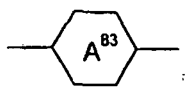

unabhängig voneinander

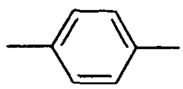, 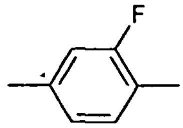, 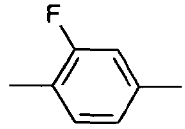,

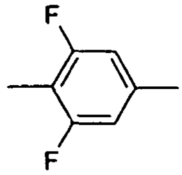, 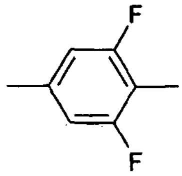, 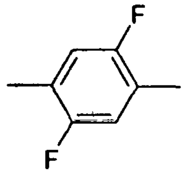,

oder

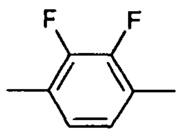

bedeuten,

L91 $R^{91}$ oder $X^{91}$ bedeutet,

L92 $R^{92}$ oder $X^{92}$ bedeutet,

$R^{91}$ und $R^{92}$ unabhängig voneinander H, unfluoriertes Alkyl oder Alkoxy mit 1 bis 15, bevorzugt 3 bis 10, C-Atomen oder unfluoriertes Alkenyl, Alkenyloxy oder Alkoxyalkyl mit 2 bis 15, bevorzugt 3 bis 10, C-Atomen, vorzugsweise unfluoriertes Alkyl oder Alkenyl bedeuten,

$X^{91}$ und $X^{92}$ unabhängig voneinander H, F, Cl, -CN, -NCS, $-SF_5$, fluoriertes Alkyl oder fluoriertes Alkoxy mit 1 bis 7 C-Atomen oder fluoriertes Alkenyl, unfluoriertes oder Alkenyloxy, oder unfluoriertes oder Alkoxyalkyl mit 2 bis 7 C-Atomen, vorzugsweise fluoriertes Alkoxy, fluoriertes Alkenyloxy, F oder Cl bedeuten, und

$Z^{91}$ bis $Z^{93}$ unabhängig voneinander *trans*- -CH=CH-, *trans*--CF=CF-, -C≡C- oder eine Einfachbindung bedeuten bevorzugt einer oder mehrere von ihnen eine Einfachbindung bedeutet, und besonders bevorzugt alle eine Einfachbindung, bedeuten,

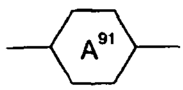 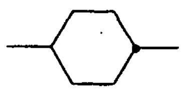, 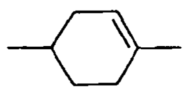, 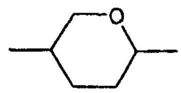

oder

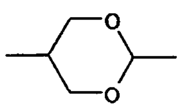

bedeutet,

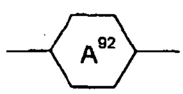 bis 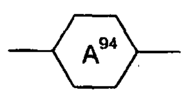

unabhängig voneinander

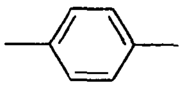, 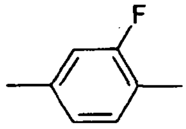, 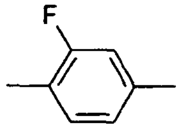,

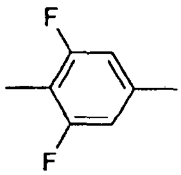, 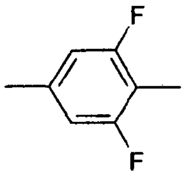, 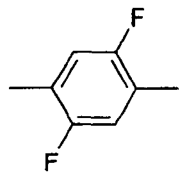

oder

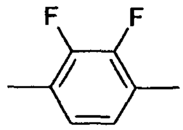

bedeuten,

und, wobei Verbindungen der Formel IIIA von den Verbindungen der Formel VI ausgeschlossen sind.

In einer bevorzugten Ausführungsform der vorliegenden Erfindung enthält das Flüssigkristallmedium eine oder mehrere Verbindungen der Formeln V, bevorzugt ausgewählt aus der Gruppe der Verbindungen der Formeln V-1 bis V-3, bevorzugt der Formeln V-1 und/oder V-2 und/oder V-3, bevorzugt der Formeln V-1 und V-2, stärker bevorzugt bestehen diese Verbindungen der Formel V überwiegend, noch stärker bevorzugt bestehen sie im Wesentlichen und ganz besonders bevorzugt bestehen sie vollständig daraus:

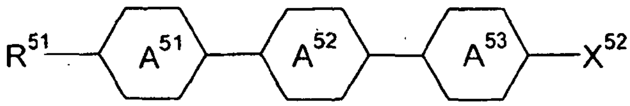 V-1

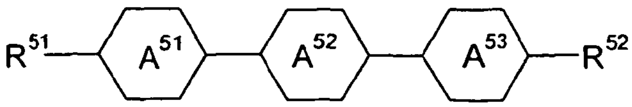 V-2

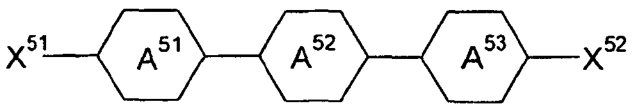 V-3

worin die Parameter die jeweiligen oben bei Formel V angegebenen Bedeutungen haben und bevorzugt

| | |
|---|---|
| $R^{51}$ | unfluoriertes Alkyl mit 1 bis 7 C-Atomen oder unfluoriertes Alkenyl mit 2 bis 7 C-Atomen, |
| $R^{52}$ | unfluoriertes Alkyl mit 1 bis 7 C-Atomen oder unfluoriertes Alkenyl mit 2 bis 7 C-Atomen, oder unfluoriertes Alkoxy mit 1 bis 7 C-Atomen, |
| $X^{51}$ und $X^{52}$ | unabhängig voneinander F, Cl, $-OCF_3$, $-CF_3$, -CN, -NCS oder $-SF_5$, bevorzugt F, Cl, $-OCF_3$, oder -CN, bedeuten. |

Bevorzugt werden die Verbindungen der Formeln V-1 ausgewählt aus der Gruppe der Verbindungen der Formeln V-1 a bis V-1d, stärker bevorzugt bestehen diese Verbindungen der Formel V überwiegend, noch stärker bevorzugt bestehen sie im Wesentlichen und ganz besonders bevorzugt bestehen sie vollständig daraus:

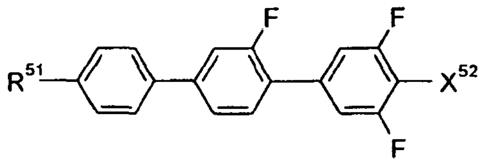

V-1a

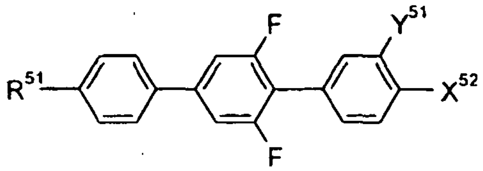

V-1b

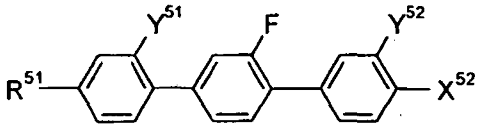

V-1c

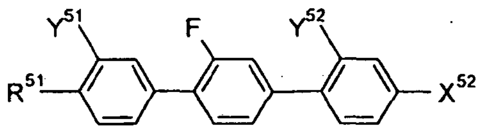

V-1d

worin die Parameter die jeweiligen oben bei Formel V-1 angegebenen Bedeutungen haben und worin

$Y^{51}$ und $Y^{52}$ jeweils unabhängig voneinander H oder F bedeuten, und bevorzugt

$R^{51}$ Alkyl oder Alkenyl, und

$X^{51}$ F, Cl oder $-OCF_3$, bedeuten.

Bevorzugt werden die Verbindungen der Formeln V-2 ausgewählt aus der Gruppe der Verbindungen der Formeln V-2a bis V-2e und/oder aus der Gruppe der Verbindungen der Formeln V-2f und V-2g, stärker bevorzugt bestehen diese Verbindungen der Formel V überwiegend, noch stärker bevorzugt bestehen sie im Wesentlichen und ganz besonders bevorzugt bestehen sie vollständig daraus:

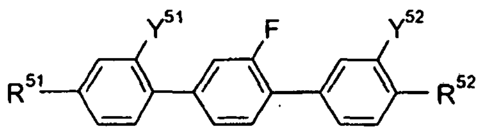

V-2a

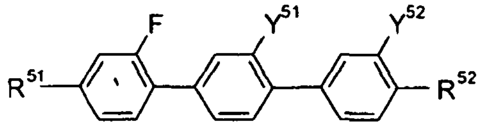

V-2b

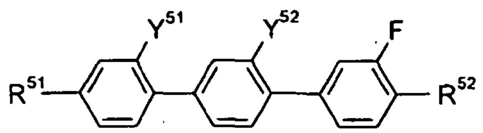

V-2c

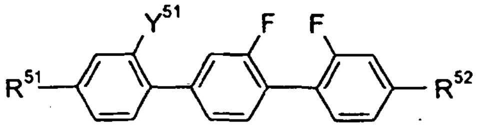

V-2d

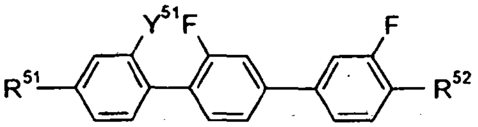

V-2e

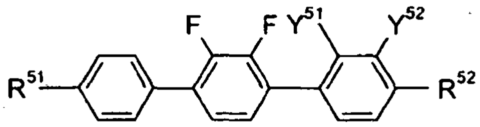

V-2f

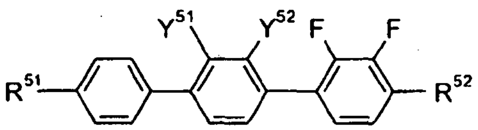

V-2g

wobei jeweils die Verbindungen der Formel V-2a von den Verbindungen der Formeln V-2b und V-2C, die Verbindungen der Formel V-2b von den Verbindungen der Formel V-2c und die Verbindungen der Formel V-2e von den Verbindungen der Formel V-2f ausgeschlossen sind, und
worin die Parameter die jeweiligen oben bei Formel V-1 angegebenen Bedeutungen haben und worin

$Y^{51}$ und $Y^{52}$ jeweils unabhängig voneinander H oder F bedeuten, und bevorzugt

$R^{51}$ Alkyl oder Alkenyl,

$R^{52}$ Alkyl, Alkenyl oder Alkoxy, bevorzugt Alkyl oder Alkenyl, und bevorzugt einer von

$Y^{51}$ und $Y^{52}$ H und der andere H oder F bevorzugt ebenfalls H, bedeutet.

Bevorzugt sind die Verbindungen der Formel V-3 Verbindungen der Formel V-3a:

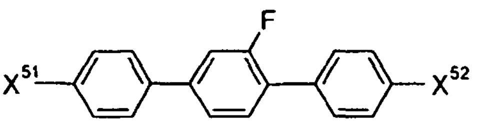

V-3a

worin die Parameter die jeweiligen oben bei Formel V-1 angegebenen Bedeutungen haben und worin bevorzugt

$X^{51}$ F, Cl, bevorzugt F,

$X^{52}$ F, Cl oder $-OCF_3$, bevorzugt $-OCF_3$, bedeutet.

In einer noch stärker bevorzugten Ausführungsform der vorliegenden Erfindung werden die Verbindungen der Formel V ausgewählt aus der Gruppe der Verbindungen V-1a bis V-1d, bevorzugt ausgewählt aus der Gruppe der Verbindungen V-1c und V-1d, stärker bevorzugt bestehen die Verbindungen der Formel V überwiegend, noch stärker bevorzugt bestehen sie im Wesentlichen und ganz besonders bevorzugt bestehen sie vollständig daraus:

Bevorzugt werden die Verbindungen der Formeln V-1 a ausgewählt aus der Gruppe der Verbindungen der Formeln V-1a-1 und V-1a-2, stärker bevorzugt bestehen diese Verbindungen der Formel V überwiegend, noch stärker bevorzugt bestehen sie im Wesentlichen und ganz besonders bevorzugt bestehen sie vollständig daraus:

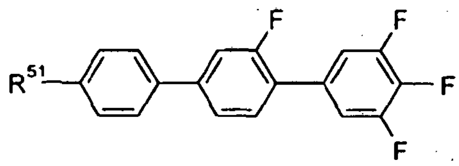

V-1a-1

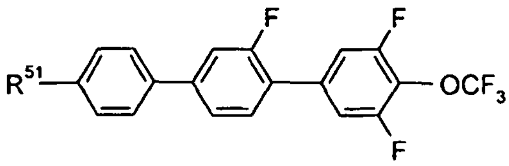

V-1a-2

worin

$R^{51}$ die oben angegebene Bedeutung hat und bevorzugt $C_nH_{2n+1}$ bedeutet, worin

n eine ganze Zahl im Bereich von 0 bis 7, bevorzugt im Bereich von 1 bis 5 und besonders bevorzugt 3 oder 7, bedeutet.

Bevorzugt sind die Verbindungen der Formeln V-1b Verbindungen der Formel V-2b-1:

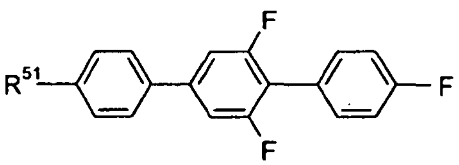

V-1b-1

worin

$R^{51}$ die oben angegebene Bedeutung hat und bevorzugt $C_nH_{2n+1}$ bedeutet, worin

n eine ganze Zahl im Bereich von 0 bis 15, bevorzugt im Bereich von 1 bis 7 und besonders bevorzugt 1 bis 5, bedeutet.

Bevorzugt werden die Verbindungen der Formeln V-1c ausgewählt aus der Gruppe der Verbindungen der Formeln V-1c-1 bis und V-1c-4, bevorzugt ausgewählt aus der Gruppe der Verbindungen der Formeln V-1c-1 und V-1c-2,stärker bevorzugt bestehen diese Verbindungen der Formel V überwiegend, noch stärker bevorzugt bestehen sie im Wesentlichen und ganz besonders bevorzugt bestehen sie vollständig daraus:

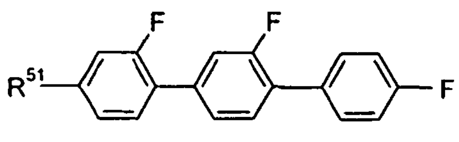

V-1c-1

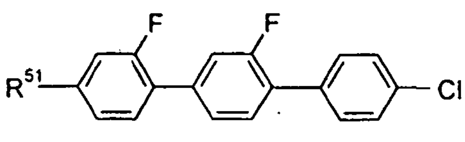

V-1c-2

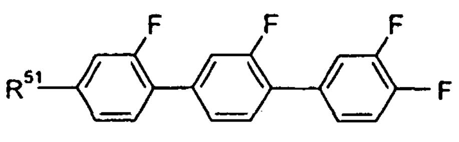

V-1c-3

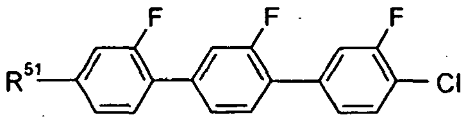

V-1c-4

worin

R^51 die oben angegebene Bedeutung hat und bevorzugt $C_nH_{2n+1}$ bedeutet, worin

n eine ganze Zahl im Bereich von 0 bis 15, bevorzugt im Bereich von 1 bis 7 und besonders bevorzugt 1 bis 5, bedeutet.

Bevorzugt werden die Verbindungen der Formeln V-1d ausgewählt aus der Gruppe der Verbindungen der Formeln V-1d-1 und V-1d-2, bevorzugt der Verbindung der Formeln V-1d-2, stärker bevorzugt bestehen diese Verbindungen der Formel V überwiegend, noch stärker bevorzugt bestehen sie im Wesentlichen und ganz besonders bevorzugt bestehen sie vollständig daraus:

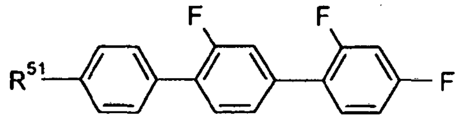

V-1d-1

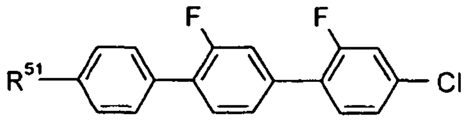

V-1d-2

worin

$R^{51}$ die oben angegebene Bedeutung hat und bevorzugt $C_nH_{2n+1}$ bedeutet, worin

n eine ganze Zahl im Bereich von 0 bis 15, bevorzugt im Bereich von 1 bis 7 und besonders bevorzugt 1 bis 5, bedeutet.

Bevorzugt werden die Verbindungen der Formeln V-2a ausgewählt aus der Gruppe der Verbindungen der Formeln V-2a-1 und V-2a-2, bevorzugt der Verbindungen der Formeln V-1a-1, stärker bevorzugt bestehen diese Verbindungen der Formel V überwiegend, noch stärker bevorzugt bestehen sie im Wesentlichen und ganz besonders bevorzugt bestehen sie vollständig daraus:

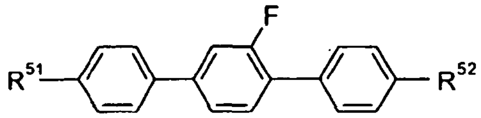

V-2a-1

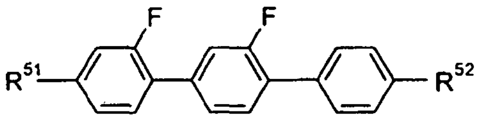

V-2a-2

worin

$R^{51}$ die oben angegebene Bedeutung hat und bevorzugt $C_nH_{2n+1}$ oder $CH_2$=CH-$(CH_2)_z$ bedeutet, und

$R^{52}$ die oben angegebene Bedeutung hat und bevorzugt $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5 bedeuten, und

z 0, 1, 2, 3 oder 4, bevorzugt 0 oder 2, bedeutet.

Bevorzugte Kombinationen von ($R^{51}$ und $R^{52}$), insbesondere bei Formel V-2a-1 sind ($C_nH_{2n+1}$ und $C_mH_{2m+1}$), ($C_nH_{2n+1}$ und O-$C_mH_{2m+1}$), ($CH_2$=CH-$(CH_2)_Z$ und $C_mH_{2m+1}$), ($CH_2$=CH-$(CH_2)_Z$ und O-$C_mH_{2m+1}$) und ($C_nH_{2n+1}$ und $(CH_2)_Z$-CH=$CH_2$).

Bevorzugte Verbindungen der Formel V-2b sind die Verbindungen der Formel V-2b-1:

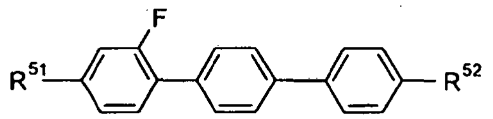

V-2b-1

worin

$R^{51}$ die oben angegebene Bedeutung hat und bevorzugt $C_nH_{2n+1}$ oder $CH_2$=CH-$(CH_2)_Z$ bedeutet, und

$R^{52}$ die oben angegebene Bedeutung hat und bevorzugt $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5, bedeuten und

z 0, 1, 2, 3 oder 4, bevorzugt 0 oder 2, bedeutet.

Die bevorzugte Kombination von ($R^{51}$ und $R^{52}$) ist hier insbesondere ($C_nH_{2n+1}$ und $C_mH_{2m+1}$).

Bevorzugte Verbindungen der Formel V-2c sind die Verbindungen der Formel V-2c-1:

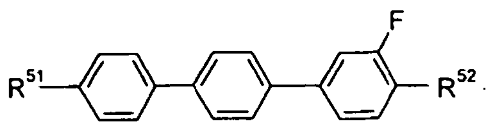

V-2c-1

worin

$R^{51}$ die oben angegebene Bedeutung hat und bevorzugt $C_nH_{2n+1}$ oder $CH_2$=CH-$(CH_2)_z$ bedeutet, und

$R^{52}$ die oben angegebene Bedeutung hat und bevorzugt $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5 bedeuten, und

z 0, 1, 2, 3 oder 4, bevorzugt 0 oder 2, bedeutet.

Die bevorzugte Kombination von ($R^{51}$ und $R^{52}$) ist hier insbesondere ($C_nH_{2n+1}$ und $C_mH_{2m+1}$).

Bevorzugte Verbindungen der Formel V-2d sind die Verbindungen der Formel V-2d-1:

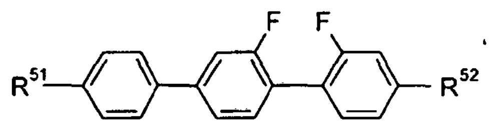

V-2d-1

worin

$R^{51}$ die oben angegebene Bedeutung hat und bevorzugt $C_nH_{2n+1}$ oder $CH_2$=CH-$(CH_2)_z$ bedeutet, und

$R^{52}$ die oben angegebene Bedeutung hat und bevorzugt $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5 bedeuten, und

z 0, 1, 2, 3 oder 4, bevorzugt 0 oder 2, bedeutet.

Die bevorzugte Kombination von ($R^{51}$ und $R^{52}$) ist hier insbesondere ($C_nH_{2n+1}$ und $C_mH_{2m+1}$).
Bevorzugte Verbindungen der Formel V-2e sind die Verbindungen der Formel V-2e-1:

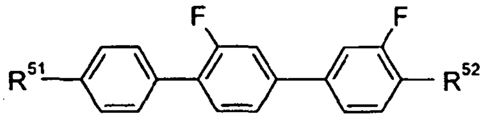

V-2e-1

worin

$R^{51}$ die oben angegebene Bedeutung hat und bevorzugt $C_nH_{2n+1}$ oder $CH_2$=CH-$(CH_2)_z$ bedeutet, und

$R^{52}$ die oben angegebene Bedeutung hat und bevorzugt $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5 bedeuten, und

z 0, 1, 2, 3 oder 4, bevorzugt 0 oder 2, bedeutet.

Die bevorzugte Kombination von ($R^{51}$ und $R^{52}$) ist hier insbesondere ($C_nH_{2n+1}$ und O-$C_mH_{2m+1}$).
Bevorzugte Verbindungen der Formel V-2f sind die Verbindungen der Formel V-2f-1:

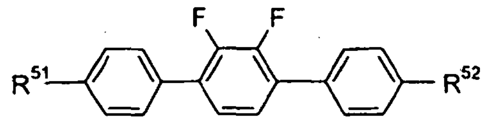

V-2f-1

worin

$R^{51}$ die oben angegebene Bedeutung hat und bevorzugt $C_nH_{2n+1}$ oder $CH_2$=CH-$(CH_2)_z$ bedeutet, und

$R^{52}$ die oben angegebene Bedeutung hat und bevorzugt $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5 bedeuten, und

z 0, 1, 2, 3 oder 4, bevorzugt 0 oder 2, bedeutet.

Die bevorzugten Kombinationen von ($R^{51}$ und $R^{52}$) sind hier insbesondere ($C_nH_{2n+1}$ und $C_mH_{2m+1}$) und ($C_nH_{2n+1}$ und O-$C_mH_{2m+1}$), besonders bevorzugt ($C_nH_{2n+1}$ und $C_mH_{2m+1}$).
Bevorzugte Verbindungen der Formel V-2g sind die Verbindungen der Formel V-2g-1:

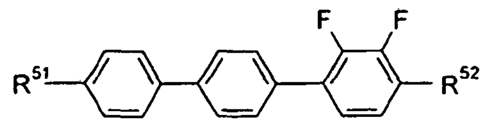

V-2g-1

worin

$R^{51}$ die oben angegebene Bedeutung hat und bevorzugt $C_nH_{2n+1}$ oder $CH_2$=CH-$(CH_2)_z$ bedeutet, und

$R^{52}$ die oben angegebene Bedeutung hat und bevorzugt $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5 bedeuten, und

z 0, 1, 2, 3 oder 4, bevorzugt 0 oder 2, bedeutet.

Die bevorzugten Kombinationen von ($R^{51}$ und $R^{52}$) sind hier insbesondere ($C_nH_{2n+1}$ und $C_mH_{2m+1}$) und ($C_nH_{2n+1}$ und O-$C_mH_{2m+1}$), besonders bevorzugt ($C_nH_{2n+1}$ und O-$C_mH_{2m+1}$).

Bevorzugt werden die Verbindungen der Formeln VI ausgewählt aus der Gruppe der Verbindungen der Formeln VI-1 bis VI-4, stärker bevorzugt bestehen diese Verbindungen der Formel VI überwiegend, noch stärker bevorzugt bestehen sie im Wesentlichen und ganz besonders bevorzugt bestehen sie vollständig daraus:

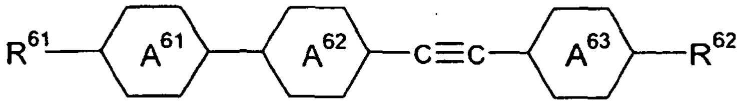
VI-1

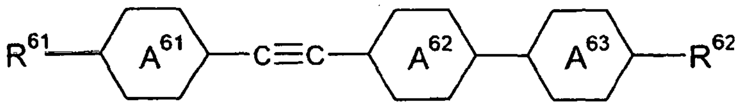
VI-2

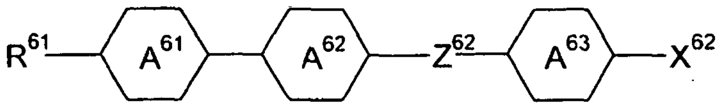
VI-3

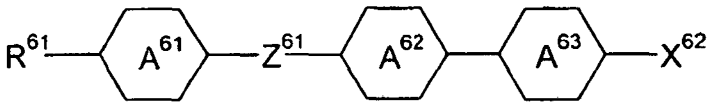
VI-4

worin

$Z^{61}$ und $Z^{62}$ *trans*- -CH=CH- oder *trans*- -CF=CF-, bevorzugt *trans*- -CH=CH-, bedeutet, und die übrigen Parameter die oben unter Formel VI gegebene Bedeutung haben und bevorzugt

$R^{61}$ und $R^{62}$ unabhängig voneinander H, unfluoriertes Alkyl oder Alkoxy mit 1 bis 7 C-Atomen oder unfluoriertes Alkenyl mit 2 bis 7 C-Atomen, bedeuten,

$X^{62}$ F, Cl, -CN oder -NCS, vorzugsweise -NCS, bedeutet, und einer von

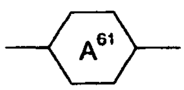
bis
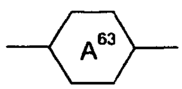

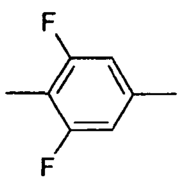
,
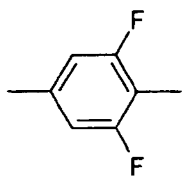
,

oder

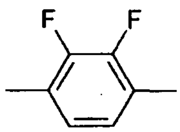

bedeutet
und die anderen unabhängig voneinander

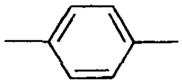, 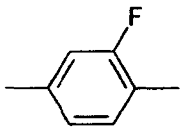, 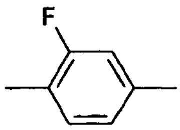,

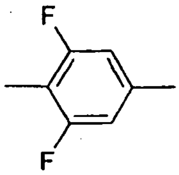, 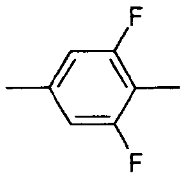, 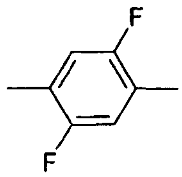,

oder

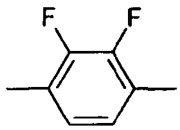,

vorzugsweise

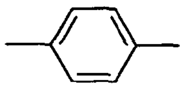, 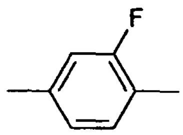, 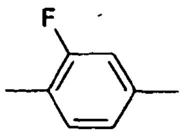,

oder

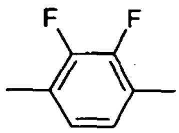,

bedeuten und bevorzugt

$R^{61}$ $C_nH_{2n+1}$ oder $CH_2$=CH-$(CH_2)_Z$ bedeutet, und

$R^{62}$ $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5 bedeuten, und

z 0, 1, 2, 3 oder 4, bevorzugt 0 oder 2, bedeutet.

Bevorzugt werden die Verbindungen der Formeln VI-1 ausgewählt aus der Gruppe der Verbindungen der Formeln VI-1a und VI-1b, bevorzugt ausgewählt Verbindungen der Formeln VI-1a, stärker bevorzugt bestehen diese Verbindungen der Formel VI überwiegend, noch stärker bevorzugt bestehen sie im Wesentlichen und ganz besonders bevorzugt bestehen sie vollständig daraus:

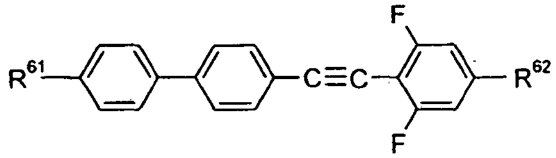

VI-1a

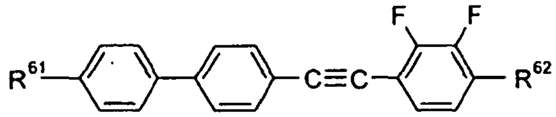

VI-1b

worin

| | |
|---|---|
| $R^{61}$ | die oben angegebene Bedeutung hat und bevorzugt $C_nH_{2n+1}$ oder $CH_2$=CH-$(CH_2)_z$ bedeutet, und |
| $R^{62}$ | die oben angegebene Bedeutung hat und bevorzugt $C_mH_{2m+1}$ oder O-$C_mH_{2m+1}$ oder $(CH_2)_Z$-CH=$CH_2$ bedeutet, und worin |
| n und m | unabhängig voneinander eine ganze Zahl im Bereich von 0 bis 15, bevorzugt im Bereich von 1 bis 7 und besonders bevorzugt 1 bis 5 bedeuten, und |
| z | 0, 1, 2, 3 oder 4, bevorzugt 0 oder 2, bedeutet. |

Die bevorzugten Kombinationen von ($R^{61}$ und $R^{62}$) sind hier insbesondere ($C_nH_{2n+1}$ und $C_mH_{2m+1}$) und ($C_nH_{2n+1}$ und O-$C_mH_{2m+1}$), bei Formel VI-1a besonders bevorzugt ($C_nH_{2n+1}$ und $C_mH_{2m+1}$) und bei Formel VI-1b besonders bevorzugt ($C_nH_{2n+1}$ und O-$C_nH_{2m+1}$).

Bevorzugt sind die Verbindungen der Formel VI-3 Verbindungen der Formel VI-3a:

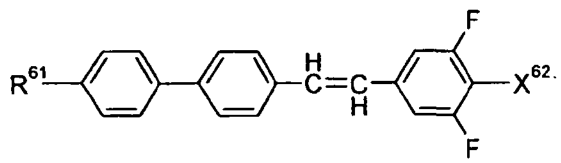

VI-3a

worin die Parameter die oben unter Formel VI-3 gegebene Bedeutung haben und bevorzugt

| | |
|---|---|
| $R^{61}$ | die oben angegebene Bedeutung hat und bevorzugt $C_nH_{2n+1}$ bedeutet, worin |
| n | eine ganze Zahl im Bereich von 0 bis 7, bevorzugt im Bereich von 1 bis 5, und |
| $X^{62}$ | F, Cl, $OCF_3$, -CN oder -NCS, besonders bevorzugt -NCS, bedeutet. |

Bevorzugt sind die Verbindungen der Formeln VI-4 Verbindungen der Formel VI-4a:

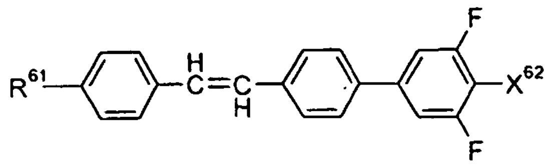

VI-4a

worin die Parameter die oben unter Formel VI-4 gegebene Bedeutung haben und bevorzugt

$R^{61}$ die oben angegebene Bedeutung hat und bevorzugt $C_nH_{2n+1}$ bedeutet, worin

n eine ganze Zahl im Bereich von 0 bis 7, bevorzugt im Bereich von 1 bis 5, und

$X^{62}$ F, Cl, $OCF_3$, -CN oder -NCS, besonders bevorzugt -NCS, bedeutet.

Weitere bevorzugte Verbindungen der Formel VI sind die Verbindungen der folgenden Formeln

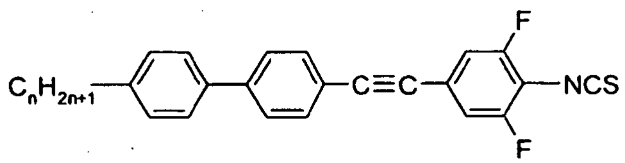

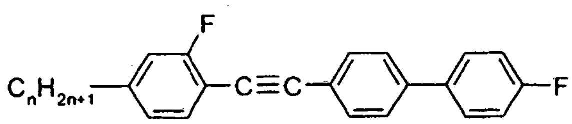

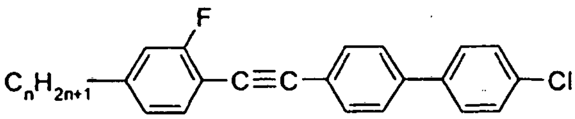

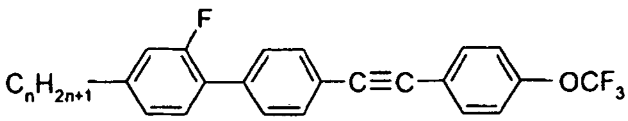

worin

n eine ganze Zahl im Bereich von 0 bis 7, bevorzugt im Bereich von 1 bis 5 bedeutet.

Bevorzugt werden die Verbindungen der Formeln VII ausgewählt aus der Gruppe der Verbindungen der Formeln VII-1 bis VII-6, stärker bevorzugt bestehen diese Verbindungen der Formel VII überwiegend, noch stärker bevorzugt bestehen sie im Wesentlichen und ganz besonders bevorzugt bestehen sie vollständig daraus:

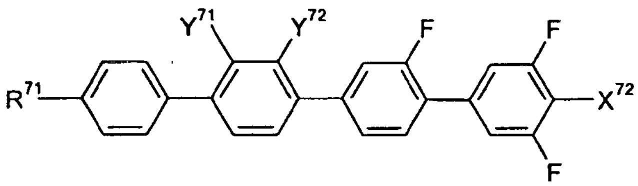

VII-1

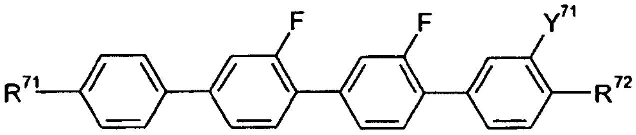

VII-2

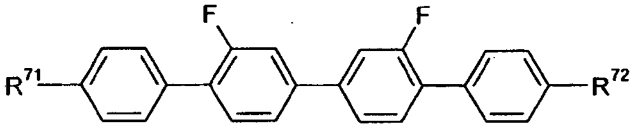

VII-3

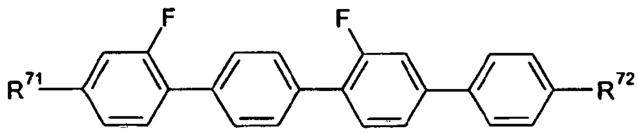

VII-4

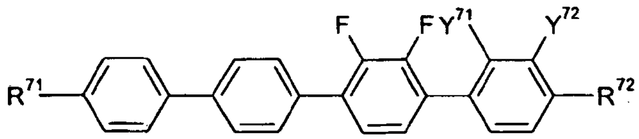

VII-5

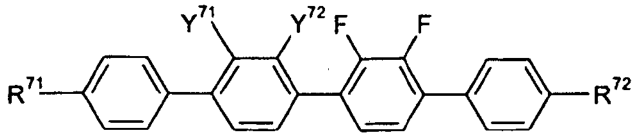

VII-6

wobei die Verbindungen der Formel VII-5 von den Verbindungen der Formel VII-6 ausgeschlossen sind, und worin die Parameter die jeweiligen oben bei Formel VII angegebenen Bedeutungen haben,

$Y^{71}$ und $Y^{72}$ jeweils unabhängig voneinander H oder F bedeuten,

und bevorzugt

$R^{71}$ unfluoriertes Alkyl oder Alkoxy jeweils mit 1 bis 7 C-Atomen oder unfluoriertes Alkenyl mit 2 bis 7 C-Atomen,

$R^{72}$ unfluoriertes Alkyl oder Alkoxy, jeweils mit 1 bis 7 C-Atomen oder unfluoriertes Alkenyl mit 2 bis 7 C-Atomen, und

$X^{72}$ F, Cl, oder $-OCF_3$, bevorzugt F, bedeutet, und,

besonders bevorzugt

$R^{71}$ die oben angegebene Bedeutung hat und bevorzugt $C_nH_{2n+1}$ oder $CH_2=CH-(CH_2)_z$ bedeutet, und

$R^{72}$ die oben angegebene Bedeutung hat und bevorzugt $C_mH_{2m+1}$ oder $O-C_mH_{2m+1}$ oder $(CH_2)_Z-CH=CH_2$ bedeutet, und worin

n und m unabhängig voneinander eine ganze Zahl im Bereich von 0 bis 15, bevorzugt im Bereich von 1 bis 7 und besonders bevorzugt 1 bis 5 bedeuten und

z 0, 1, 2, 3 oder 4, bevorzugt 0 oder 2, bedeutet.

Bevorzugt werden die Verbindungen der Formeln VII-1 ausgewählt aus der Gruppe der Verbindungen der Formeln VII-1a bis VII-1d, stärker bevorzugt bestehen diese Verbindungen der Formel VII-1 überwiegend, noch stärker bevorzugt bestehen sie im Wesentlichen und ganz besonders bevorzugt bestehen sie vollständig daraus:

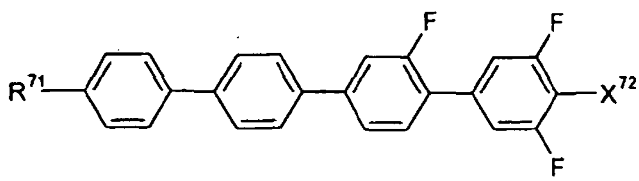

VII-1a

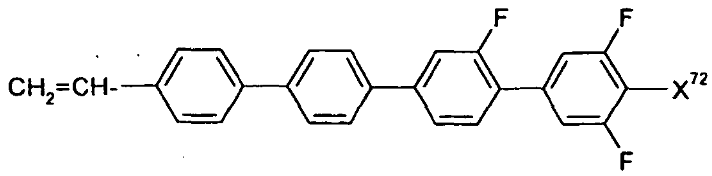

VII-1b

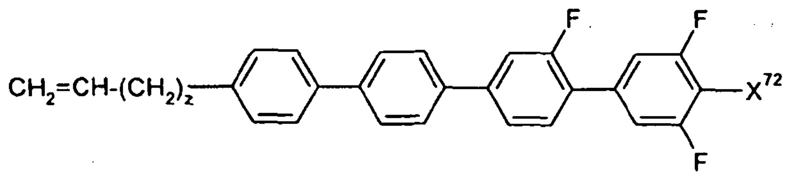

VII-1c

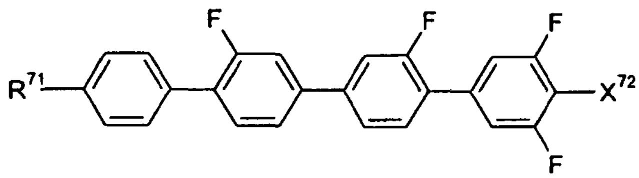

VII-1d

worin $X^{72}$ die oben bei Formel VII-2 gegebene Bedeutung hat und

$R^{71}$ die oben angegebene Bedeutung hat und bevorzugt $C_nH_{2n+1}$ bedeutet, worin

n 1 bis 7, bevorzugt 2 bis 6, besonders bevorzugt 2, 3 oder 5, und

z 0, 1, 2, 3 oder 4, bevorzugt 0 oder 2, und

$X^{72}$ bevorzugt F

bedeutet.

Bevorzugt werden die Verbindungen der Formeln VII-2 ausgewählt aus der Gruppe der Verbindungen der Formeln VII-2a und VII-2b, bevorzugt der Formel VII-2a, stärker bevorzugt bestehen diese Verbindungen der Formel VII-2 überwiegend, noch stärker bevorzugt bestehen sie im Wesentlichen und ganz besonders bevorzugt bestehen sie vollständig daraus:

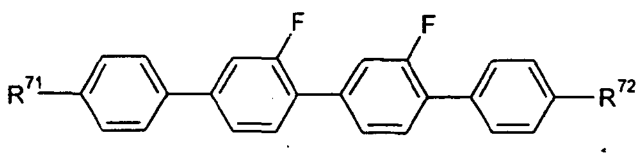

VII-2a

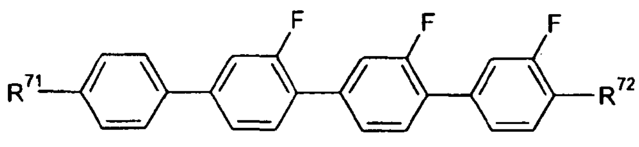

VII-2b

worin

$R^{71}$ die oben angegebene Bedeutung hat und bevorzugt $C_nH_{2n+1}$ oder $CH_2=CH-(CH_2)_Z$ bedeutet, und

$R^{72}$ die oben angegebene Bedeutung hat und bevorzugt $C_mH_{2m+1}$ oder $O-C_mH_{2m+1}$ oder $(CH_2)_Z-CH=CH_2$ bedeutet, und worin

n und m unabhängig voneinander eine ganze Zahl im Bereich von 0 bis 15, bevorzugt im Bereich von 1 bis 7 und

besonders bevorzugt 1 bis 5 bedeuten, und

z 0, 1, 2, 3 oder 4, bevorzugt 0 oder 2, bedeutet.

Die bevorzugten Kombinationen von ($R^{71}$ und $R^{72}$) sind hier insbesondere ($C_nH_{2n+1}$ und $C_mH_{2m+1}$) und ($C_nH_{2n+1}$ und $O-C_mH_{2m+1}$). Besonders bevorzugt ($C_nH_{2n+1}$ und $C_mH_{2m+1}$).

Bevorzugt sind die Verbindungen der Formel VII-3 Verbindungen der Formel VII-3a:

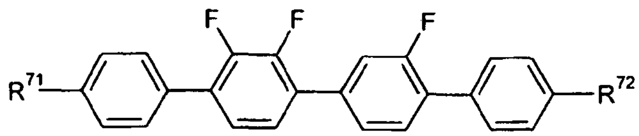

VII-3a

worin

$R^{71}$ die oben angegebene Bedeutung hat und bevorzugt $C_nH_{2n+1}$ oder $CH_2=CH-(CH_2)_Z$ bedeutet, und

$R^{72}$ die oben angegebene Bedeutung hat und bevorzugt $C_mH_{2m+1}$ oder $O-C_mH_{2m+1}$ oder $(CH_2)_Z-CH=CH_2$ bedeutet, und worin

n und m unabhängig voneinander eine ganze Zahl im Bereich von 0 bis 15, bevorzugt im Bereich von 1 bis 7 und besonders bevorzugt 1 bis 5 bedeuten, und

z 0, 1, 2, 3 oder 4, bevorzugt 0 oder 2, bedeutet.

Die bevorzugten Kombinationen von ($R^{71}$ und $R^{72}$) sind hier insbesondere ($C_nH_{2n+1}$ und $C_mH_{2m+1}$) und ($C_nH_{2n+1}$ und $O-C_mH_{2m+1}$). Besonders bevorzugt ($C_nH_{2n+1}$ und $C_mH_{2m+1}$).

Bevorzugt sind die Verbindungen der Formel VII-4 die Verbindungen, bei denen

$R^{71}$ die oben angegebene Bedeutung hat und bevorzugt $C_nH_{2n+1}$ oder $CH_2=CH-(CH_2)_z$ bedeutet, und

$R^{72}$ die oben angegebene Bedeutung hat und bevorzugt $C_mH_{2m+1}$ oder $O-C_mH_{2m+1}$ oder $(CH_2)_Z-CH=CH_2$ bedeutet, und worin

n und m unabhängig voneinander eine ganze Zahl im Bereich von 0 bis 15, bevorzugt im Bereich von 1 bis 7 und besonders bevorzugt 1 bis 5 bedeuten, und

z 0, 1, 2, 3 oder 4, bevorzugt 0 oder 2, bedeutet.

Die bevorzugten Kombinationen von ($R^{71}$ und $R^{72}$) sind hier insbesondere ($C_nH_{2n+1}$ und $C_mH_{2m+1}$) und ($C_nH_{2n+1}$ und $O-C_mH_{2m+1}$). Besonders bevorzugt ($C_nH_{2n+1}$ und $C_mH_{2m+1}$).

Bevorzugt werden die Verbindungen der Formeln VII-5 ausgewählt aus der Gruppe der Verbindungen der Formeln VII-5a und VII-5b, bevorzugt der Formel VII-5a stärker bevorzugt bestehen diese Verbindungen der Formel VII-5 überwiegend, noch stärker bevorzugt bestehen sie im Wesentlichen und ganz besonders bevorzugt bestehen sie vollständig daraus:

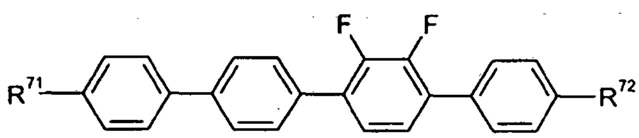

VII-5a

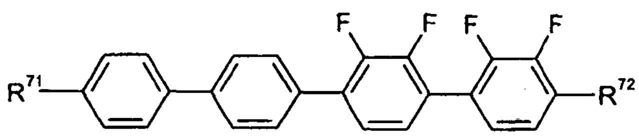

VII-5b

worin

$R^{71}$ die oben angegebene Bedeutung hat und bevorzugt $C_nH_{2n+1}$ oder $CH_2=CH-(CH_2)_Z$ bedeutet, und
$R^{72}$ die oben angegebene Bedeutung hat und bevorzugt $C_mH_{2m+1}$ oder $O-C_mH_{2m+1}$ oder $(CH_2)_Z-CH=CH_2$ bedeutet, und worin
n und m unabhängig voneinander eine ganze Zahl im Bereich von 0 bis 15, bevorzugt im Bereich von 1 bis 7 und besonders bevorzugt 1 bis 5 bedeuten und
z 0, 1, 2, 3 oder 4, bevorzugt 0 oder 2, bedeutet.

Die bevorzugten Kombinationen von ($R^{71}$ und $R^{72}$) sind hier insbesondere ($C_nH_{2n+1}$ und $C_mH_{2m+1}$) und ($C_nH_{2n+1}$ und $O-C_mH_{2m+1}$). Besonders bevorzugt ($C_nH_{2n+1}$ und $C_mH_{2m+1}$).

Bevorzugt werden die Verbindungen der Formeln VII-6 ausgewählt aus der Gruppe der Verbindungen der Formeln VII-6a und VII-6b, stärker bevorzugt bestehen diese Verbindungen der Formel VII-6 überwiegend, noch stärker bevorzugt bestehen sie im Wesentlichen und ganz besonders bevorzugt bestehen sie vollständig daraus:

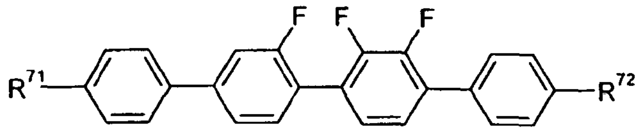

VII-6a

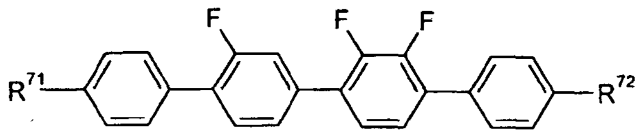

VII-6b

worin

$R^{71}$ die oben angegebene Bedeutung hat und bevorzugt $C_nH_{2n+1}$ oder $CH_2=CH-(CH_2)_Z$ bedeutet, und

$R^{72}$ die oben angegebene Bedeutung hat und bevorzugt $C_mH_{2m+1}$ oder $O-C_mH_{2m+1}$ oder $(CH_2)_Z-CH=CH_2$ bedeutet, und worin

n und m unabhängig voneinander eine ganze Zahl im Bereich von 0 bis 15, bevorzugt im Bereich von 1 bis 7 und besonders bevorzugt 1 bis 5 bedeuten, und

z 0, 1, 2, 3 oder 4, bevorzugt 0 oder 2, bedeutet.

Die bevorzugten Kombinationen von ($R^{71}$ und $R^{72}$) sind hier insbesondere ($C_nH_{2n+1}$ und $C_mH_{2m+1}$) und ($C_nH_{2n+1}$ und $O-C_mH_{2m+1}$), besonders bevorzugt ($C_nH_{2n+1}$ und $C_mH_{2m+1}$).

Bevorzugt enthalten die flüssigkristallinen Medien gemäß der vorliegenden Anmeldung insgesamt 0 bis 40 %, bevorzugt 0 bis 30 % und besonders bevorzugt 5 bis 25 % an Verbindungen der Formel VIII.

Bevorzugt werden die Verbindungen der Formeln VIII ausgewählt aus der Gruppe der Verbindungen der Formeln VIII-1 bis VIII-3, stärker bevorzugt bestehen diese Verbindungen der Formel VIII überwiegend, noch stärker bevorzugt bestehen sie im Wesentlichen und ganz besonders bevorzugt bestehen sie vollständig daraus:

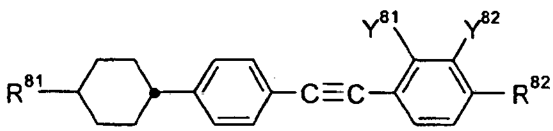

VIII-1

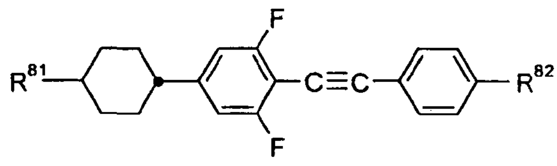

VIII-2

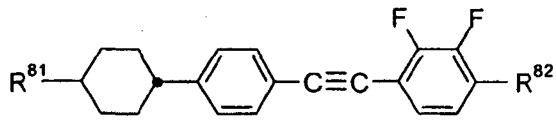

VIII-3

worin
einer von

$Y^{81}$ und $Y^{82}$ H bedeutet und der andere H oder F bedeutet, und

$R^{81}$ die oben angegebene Bedeutung hat und bevorzugt $C_nH_{2n+1}$ oder $CH_2$=CH-$(CH_2)_Z$ bedeutet, und

$R^{82}$ die oben angegebene Bedeutung hat und bevorzugt $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5 bedeuten, und

z 0, 1, 2, 3 oder 4, bevorzugt 0 oder 2, bedeutet.

Die bevorzugten Kombinationen von ($R^{81}$ und $R^{82}$) sind hier insbesondere ($C_nH_{2n+1}$ und $C_mH_{2m+1}$) und ($C_nH_{2n+1}$ und O-$C_mH_{2m+1}$) besonders bevorzugt ($C_nH_{2n+1}$ und $C_mH_{2m+1}$).

Bevorzugt werden die Verbindungen der Formeln VIII-1 ausgewählt aus der Gruppe der Verbindungen der Formeln VIII-1a bis VIII-1c, stärker bevorzugt bestehen diese Verbindungen der Formel VIII-1 überwiegend, noch stärker bevorzugt bestehen sie im Wesentlichen und ganz besonders bevorzugt bestehen sie vollständig daraus:

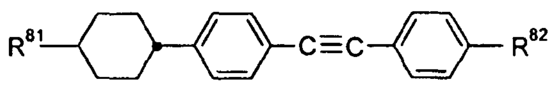

VIII-1a

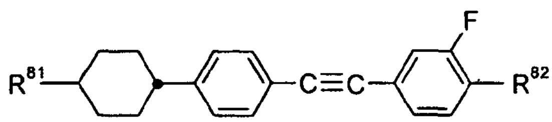

VIII-1b

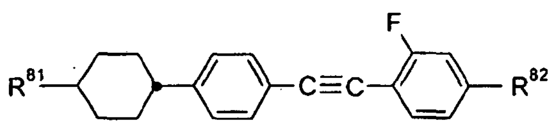

VIII-1c

worin

$R^{81}$ die oben angegebene Bedeutung hat und bevorzugt $C_nH_{2n+1}$ oder $CH_2$=CH-$(CH_2)_Z$ bedeutet, und

$R^{82}$ die oben angegebene Bedeutung hat und bevorzugt $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5, bedeuten und

z 0, 1, 2, 3 oder 4, bevorzugt 0 oder 2, bedeutet.

Die bevorzugten Kombinationen von ($R^{81}$ und $R^{82}$) sind hier insbesondere ($C_nH_{2n+1}$ und $C_mH_{2m+1}$) und ($C_nH_{2n+1}$ und O-$C_mH_{2m+1}$) besonders bevorzugt ($C_nH_{2n+1}$ und $C_mH_{2m+1}$).

Bevorzugt sind die Verbindungen der Formel VIII-2 die Verbindungen, bei denen

$R^{81}$ die oben angegebene Bedeutung hat und bevorzugt $C_nH_{2n+1}$ oder $CH_2$=CH-$(CH_2)_Z$ bedeutet, und

$R^{82}$ die oben angegebene Bedeutung hat und bevorzugt $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5, bedeuten und

z 0, 1, 2, 3 oder 4, bevorzugt 0 oder 2, bedeutet.

Die bevorzugten Kombinationen von ($R^{81}$ und $R^{82}$) sind hier insbesondere ($C_nH_{2n+1}$ und $C_mH_{2m+1}$), $C_nH_{2n+1}$ und O-$C_mH_{2m+1}$) und ($CH_2$=CH-$(CH_2)_Z$ und $C_mH_{2m+1}$) besonders bevorzugt ($C_nH_{2n+1}$ und $C_mH_{2m+1}$).

Bevorzugt sind die Verbindungen der Formel VIII-3 die Verbindungen, bei denen

$R^{81}$ die oben angegebene Bedeutung hat und bevorzugt $C_nH_{2n+1}$ oder $CH_2$=CH-$(CH_2)_Z$ bedeutet, und

$R^{82}$ die oben angegebene Bedeutung hat und bevorzugt $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5, bedeuten und

z 0, 1, 2, 3 oder 4, bevorzugt 0 oder 2, bedeutet.

Die bevorzugten Kombinationen von ($R^{81}$ und $R^{82}$) sind hier insbesondere ($C_nH_{2n+1}$ und $C_mH_{2m+1}$) und ($C_nH_{2n+1}$ und O-$C_mH_{2m+1}$).

Bevorzugt werden die Verbindungen der Formeln IX ausgewählt aus der Gruppe der Verbindungen der Formeln IX-1 bis IX-3, stärker bevorzugt bestehen diese Verbindungen der Formel IX überwiegend, noch stärker bevorzugt bestehen sie im Wesentlichen und ganz besonders bevorzugt bestehen sie vollständig daraus:

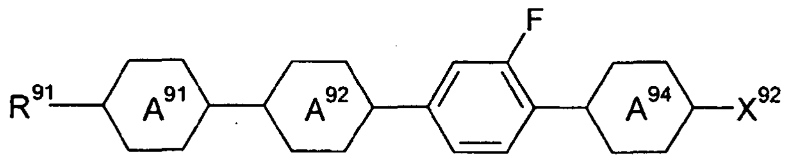

IX-1

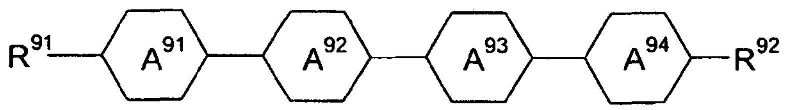

IX-2

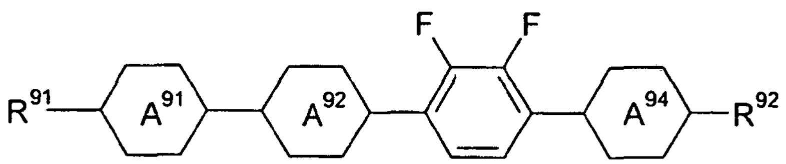

IX-3

worin die Parameter die jeweilige oben unter Formel IX angegebene Bedeutung haben und bevorzugt

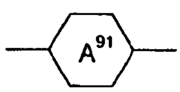

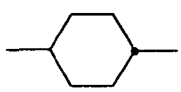

oder

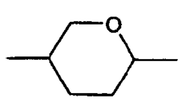

einer von

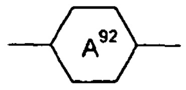 bis 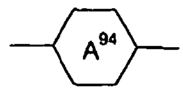

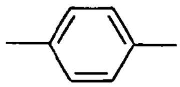, 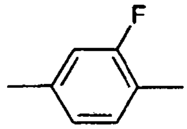, 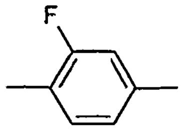,

bedeutet
und
worin

R91 die oben angegebene Bedeutung hat und bevorzugt $C_nH_{2n+1}$ oder $CH_2$=CH-$(CH_2)_Z$ bedeutet, und

R92 die oben angegebene Bedeutung hat und bevorzugt $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5, bedeuten und

z 0, 1, 2, 3 oder 4, bevorzugt 0 oder 2, bedeutet.

Die bevorzugten Kombinationen von ($R^{91}$ und $R^{92}$) sind hier insbesondere ($C_nH_{2n+1}$ und $C_mH_{2m+1}$) und ($C_nH_{2n+1}$ und O-$C_mH_{2m+1}$).

Bevorzugt enthalten die flüssigkristallinen Medien gemäß der vorliegenden Anmeldung insgesamt 5 bis 30 %, bevorzugt 10 bis 25 % und besonders bevorzugt 15 bis 20 % an Verbindungen der Formel IX.

Bevorzugt werden die Verbindungen der Formeln IX-1 ausgewählt aus der Gruppe der Verbindungen der Formeln IX-1a bis IX-1e, stärker bevorzugt bestehen diese Verbindungen der Formel IX-1 überwiegend, noch stärker bevorzugt bestehen sie im Wesentlichen und ganz besonders bevorzugt bestehen sie vollständig daraus:

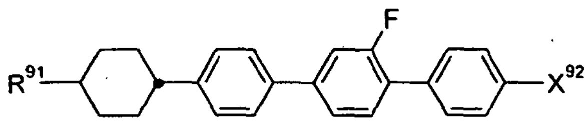 IX-1a

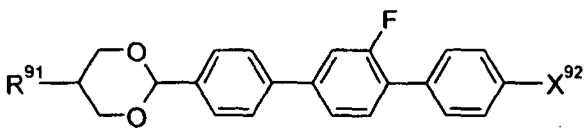 IX-1b

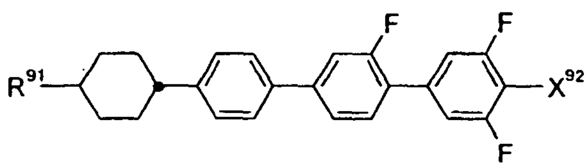 IX-1c

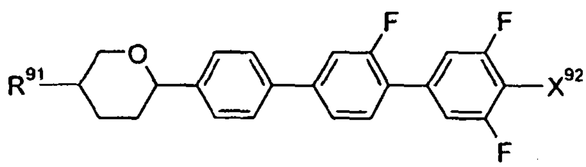 IX-1d

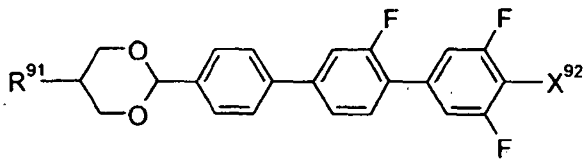

IX-1e

worin die Parameter die oben gegebene Bedeutung haben und bevorzugt

$R^{91}$ die oben angegebene Bedeutung hat und bevorzugt $C_nH_{2n+1}$ bedeutet, und

n eine ganze Zahl im Bereich von 0 bis 15, bevorzugt im Bereich von 1 bis 7 und besonders bevorzugt 1 bis 5, bedeutet und

$X^{92}$ bevorzugt F oder Cl bedeutet.

Bevorzugt werden die Verbindungen der Formeln IX-2 ausgewählt aus der Gruppe der Verbindungen der Formeln IX-2a und IX-2b, stärker bevorzugt bestehen diese Verbindungen der Formel IX-2 überwiegend, noch stärker bevorzugt bestehen sie im Wesentlichen und ganz besonders bevorzugt bestehen sie vollständig daraus:

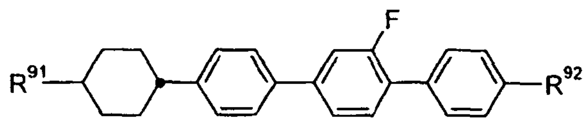

IX-2a

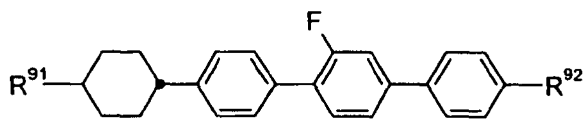

IX-2b

worin

$R^{91}$ die oben angegebene Bedeutung hat und bevorzugt $C_nH_{2n+1}$ oder $CH_2=CH-(CH_2)_Z$ bedeutet, und

$R^{92}$ die oben angegebene Bedeutung hat und bevorzugt $C_mH_{2m+1}$ oder $O-C_mH_{2m+1}$ oder $(CH_2)_Z-CH=CH_2$ bedeutet, und worin

n und m unabhängig voneinander eine ganze Zahl im Bereich von 0 bis 15, bevorzugt im Bereich von 1 bis 7 und besonders bevorzugt 1 bis 5, bedeuten und

z 0, 1, 2, 3 oder 4, bevorzugt 0 oder 2, bedeutet.

Die bevorzugte Kombination von ($R^{91}$ und $R^{92}$) ist hier insbesondere ($C_nH_{2n+1}$ und $C_mH_{2m+1}$).

Bevorzugt sind die Verbindungen der Formel IX-3 Verbindungen der Formel IX-3:

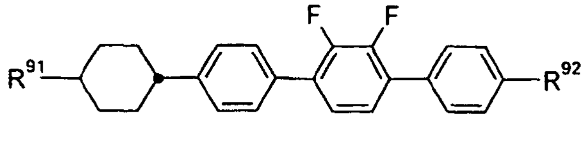

IX-3a

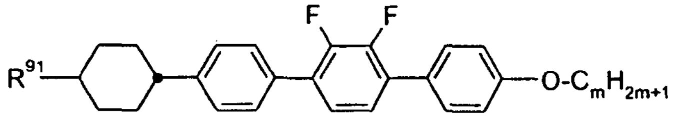

IX-3b

worin

$R^{91}$ die oben angegebene Bedeutung hat und bevorzugt $C_nH_{2n+1}$ oder $CH_2=CH-(CH_2)_Z$ bedeutet, und

$R^{92}$ die oben angegebene Bedeutung hat und bevorzugt $C_mH_{2m+1}$ oder O-$C_mH_{2m+1}$ oder $(CH_2)_Z$-CH=$CH_2$ bedeutet, und worin

n und m unabhängig voneinander eine ganze Zahl im Bereich von 0 bis 15, bevorzugt im Bereich von 1 bis 7 und besonders bevorzugt 1 bis 5, bedeuten und

z 0, 1, 2, 3 oder 4, bevorzugt 0 oder 2, bedeutet.

Die bevorzugten Kombinationen von ($R^{91}$ und $R^{92}$) sind hier insbesondere ($C_nH_{2n+1}$ und $C_mH_{2m+1}$) und ($C_nH_{2n+1}$ und O-$C_mH_{2m+1}$), besonders bevorzugt ($C_nH_{2n+1}$ und O-$C_mH_{2m+1}$).

In einer bevorzugten Ausführungsform der vorliegenden Erfindung enthält das Medium eine oder mehrere dielektrisch positive Verbindungen mit einer dielektrischen Anisotropie von mehr als 3 der Formel V-1. Bevorzugt enthalten die flüssigkristallinen Medien gemäß der vorliegenden Erfindung 10 % oder weniger, bevorzugt 5 % oder weniger, besonders bevorzugt 2 % oder weniger, ganz besonders bevorzugt 1 % oder weniger, und insbesondere überhaupt keine Verbindung mit nur zwei oder weniger fünf und/oder sechsgliedrigen Ringen.

In einer bevorzugten Ausführungsform der vorliegenden Erfindung enthält das Medium eine oder mehrere Verbindungen der Formel VI.

In einer weiteren bevorzugten Ausführungsform der vorliegenden Erfindung enthält das Medium eine oder mehrere Verbindungen der Formel VII.

Die Definitionen der Abkürzungen (Akronyme) sind unten in Tabelle D angegeben, bzw. aus den Tabellen A bis C zu ersehen.

Vorzugsweise enthalten die flüssigkristallinen Medien gemäß der vorliegenden Erfindung Verbindungen ausgewählt aus der Gruppe der Verbindungen der Formeln I, II, IV und V, vorzugsweise I, II und IV oder ausgewählt aus der Gruppe der Verbindungen der Formeln I, III, IV und V, vorzugsweise I, III und IV, stärker bevorzugt bestehen sie überwiegend, noch stärker bevorzugt bestehen sie im Wesentlichen und ganz bevorzugt bestehen sie vollständig daraus.

In dieser Anmeldung bedeutet enthalten im Zusammenhang mit Zusammensetzungen, dass die betreffende Entität, d.h. das Medium oder die Komponente, die angegebene Komponente oder Komponenten oder Verbindung oder Verbindungen enthält, vorzugsweise in einer Gesamtkonzentration von 10 % oder mehr und ganz bevorzugt von 20 % oder mehr.

Überwiegend bestehen bedeutet in diesem Zusammenhang, dass die betreffende Entität 55 % oder mehr, vorzugsweise 60 % oder mehr und ganz bevorzugt 70 % oder mehr der angegebenen Komponente oder Komponenten oder Verbindung oder Verbindungen enthält.

Im Wesentlichen bestehen bedeutet in diesem Zusammenhang, dass die betreffende Entität 80 % oder mehr, vorzugsweise 90 % oder mehr und ganz bevorzugt 95 % oder mehr der angegebenen Komponente oder Komponenten oder Verbindung oder Verbindungen enthält.

Vollständig bestehen bedeutet in diesem Zusammenhang, dass die betreffende Entität 98 % oder mehr, vorzugsweise 99 % oder mehr und ganz bevorzugt 100,0 % der angegebenen Komponente oder Komponenten oder Verbindung oder Verbindungen enthält.

Auch andere mesogene Verbindungen, die oben nicht explizit genannt sind, können gegebenenfalls und in vorteilhafter Weise in den Medien gemäß der vorliegenden Erfindung verwendet werden. Solche Verbindungen sind dem Fachmann bekannt.

Die Flüssigkristallmedien gemäß der vorliegenden Erfindung weisen bevorzugt einen Klärpunkt von 90°C oder mehr, stärker bevorzugt von 100°C oder mehr, noch stärker bevorzugt von 120°C oder mehr, besonders bevorzugt von 150°C oder mehr und ganz besonders bevorzugt von 170°C oder mehr auf.

Vorzugsweise erstreckt sich die nematische Phase der erfindungsgemäßen Medien mindestens von 20°C oder weniger bis 90°C oder mehr, bevorzugt bis 100°C oder mehr, stärker bevorzugt mindestens von 0°C oder weniger bis 120°C oder mehr, ganz bevorzugt mindestens von -10°C oder weniger bis 140°C oder mehr und insbesondere mindestens von -20°C oder weniger bis 150°C oder mehr.

Das $\Delta\varepsilon$ des Flüssigkristallmediums gemäß der Erfindung bei 1 kHz und 20°C beträgt vorzugsweise 1 oder mehr, stärker bevorzugt 2 oder mehr und ganz bevorzugt 3 oder mehr.

Das $\Delta n$ der Flüssigkristallmedien gemäß der vorliegenden Erfindung liegt bei 589 nm ($Na^D$) und 20°C vorzugsweise im Bereich von 0,200 oder mehr bis 0,90 oder weniger, stärker bevorzugt im Bereich von 0,250 oder mehr bis 0,90 oder weniger, noch stärker bevorzugt im Bereich von 0,300 oder mehr bis 0, 85 oder weniger und ganz besonders bevorzugt im Bereich von 0,350 oder mehr bis 0,800 oder weniger.

In einer bevorzugten Ausführungsform der vorliegenden Anmeldung beträgt das $\Delta n$ der Flüssigkristallmedien gemäß der vorliegenden Erfindung vorzugsweise 0,50 oder mehr, stärker bevorzugt 0,55 oder mehr.

Gemäß der vorliegenden Erfindung werden die einzelnen Verbindungen der Formel I in den Flüssigkristallmedien vorzugsweise in einer Gesamtkonzentration von 1 % bis 20 %, stärker bevorzugt von 2 % bis 15 %, noch stärker

bevorzugt von 3 % bis 12 % und ganz bevorzugt von 5 % bis 10 % der Gesamtmischung verwendet.

In der Ausführungsform der vorliegenden Erfindung in der die Flüssigkristallmedien eine oder mehrere Verbindungen ausgewählt aus der Gruppe der Verbindungen der Formeln IIA bis IID enthalten, werden die weiteren Verbindungen vorzugsweise wie folgt eingesetzt.

Die Verbindungen ausgewählt aus der Gruppe der Verbindungen der Formeln IIA bis IID werden vorzugsweise in einer Gesamtkonzentration von 1 % bis 30 %, stärker bevorzugt von 2 % bis 20 %, stärker bevorzugt von 3 % bis 18 % und ganz bevorzugt von 4 % bis 16 % der Gesamtmischung verwendet.

Die Verbindungen der Formel IV werden vorzugsweise in einer Gesamtkonzentration von 10 % bis 100 %, stärker bevorzugt von 30 % bis 95 %, noch stärker bevorzugt von 40 % bis 90 % und ganz bevorzugt von 50 % bis 90 % der Gesamtmischung verwendet.

Die Flüssigkristallmedien enthalten vorzugsweise insgesamt 70 % bis 100 %, stärker bevorzugt 80 % bis 100 % und ganz bevorzugt 90 % bis 100 % und insbesondere 95 % bis 100 % der Verbindungen der Formeln I, IIA, IIB, IIC, IID und IV bis IX, vorzugsweise der Formeln I, IIA, IIB, IIC, IID und IV, vorzugsweise bestehen sie überwiegend daraus und ganz bevorzugt bestehen sie vollständig daraus.

In der Ausführungsform der vorliegenden Erfindung in der die Flüssigkristallmedien eine oder mehrere Verbindungen ausgewählt aus der Gruppe der Verbindungen der Formel IIIA und IIIB enthalten, werden die weiteren Verbindungen vorzugsweise wie folgt eingesetzt.

Die Verbindungen ausgewählt aus der Gruppe der Verbindungen der Formel IIIA und IIIB werden vorzugsweise in einer Gesamtkonzentration von 1 % bis 60 %, stärker bevorzugt von 5 % bis 55 %, stärker bevorzugt von 7 % bis 50 % und ganz bevorzugt von 10 % bis 45 % der Gesamtmischung verwendet.

Wenn die Flüssigkristallmedien nur eine oder mehrere Verbindungen der Formel IIIA aber keine Verbindungen der Formel IIIB enthält, werden die Verbindungen der Formel IIIA vorzugsweise in einer Gesamtkonzentration von 10 % bis 60 %, stärker bevorzugt von 20 % bis 55 %, stärker bevorzugt von 30 % bis 50 % und ganz bevorzugt von 35 % bis 45 % der Gesamtmischung verwendet.

Wenn die Flüssigkristallmedien nur eine oder mehrere Verbindungen der Formel IIIB aber keine Verbindungen der Formel IIIA enthält, werden die Verbindungen der Formel IIIB vorzugsweise in einer Gesamtkonzentration von 5 % bis 45 %, stärker bevorzugt von 10 % bis 40 %, stärker bevorzugt von 15 % bis 35 % und ganz bevorzugt von 20 % bis 30 % der Gesamtmischung verwendet.

Wenn die Flüssigkristallmedien sowohl eine oder mehrere Verbindungen der Formel IIIA, als auch eine oder mehrere Verbindungen der Formel IIIB enthält, werden die Verbindungen der Formel IIIA vorzugsweise in einer Gesamtkonzentration von 5 % bis 50 %, stärker bevorzugt von 10 % bis 45 %, stärker bevorzugt von 15 % bis 30 % und ganz bevorzugt von 20 % bis 25 % der Gesamtmischung und die Verbindungen der Formel IIIB in einer Gesamtkonzentration von 1 % bis 35 %, stärker bevorzugt von 5 % bis 30 %, stärker bevorzugt von 7 % bis 25 % und ganz bevorzugt von 10 % bis 20 % der Gesamtmischung verwendet.

Die Verbindungen der Formel I werden in dieser Ausführungsform vorzugsweise in einer Gesamtkonzentration von 1 % bis 20 %, stärker bevorzugt von 2 % bis 15 %, noch stärker bevorzugt von 3 % bis 12 % und ganz bevorzugt von 5 % bis 10 % der Gesamtmischung verwendet.

Die Flüssigkristallmedien enthalten vorzugsweise insgesamt 70 % bis 100 %, stärker bevorzugt 80 % bis 100 % und ganz bevorzugt 90 % bis 100 % und insbesondere 95 % bis 100 % der Verbindungen der Formeln I, IIIA, IIIB, und IV bis IX, vorzugsweise der Formeln I, IIIA und/oder IIIB und/oder IV, vorzugsweise bestehen sie überwiegend daraus und ganz bevorzugt bestehen sie vollständig daraus.

In einer besonders bevorzugten Ausführungsform der vorliegenden Erfindung enthalten die flüssigkristallinen Medien eine oder mehrere Verbindungen der Formel V und eine oder mehrere Verbindungen der Formel VI.

In einer weiteren besonders bevorzugten Ausführungsform der vorliegenden Erfindung enthalten die flüssigkristallinen Medien eine oder mehrere Verbindungen der Formel V und eine oder mehrere Verbindungen der Formel VII.

Ebenfalls bevorzugt enthalten die flüssigkristallinen Medien gemäß der vorliegenden Erfindung eine oder mehrere Verbindungen der Formel V, eine oder mehrere Verbindungen der Formel VI und eine oder mehrere Verbindungen der Formel VIII.

Wenn die flüssigkristallinen Medien gemäß der vorliegenden Anmeldung eine oder mehrere Verbindungen der Formel V enthalten, beträgt die Konzentration dieser Verbindungen bevorzugt insgesamt 10 bis 30 %, bevorzugt 15 bis 25 % und besonders bevorzugt 18 bis 22.

Wenn die flüssigkristallinen Medien gemäß der vorliegenden Anmeldung eine oder mehrere Verbindungen der Formel VI enthalten, beträgt die Konzentration dieser Verbindungen bevorzugt insgesamt 15 bis 35 %, bevorzugt 18 bis 30 % und besonders bevorzugt 22 bis 26 %.

Wenn die flüssigkristallinen Medien gemäß der vorliegenden Anmeldung eine oder mehrere Verbindungen der Formel VII enthalten, beträgt die Konzentration dieser Verbindungen bevorzugt insgesamt 4 bis 25 %, bevorzugt 8 bis 20 % und besonders bevorzugt 10 bis 14 %.

Wenn die flüssigkristallinen Medien gemäß der vorliegenden Anmeldung eine oder mehrere Verbindungen der

Formel VIII enthalten, beträgt die Konzentration dieser Verbindungen bevorzugt insgesamt 15 bis 35 %, bevorzugt 18 bis 30 % und besonders bevorzugt 22 bis 26 %.

Wenn die flüssigkristallinen Medien gemäß der vorliegenden Anmeldung eine oder mehrere Verbindungen der Formel IX enthalten, beträgt die Konzentration dieser Verbindungen bevorzugt insgesamt 5 bis 25 %, bevorzugt 10 bis 20 % und besonders bevorzugt 13 bis 17 %.

In der vorliegenden Anmeldung beschreibt der Ausdruck dielektrisch positiv Verbindungen oder Komponenten mit $\Delta\varepsilon > 3{,}0$, dielektrisch neutral mit $-1{,}5 \leq \Delta\varepsilon \leq 3{,}0$ und dielektrisch negativ mit $\Delta\varepsilon < -1{,}5$. $\Delta\varepsilon$ wird bei einer Frequenz von 1 kHz und 20°C bestimmt. Die dielektrische Anisotropie der jeweiligen Verbindung wird aus den Ergebnissen einer Lösung von 10 % der jeweiligen einzelnen Verbindung in einer nematischen Host-Mischung bestimmt. Wenn die Löslichkeit der jeweiligen Verbindung in der Host-Mischung weniger als 10 % beträgt, wird die Konzentration auf 5 % reduziert. Die Kapazitäten der Testmischungen werden sowohl in einer Zelle mit homeotroper als auch mit homogener Orientierung bestimmt. Die Schichtdicke beträgt bei beiden Zelltypen ca. 20 µm. Die angelegte Spannung ist eine Rechteckwelle mit einer Frequenz von 1 kHz und einem Effektivwert von typischerweise 0,5 V bis 1,0 V, wird jedoch stets so ausgewählt, dass sie unterhalb der kapazitiven Schwelle für die jeweilige Testmischung liegt.

Hierbei gelten die folgenden Definitionen.

$$\Delta\varepsilon \equiv (\varepsilon_{||} - \varepsilon_{\perp})$$

und

$$\varepsilon_{\text{Mittel}} \equiv (\varepsilon_{||} + 2\,\varepsilon_{\perp}) / 3\,.$$

Als Host-Mischung wird für dielektrisch positive Verbindungen die Mischung ZLI-4792 und für dielektrisch neutrale sowie für dielektrisch negative Verbindungen die Mischung ZLI-3086 verwendet, beide von Merck KGaA, Deutschland. Die absoluten Werte der dielektrischen Konstanten der Verbindungen werden aus der Änderung der jeweiligen Werte der Host-Mischung bei Zugabe der interessierenden Verbindungen bestimmt. Die Werte werden auf eine Konzentration der interessierenden Verbindungen von 100 % extrapoliert.

Komponenten, die bei der Messtemperatur von 20°C eine nematische Phase aufweisen, werden als solche gemessen, alle anderen werden wie Verbindungen behandelt.

Der Ausdruck Schwellenspannung bezeichnet in der vorliegenden Anmeldung die optische Schwelle und ist für 10 % relativen Kontrast ($V_{10}$) angegeben, der Ausdruck Sättigungsspannung bezeichnet die optische Sättigung und ist für 90 % relativen Kontrast ($V_{90}$) angegeben, in beiden Fällen, soweit nicht ausdrücklich etwas anderes angegeben ist. Die kapazitive Schwellenspannung ($V_0$), auch Freedericks-Schwelle $V_{Fr}$ genannt, wird nur verwendet, wenn dies ausdrücklich genannt ist.

Die in dieser Anmeldung angegebenen Parameterbereiche schließen sämtlich die Grenzwerte ein, wenn nicht ausdrücklich etwas anderes angegeben ist.

Die unterschiedlichen für verschiedene Bereiche von Eigenschaften angegebenen oberen und unteren Grenzwerte ergeben in Kombination miteinander zusätzliche bevorzugte Bereiche.

In der gesamten Anmeldung gelten, wenn nicht ausdrücklich anders angegeben, die folgenden Bedingungen und Definitionen. Alle Konzentrationen sind in Massenprozent angegeben und beziehen sich jeweils auf die Gesamtmischung, alle Temperaturen und alle Temperaturunterschiede sind in Grad Celsius bzw. Differenzgrad angegeben. Alle physikalischen Eigenschaften werden nach "Merck Liquid Crystals, Physical Properties of Liquid Crystals", Stand Nov. 1997, Merck KGaA, Deutschland, bestimmt und sind für eine Temperatur von 20°C aufgeführt, wenn nicht ausdrücklich anders angegeben. Die optische Anisotropie (An) wird bei einer Wellenlänge von 589,3 nm bestimmt. Die dielektrische Anisotropie ($\Delta\varepsilon$) wird bei einer Frequenz von 1 kHz bestimmt. Die Schwellenspannungen sowie alle anderen elektrooptischen Eigenschaften werden mit bei Merck KGaA, Deutschland, hergestellten Testzellen bestimmt. Die Testzellen für die Bestimmung von $\Delta\varepsilon$ besitzen eine Schichtdicke von circa 20 µm. Bei der Elektrode handelt es sich um eine kreisförmige ITO-Elektrode mit einer Fläche von 1,13 $cm^2$ und einem Schutzring. Die Ausrichtungsschichten sind SE-1211 von Nissan Chemicals, Japan, für homeotrope Ausrichtung ($\varepsilon_{||}$) und Polyimid AL-1054 von Japan Synthetic Rubber, Japan, für homogene Ausrichtung ($\varepsilon_{\perp}$). Die Bestimmung der Kapazitäten erfolgt mit einem Frequenzgang-Analysegerät Solatron 1260 unter Verwendung einer Sinuswelle mit einer Spannung von 0,3 $V_{rms}$. Als Licht wird bei den elektrooptischen Messungen weißes Licht verwendet. Dabei wird ein Aufbau mit einem im Handel erhältlichen Gerät DMS der Fa. Autronic-Melchers, Germany verwendet. Die charakteristischen Spannungen wurden unter senkrechter Beobachtung bestimmt. Die Schwellenspannung ($V_{10}$), "Mittgrau-Spannung" ($V_{50}$) und Sättigungsspannung ($V_{90}$) wurden für 10 %, 50 % bzw. 90 % relativen Kontrast bestimmt.

Die flüssigkristallinen Medien werden bezüglich ihrer Eigenschaften im Frequenzbereich der Mikrowellen untersucht wie in A. Penirschke, S. Müller, P. Scheele, C. Weil, M. Wittek, C. Hock und R. Jakoby: "Cavity Perturbation Method for Characterization of Liquid Crystals up to 35GHz", 34th European Microwave Conference - Amsterdam, S. 545-548 beschreiben.

Vergleiche hierzu auch A. Gaebler, F. Goelden, S. Müller, A. Penirschke und R. Jakoby "Direct Simulation of Material Permittivites ...", 12MTC 2009 - International Instrumentation and Measurement Technology Conference, Singapur, 2009 (IEEE), S. 463-467 und DE 10 2004 029 429 A, in der ebenfalls detailliert ein Meßverfahren beschrieben wird.

Der Flüssigkristall wird in eine Kapillare aus Polytetrafluorethylen (PTFE) gefüllt. Die Kapillare hat einen inneren Radius von 180 μm und einen äußeren Radius von 350 μm. Die effektive Länge beträgt 2,0 cm. Die gefüllte Kapillare wird in die Mitte der Kavität mit einer Resonanzfrequenz von 30 GHz eingebracht. Diese Kavität hat eine Länge von 6,6 mm, eine Breite von 7,1 mm und eine Höhe von 3,6 mm. Daraufhin wird das Eingangssignal ("source") angelegt und das Ergebnis des Ausgangssignals mit einem kommerziellen Netzwerkanalysator ("vector network analyzer") aufgenommen.

Aus der Änderung der Resonanzfrequenz und des Q-Faktors, zwischen der Messung mit der mit dem Flüssigkristall gefüllten Kapillare und der Messung ohne der mit dem Flüssigkristall gefüllten Kapillare, wird die dielektrische Konstante und der Verlustwinkel bei der entsprechenden Zielfrequenz mittels der Gleichungen 10 und 11 der A. Penirschke, S. Müller, P. Scheele, C. Weil, M. Wittek, C. Hock und R. Jakoby: "Cavity Perturbation Method for Characterization of Liquid Crystals up to 35GHz", 34th European Microwave Conference - Amsterdam, S. 545-548 bestimmt, wie dort beschrieben.

Die Werte für die Komponenten der Eigenschaften senkrecht bzw. and parallel zum Direktor des Flüssigkristalls werden durch Orientierung des Flüssigkristalls in einem Magnetfeld ergalten. Dazu wird das Magnetfeld eines Permanentmagneten verwendet. Die Stärke des Magnetfelds beträgt 0.35 Tesla. Die Orientierung des Magneten wird entsprechend eingestellt und dann entsprechend um 90° gedreht.

Bevorzugte Bauelemente sind Phasenschieber, Varaktoren, Funk- und Radiowellenantennenarrays, "matching circuit adaptive filters" und andere.

In der vorliegenden Anmeldung, bedeutet, wenn nicht ausdrücklich anders angegeben, der Begriff Verbindungen sowohl eine Verbindung, als auch mehrere Verbindungen.

Die erfindungsgemäßen Flüssigkristallmedien weisen in einer bevorzugten Ausführungsform nematische Phasen von jeweils mindestens von -20°C bis 80°C, bevorzugt von -30°C bis 85°C und ganz besonders bevorzugt von -40°C bis 100°C auf. Insbesondere bevorzugt reicht die Phase bis 120°C oder mehr, bevorzugt bis140°C oder mehr und ganz besonders bevorzugt bis 160°C oder mehr. Hierbei bedeutet der Begriff eine nematische Phase aufweisen einerseits, dass bei tiefen Temperaturen bei der entsprechenden Temperatur keine smektische Phase und keine Kristallisation beobachtet wird und andererseits, dass beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen, mit einer Schichtdicke von 5 μm, für mindestens 100 Stunden überprüft. Bei hohen Temperaturen wird der Klärpunkt nach üblichen Methoden in Kapillaren gemessen.

Ferner sind die erfindungsgemäßen Flüssigkristallmedien durch hohe optische Anisotropien im sichtbaren Bereich gekennzeichnet. Die Doppelbrechung bei 589 nm beträgt bevorzugt 0,20 oder mehr, besonders bevorzugt 0,25 oder mehr, besonders bevorzugt 0,30 oder mehr, besonders bevorzugt 0,40 oder mehr und ganz besonders bevorzugt 0,45 oder mehr. Außerdem beträgt die Doppelbrechung bevorzugt 0,80 oder weniger.

In einer bevorzugten Ausführungsform der vorliegenden Erfindung weisen die eingesetzten Flüssigkristallmedien eine positive dielektrische Anisotropie ($\Delta\varepsilon$) mit einem Wert, gemessen bei 1 kHz, bevorzugt von 0,5 oder mehr, stärker bevorzugt von 1 oder mehr, besonders bevorzugt von 2 oder mehr und gang besonders bevorzugt von 3 oder mehr, auf. Die dielektrische Anisotropie ist insbesondere bevorzugt 1,8 oder größer und 15,0 oder kleiner, mehr bevorzugt 2,0 oder größer und 10,0 oder kleiner, besonders bevorzugt 3,0 oder größer und 8,0 oder kleiner und ganz besonders bevorzugt 3,5 oder größer und 6,0 oder kleiner.

In einigen Ausführungsformen können jedoch auch Flüssigkristalle mit einem negativen Wert der dielektrischen Anisotropie vorteilhaft verwendet werden. In diesem Fall ist die dielektrische Anisotropie bevorzugt kleiner oder gleich -2,5, besonders bevorzugt kleiner oder gleich -4,0 und ganz besonders bevorzugt kleiner oder gleich -5,0. Besonders bevorzugt ist der Betrag von $\Delta\varepsilon$ in dieser Ausführungsform 1,5 oder größer und 15,0 oder kleiner, besonders bevorzugt 1,8 oder größer und 12,0 oder kleiner und ganz besonders bevorzugt 2,0 oder größer und 10,0 oder kleiner.

Ferner sind die erfindungsgemäßen Flüssigkristallmedien durch hohe Anisotropien im Mikrowellenbereich gekennzeichnet. Die Doppelbrechung beträgt z.B. bei ca. 8,3 GHz bevorzugt 0,14 oder mehr, besonders bevorzugt 0,15 oder mehr, besonders bevorzugt 0,20 oder mehr, besonders bevorzugt 0,25 oder mehr und ganz besonders bevorzugt 0,30 oder mehr. Außerdem beträgt die Doppelbrechung bevorzugt 0,80 oder weniger.

Die dielektrische Anisotropie im Mikrowellenbereich ist definiert als

$$\Delta\varepsilon_r \equiv (\varepsilon_{r,||} - \varepsilon_{r,\perp}) .$$

Die Modulierbarkeit bzw. Steuerbarkeit ("tunability", τ) ist definiert als

$$\tau \equiv (\Delta\varepsilon_r / \varepsilon_{r,||}) .$$

Die Materialgüte (η) ist definiert als

$$\eta \equiv (\tau / \tan \delta_{\varepsilon\ r,Max.}) ,$$

mit
dem maximalen dielektrischen Verlust

$$\tan \delta_{\varepsilon\ r,Max.} \equiv Max. \{ \tan \delta_{\varepsilon\ r,\perp} ; \tan \delta_{\varepsilon\ r,||} \} .$$

Die Materialgüte der bevorzugten Flüssigkristallmaterialien beträgt 4,5 oder mehr, bevorzugt 5 oder mehr, stärker bevorzugt 6 oder mehr, stärker bevorzugt 10 oder mehr, stärker bevorzugt 15 oder mehr, stärker bevorzugt 17 oder mehr, stärker bevorzugt 20 oder mehr, besonders bevorzugt 25 oder mehr und ganz besonders bevorzugt 30 oder mehr.

Dabei ist in der Regel die Materialgüte der Flüssigkristallmedien mit deren dielektrischer Anisotropie (z. B. bei 1 kHz) korreliert. Die Materialgüte ist bei den Flüssigkristallmedien, die eine größere dielektrische Anisotropie bei 1 kHz (Δε) haben, niedriger als bei solchen, die eine kleinere dielektrische Anisotropie bei 1 kHz (Δε) haben. Die Flüssigkristallmedien, die eine kleinere dielektrische Anisotropie bei 1 kHz (Δε) haben, sind jedoch schlechter elektrisch anzusteuern.

Bevorzugt sind gemäß der vorliegenden Erfindung deren Materialgüte entsprechend ihrer dielektrischen Anisotropien, wie in der folgenden Tabelle angegeben, charakterisiert ist.

| | Grenzen von η | | |
|---|---|---|---|
| Δε | bevorzugt | stärker bevorzugt | besonders bevorzugt |
| 0 bis < 1 | ≥ 40 | ≥ 45 | ≥ 50 |
| 1 bis < 2 | ≥ 35 | ≥ 40 | ≥ 45 |
| 2 bis < 3 | ≥ 30 | ≥ 35 | ≥ 40 |
| 3 bis < 4 | ≥ 25 | ≥ 30 | ≥ 35 |
| 4 bis < 5 | ≥ 20 | ≥ 25 | ≥ 30 |
| 5 bis < 6 | ≥ 15 | ≥ 20 | ≥ 25 |

Die bevorzugten Flüssigkristallmaterialien haben in den entsprechenden Bauteilen Phasenschiebergüten von 15°/dB oder mehr, bevorzugt von 20°/dB oder mehr, bevorzugt von 30°/dB oder mehr, bevorzugt von 40°/dB oder mehr, bevorzugt von 50°/dB oder mehr, besonders bevorzugt von 80°/dB oder mehr und ganz besonders bevorzugt von 100°/dB oder mehr.

Die eingesetzten Flüssigkristalle sind entweder Einzelsubstanzen oder Mischungen. Bevorzugt weisen sie eine nematische Phase auf.

Die Verbindungen der Formel I können nach den folgenden allgemeinen Reaktionsschemata (Reaktionsschema I bis IV) erhalten werden.
Die Verbindungen der Formel I mit i = 6, bzw. mit i = 10, werden beispielsweise vorteilhaft nach den Reaktionsschemata III bzw. IV erhalten.

## Reaktionsschema I

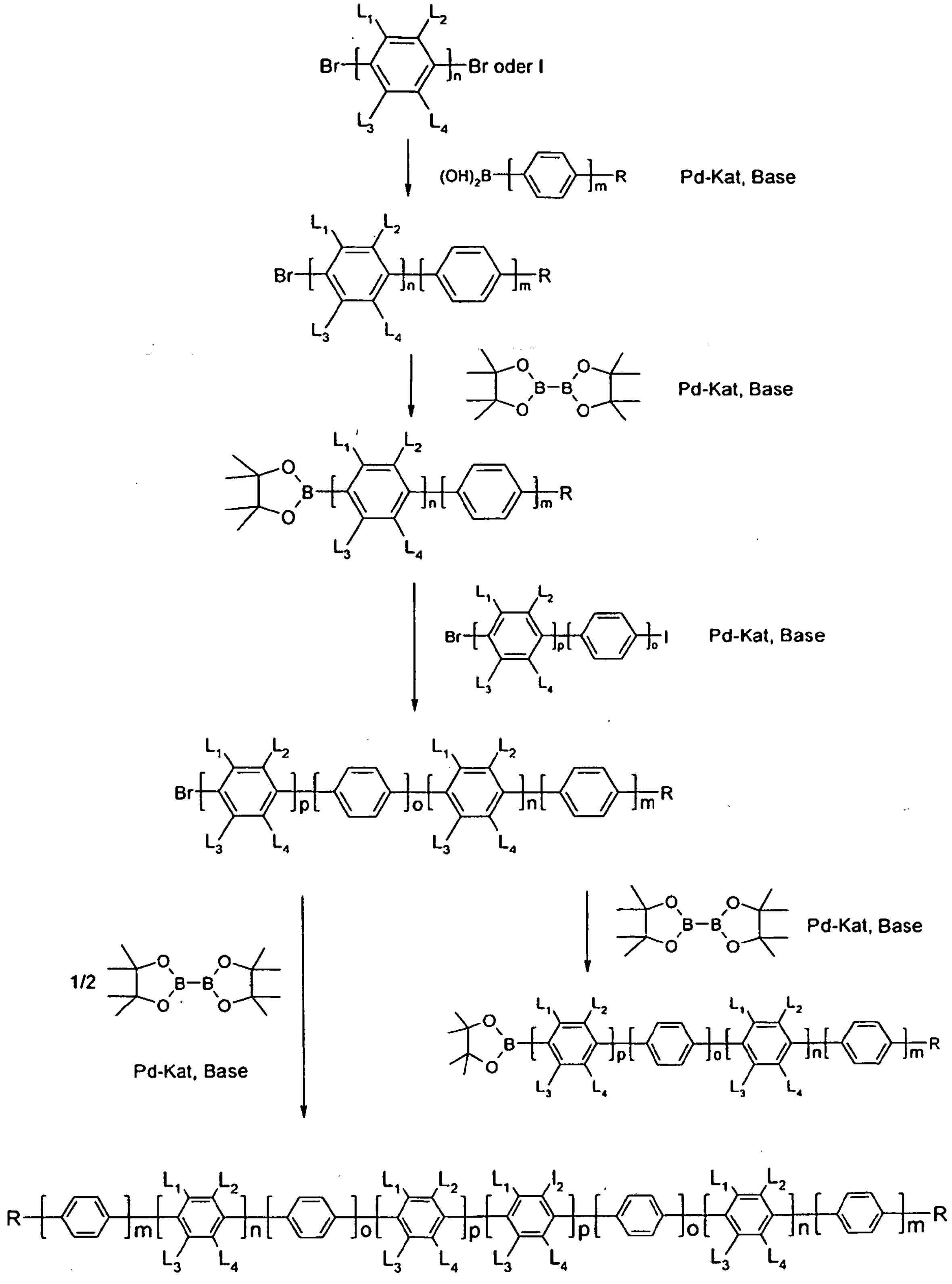

## Reaktionsschema II

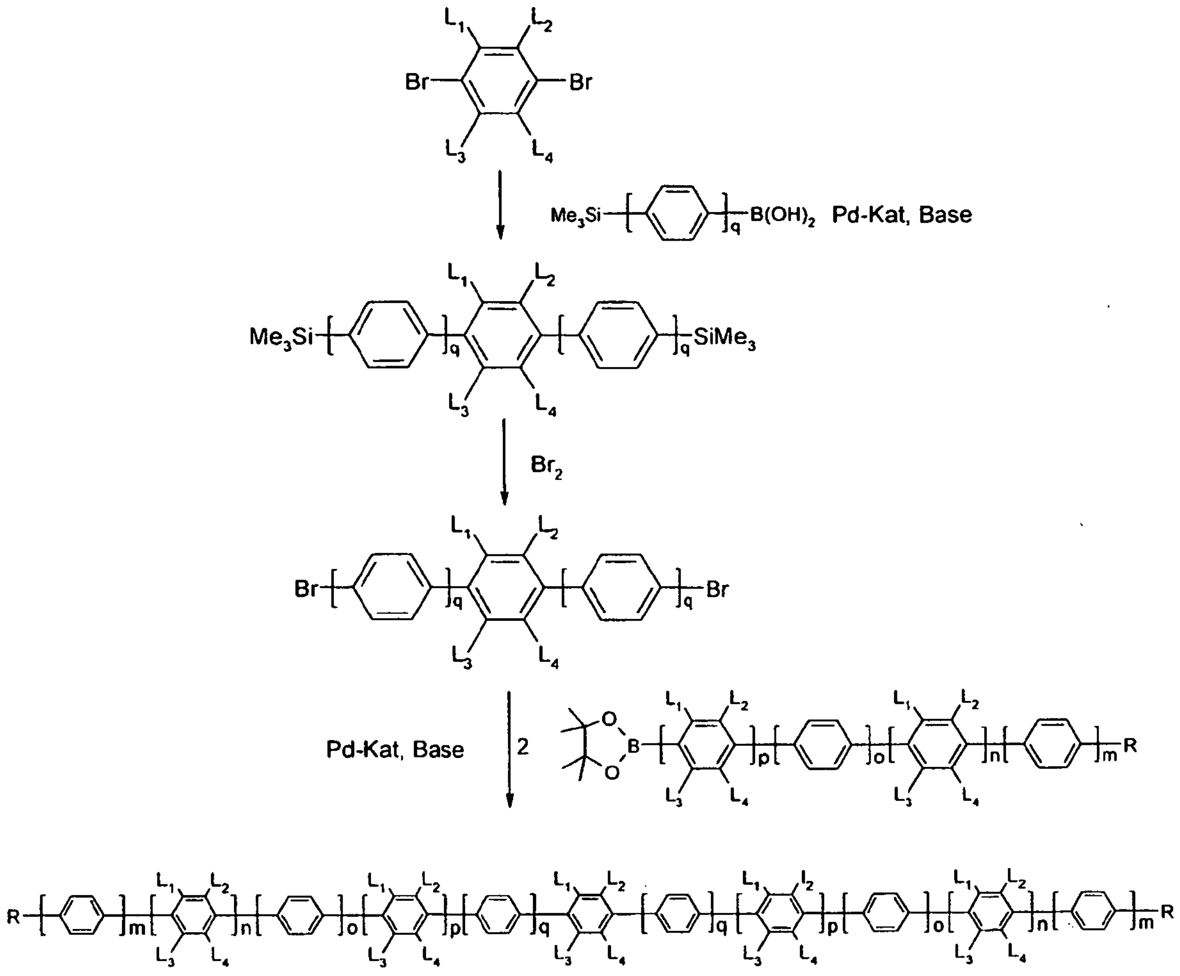


wobei in den Schemata I und II
$L_i$ jeweils unabhängig voneinander die oben bei Formel I für $L^{1i}$, also
$L_1$ die für $L^{11}$ bis
$L_4$ die für $L^{14}$, gegebenen Bedeutungen haben,
m, n, o, p und q 0, 1, oder 2 und
die Summe (m+n+o+p+q) 3 bis 7 bedeuten.

Verbindungen mit lateralem Methylsubstituenten können nach Schema I hergestellt werden. Das dafür benötigte Edukt, Bromjodtoluol, ist kommerziell erhältlich. Alternativ kann nach Analog zum folgenden Schema III vorgegangen werden. Dabei kann die zweite Stufe, die Umsetzung des Aldehyds mit der Grignard-Verbindung entfallen. Der Aldehyd wird in diesem Fall direkt zum Methyl reduziert.

Reaktionsschema III

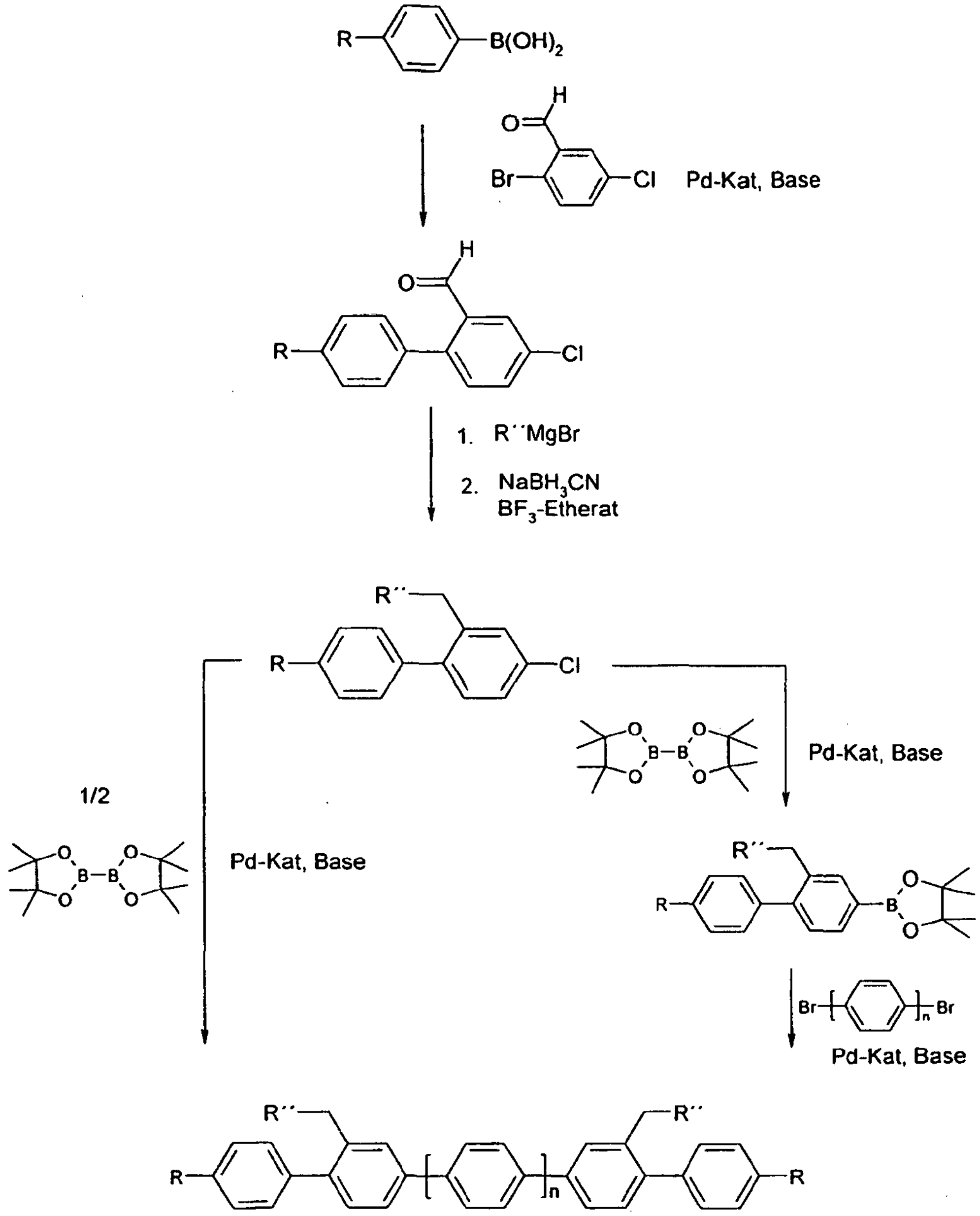


worin

R, jeweils unabhängig voneinander, $R^{11}$, wie unter Formel I definiert, bevorzugt Alkyl,
n 2 und ggf. 3, bevorzugt 2 und
R", jeweils unabhängig voneinander Alkyl, bevorzugt mit 1 bis 11, besonders bevorzugt mit 1 bis 7 und ganz besonders bevorzugt mit 2 bis 5 C-Atomen bedeutet.

Reaktionsschema IV

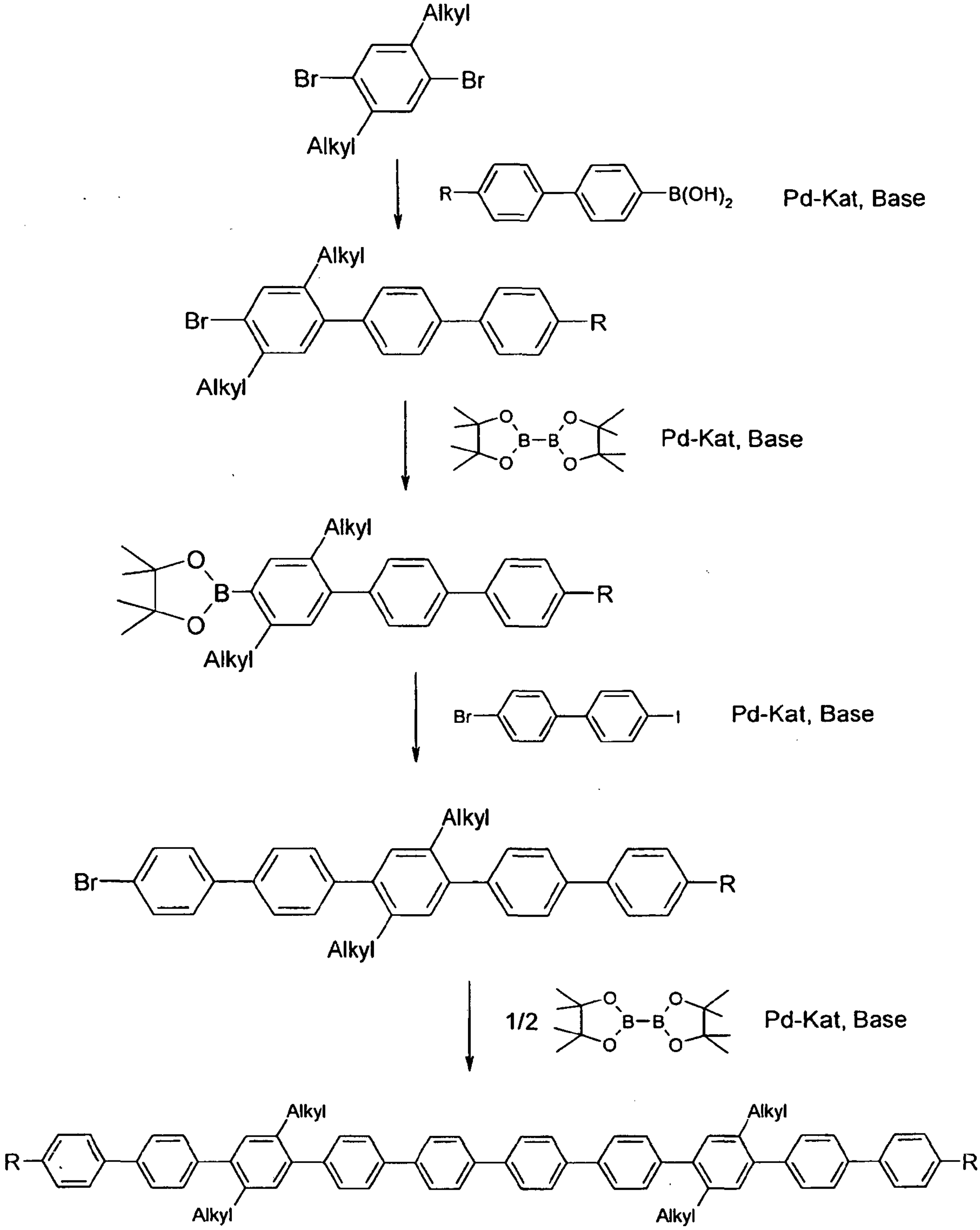


worin

R, jeweils unabhängig voneinander, $R^{11}$, wie unter Formel I definiert, bevorzugt Alkyl, und
"Alkyl" bevorzugt Alkyl mit 1 bis 15 C-Atomen, bedeuten.

Der Ausdruck "Alkyl" umfasst vorzugsweise geradkettige und verzweigte Alkylgruppen, sowie Cycloalkylgruppen, jeweils mit 1 bis 15 Kohlenstoffatomen, insbesondere die geradkettigen Gruppen Methyl, Ethyl, Propyl, Butyl, Pentyl, Hexyl und Heptyl, sowie Cyclopropyl und Cyclohexyl. Gruppen mit 2 bis 10 Kohlenstoffatomen sind im Allgemeinen bevorzugt.

Der Ausdruck "Alkenyl" umfasst vorzugsweise geradkettige und verzweigte Alkenylgruppen mit 2 bis 15 Kohlenstoffatomen, insbesondere die geradkettigen Gruppen. Besonders bevorzugte Alkenylgruppen sind $C_2$ bis $C_7$-1E-Alkenyl, $C_4$ bis $C_7$-3E-Alkenyl, $C_5$ bis $C_7$-4-Alkenyl, $C_6$ bis $C_7$-5-Alkenyl und $C_7$-6-Alkenyl, insbesondere $C_2$ bis $C_7$-1E-Alkenyl, $C_4$ bis $C_7$-3E-Alkenyl und $C_5$ bis $C_7$-4-Alkenyl. Beispiele weiterer bevorzugter Alkenylgruppen sind Vinyl, 1E-Propenyl, 1 E-Butenyl, 1 E-Pentenyl, 1 E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im Allgemeinen bevorzugt.

Der Ausdruck "Fluoralkyl" umfasst vorzugsweise geradkettige Gruppen mit endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl", bzw. Alkoxyalkyl umfasst vorzugsweise geradkettige Reste der Formel $C_nH_{2n+1}$-O-$(CH_2)_m$, worin n und m jeweils unabhängig voneinander eine ganze Zahl von 1 bis 10 bedeuten. Vorzugsweise ist n 1 und m 1 bis 6.

Verbindungen mir einer Vinyl-Endgruppe und Verbindungen mit einer Methyl-Endgruppe haben eine geringe Rotationsviskosität.

In der vorliegenden Anmeldung bedeuten sowohl Hochfrequenztechnik als auch Höchstfrequenztechnik Anwendungen mit Frequenzen im Bereich von 1 MHz bis 1 THz, bevorzugt von 1 GHz bis 500 GHz, stärker bevorzugt 2 GHz bis 300 GHz, insbesondere bevorzugt von ca. 5 bis 150 GHz und ganz besonders bevorzugt im Bereich von 10 bis 80 GHz.

Die Flüssigkristallmedien gemäß der vorliegenden Erfindung können weitere Zusatzstoffe und chirale Dotierstoffe in den üblichen Konzentrationen beinhalten. Die Gesamtkonzentration dieser weiteren Bestandteile liegt im Bereich von 0 % bis 10 %, vorzugsweise 0,1 % bis 6 %, bezogen auf die Gesamtmischung. Die Konzentrationen der einzelnen verwendeten Verbindungen liegen vorzugsweise jeweils im Bereich von 0,1 % bis 3 %. Die Konzentration dieser und ähnlicher Zusatzstoffe wird bei der Angabe der Werte und Konzentrationsbereiche der Flüssigkristallkomponenten und Flüssigkristallverbindungen der Flüssigkristallmedien in dieser Anmeldung nicht berücksichtigt.

Die erfindungsgemäßen Flüssigkristallmedien bestehen aus mehreren Verbindungen, vorzugsweise aus 3 bis 30, stärker bevorzugt aus 4 bis 20 und ganz bevorzugt aus 4 bis 15 Verbindungen. Diese Verbindungen werden auf herkömmliche Weise gemischt. In der Regel wird die gewünschte Menge der in der geringeren Menge verwendeten Verbindung in der in der größeren Menge verwendeten Verbindung gelöst. Liegt die Temperatur über dem Klärpunkt der in der höheren Konzentration verwendeten Verbindung, ist die Vervollständigung des Lösungsvorgangs besonders leicht zu beobachten. Es ist jedoch auch möglich, die Medien auf anderen üblichen Wegen, beispielsweise unter Verwendung von so genannten Vormischungen, bei denen es sich z.B. um homologe oder eutektische Mischungen von Verbindungen handeln kann, oder unter Verwendung von so genannten "Multi-Bottle"-Systemen, deren Bestandteile selbst gebrauchsfertige Mischungen sind, herzustellen.

Alle Temperaturen, wie z.B. der Schmelzpunkt T(K,N) bzw. T(K,S), der Übergang von der smektischen (S) zur nematischen (N) Phase T(S,N) und der Klärpunkt T (N,I) der Flüssigkristalle sind in Grad Celsius angegeben. Alle Temperaturdifferenzen sind in Differenzgraden angegeben.

In der vorliegenden Erfindung und insbesondere in den folgenden Beispielen sind die Strukturen der mesogenen Verbindungen durch Abkürzungen angegeben, die auch als Akronyme bezeichnet werden. In diesen Akronymen sind die chemischen Formeln unter Verwendung der folgenden Tabellen A bis C wie folgt abgekürzt. Alle Gruppen $C_nH_{2n+1}$, $C_mH_{2m+1}$ und $C_lH_{2l+1}$ bzw. $C_nH_{2n-1}$, $C_nH_{2m-1}$ und $C_lH_{2l-1}$ bedeuten geradkettiges Alkyl bzw. Alkenyl, vorzugsweise 1-E-Alkenyl, jeweils mit n, m bzw. l C-Atomen, wobei n, m und l unabhängig voneinander eine ganze Zahl von 1 bis 9, bevorzugt bis 7 bzw. von 2 bis 9, bevorzugt bis 7 bedeuten. $C_oH_{2o+1}$ bedeutet geradkettiges Alkyl mit 1 bis 7, bevorzugt bis 4 C-Atomen, oder verzweigtes Alkyl mit 1 bis 7, bevorzugt bis 4 C-Atomen.

In der Tabelle A werden die für die Ringelemente der Kernstrukturen der Verbindungen verwendeten Codes aufgeführt, während in der Tabelle B die Verknüpfungsgruppen gezeigt sind. Tabelle C gibt die Bedeutungen der Codes für die Endgruppen der linken bzw. rechten Seite. In Tabelle D sind Beispielstrukturen von Verbindungen mit ihren jeweiligen Abkürzungen zusammengestellt.

**<u>Tabelle A: Ringelemente</u>**

| | | | |
|---|---|---|---|
| C | 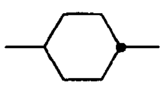 | P | 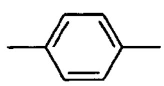 |
| D | 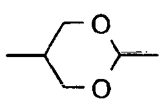 | DI | 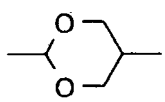 |

(fortgesetzt)

| | | | |
|---|---|---|---|
| **A** | 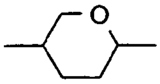 | **AI** | 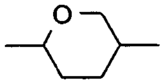 |
| **G** | 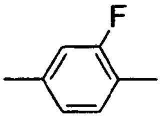 | **GI** | 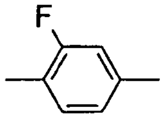 |
| **U** | 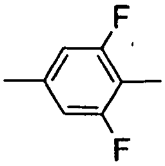 | **UI** | 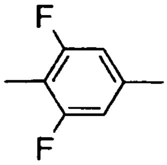 |
| **Y** | 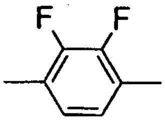 | | |
| **fX** | 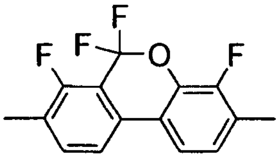 | **fXI** | 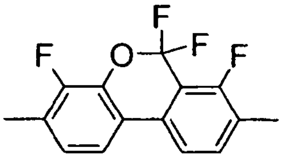 |
| **M** | 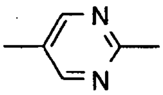 | **MI** | 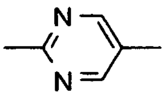 |
| **N** | 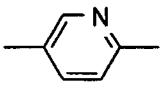 | **NI** | 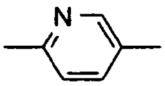 |
| **fN** | 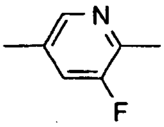 | **fNI** | 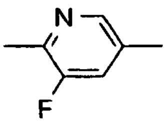 |
| **dH** | 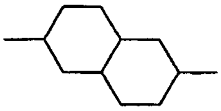 | **Np** | 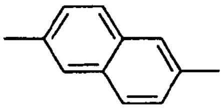 |
| **N3f** | 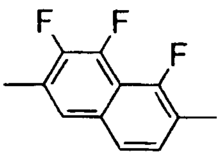 | **N3fI** | 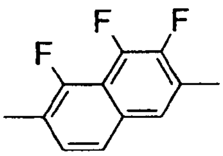 |
| **tH** | 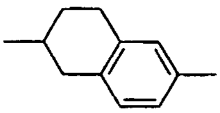 | **tHI** | 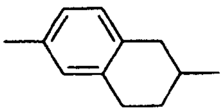 |
| **tH2f** | 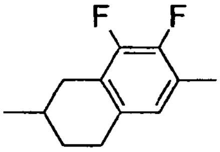 | **tH2fI** | 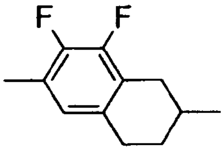 |
| **(1,4N)** | 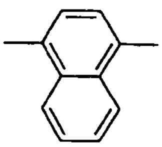 | | |

(fortgesetzt)

| | | | |
|---|---|---|---|
| **K** | 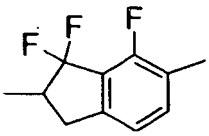 | **KI** | 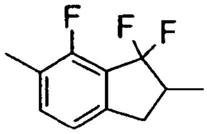 |
| **L** | 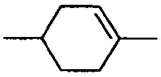 | **LI** | 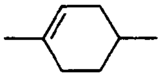 |
| **F** | 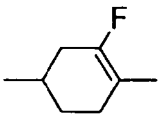 | **FI** | 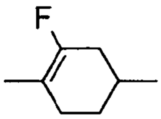 |
| **P(o)** | 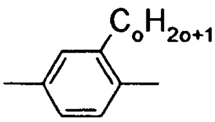 | **PI(o)** | 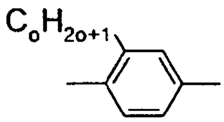 |
| **P(c3)** | 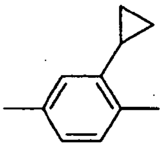 | **PI(c3)** | 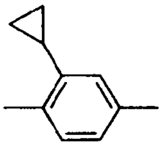 |
| **P(c4)** | 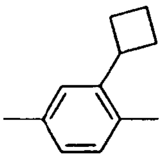 | **PI(c4)** | 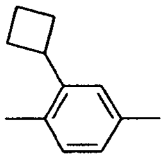 |
| **P(c5)** | 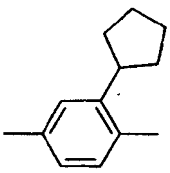 | **PI(c5)** | 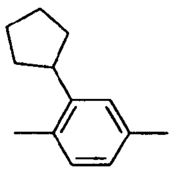 |
| **P(c6)** | 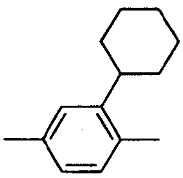 | **PI(c6)** | 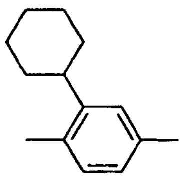 |

**Tabelle B: Verknüpfungsgruppen**

| | | | |
|---|---|---|---|
| **E** | $-CH_2CH_2-$ | **Z** | -CO-O- |
| **V** | -CH=CH- | **ZI** | -O-CO- |
| **X** | -CF=CH- | **O** | $-CH_2-O-$ |
| **XI** | -CH=CF- | **OI** | $-O-CH_2-$ |
| **B** | -CF=CF- | **Q** | $-CF_2-O-$ |
| **T** | -C≡C- | **QI** | $-O-CF_2-$ |
| **W** | $-CF_2CF_2-$ | **T** | -C≡C- |

**Tabelle C: Endgruppen**

| **Linke Seite** | | **Rechte Seite, Verwendung allein** | |
|---|---|---|---|
| **-n-** | $C_nH_{2n+1}-$ | **-n** | $--C_nH_{2n+1}$ |
| **-nO-** | $C_nH_{2n+1}-O-$ | **-nO** | $-O-C_nH_{2n+1}$ |

(fortgesetzt)

| Linke Seite | | Rechte Seite, Verwendung allein | |
|---|---|---|---|
| **-V-** | $CH_2=CH-$ | **-V** | $-CH=CH_2$ |
| **-nV-** | $C_nH_{2n+1}-CH=CH-$ | **-nV** | $-C_nH_{2n}-CH=CH_2$ |
| **-Vn-** | $CH_2=CH-C_nH_{2n+1}-$ | **-Vn** | $-CH=CH-C_nH_{2n+1}$ |
| **-nVm-** | $C_nH_{2n+1}-CH=CH-C_mH_{2m}-$ | **-nVm** | $-C_nH_{2n}-CH=CH-C_mH_{2m+1}$ |
| **-N-** | N=C- | **-N** | -C≡N |
| **-S-** | S=C=N- | **-S** | -N=C=S |
| **-F-** | F- | **-F** | -F |
| **-CL-** | Cl- | **-CL** | -Cl |
| **-M-** | $CFH_2-$ | **-M** | $-CFH_2$ |
| **-D-** | $CF_2H-$ | **-D** | $-CF_2H$ |
| **-T-** | $CF_3-$ | **-T** | $-CF_3$ |
| **-MO-** | $CFH_2O-$ | **-OM** | $-OCFH_2$ |
| **-DO-** | $CF_2HO-$ | **-OD** | $-OCF_2H$ |
| **-TO-** | $CF_3O-$ | **-OT** | $-OCF_3$ |
| **-OXF-** | $CF_2=CH-O-$ | **-OXF** | $-O-CH=CF_2$ |
| **-A-** | H-C=C- | **-A** | -C≡C-H |
| **-nA-** | $C_nH_{2n+1}-C\equiv C-$ | **-An** | $-C\equiv C-C_nH_{2n+1}$ |
| **-NA-** | N≡C-C≡C- | **-AN** | -C=C-C=N |
| | **Verwendung zusammen mit anderen** | | |
| **-...A...-** | -C≡C- | **-...A...** | -C≡C- |
| **-...V...-** | CH=CH- | **-...V...** | -CH=CH- |
| **-...Z...-** | -CO-O- | **-...Z...** | -CO-O- |
| **-...ZI...-** | -O-CO- | **-...ZI...** | -O-CO- |
| **-...K...-** | -CO- | **-...K...** | -CO- |
| **-...W...-** | -CF=CF- | **-...W...** | -CF=CF- |

worin n und m jeweils ganze Zahlen bedeuten und die drei Punkte "..." Platzhalter für andere Abkürzungen aus dieser Tabelle sind.

In der folgenden Tabelle werden Beispielstrukturen zusammen mit ihren jeweiligen Abkürzungen angegeben. Diese werden gezeigt, um die Bedeutung der Regeln für die Abkürzungen zu demonstrieren. Weiterhin stellen sie Verbindungen dar, die vorzugsweise verwendet werden.

**Tabelle D: Beispielstrukturen**

Die Beispielstrukturen zeigen besonders bevorzugt eingesetzte Verbindungen.

Beispiele für Verbindungen der *Komponente A*

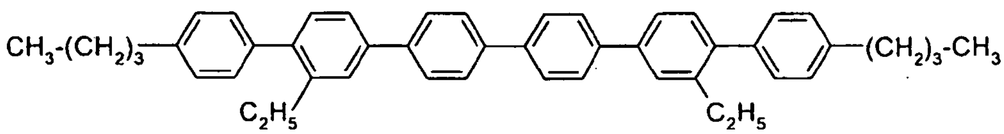

**6*P-1,** Phasensequenz: k 197°C N 330,4°C I

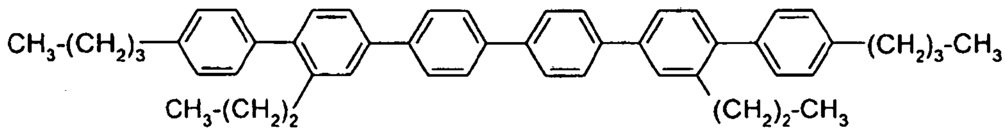

**6*P-2,** Phasensequenz: K 174°C N 252,7°C I

(fortgesetzt)

Die Beispielstrukturen zeigen besonders bevorzugt eingesetzte Verbindungen.

Beispiele für Verbindungen der *Komponente A*

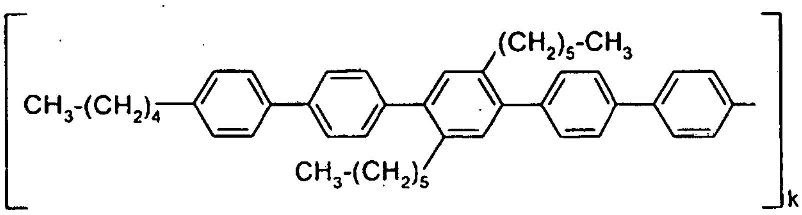

**10*P-1** (k = 2), Phasensequenz: K 154°C N 283,9°C I

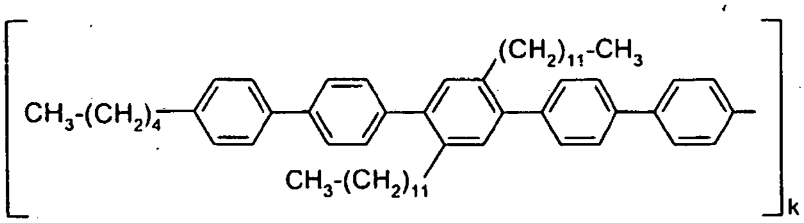

**10*P-2** (k = 2), Phasensequenz: K 103°C N 199,9°C I

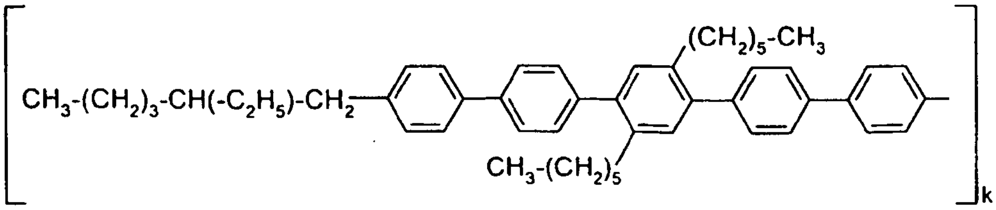

**10°P-3** k = 2), Phasensequenz: K 147°C N 238,4°C I

Beispiele für Verbindungen der *Komponente B*

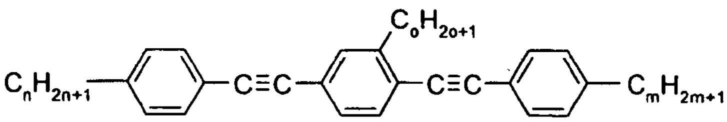

**PTP(o)TP-n-m**

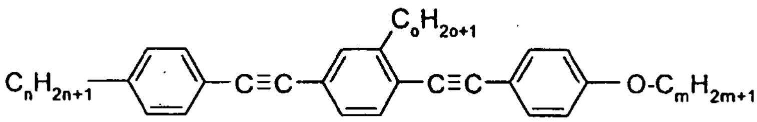

**PTP(o)TP-n-m**

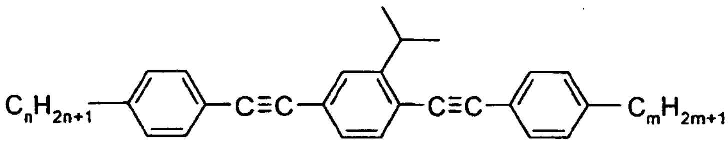

**PTP(i3)TP-n-m**

Phasensequenz: n=m=2; K 99°C N (45,4)°C I; n=m=3 $T_g$ -30°C K 68°C N 76,2C I; n=m=4: K 44°C N 49,5°C I

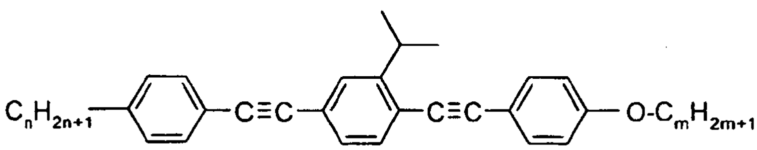

**PTP(i3)TP-n-Om**

(fortgesetzt)

Beispiele für Verbindungen der *Komponente B*

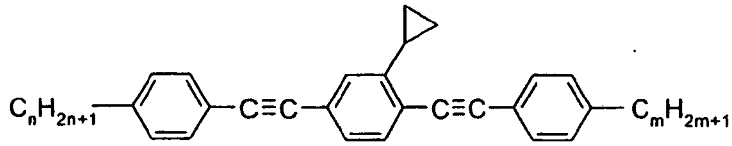

**PTP(c3)TP-n-Om**

Phasensequenz: n=m=3. $T_g$ -43°C N 86,0°C I; n=m=4; K 72°C N 84,5°C I

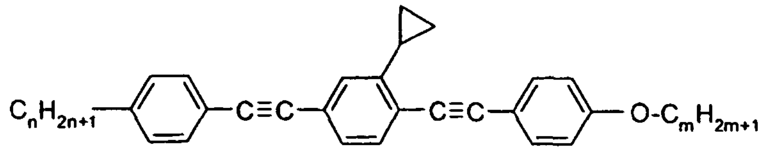

**PTP(c3)TP-n-Om**

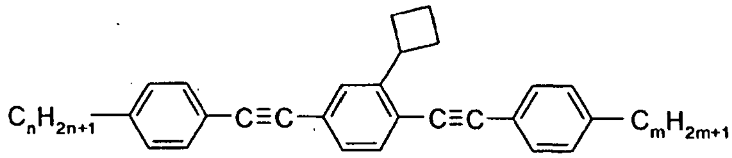

**PTP(c4)TP-n-m**

Phasensequenz: n=m=4 : $T_g$ -39°C K 69° C N 70,1°C I

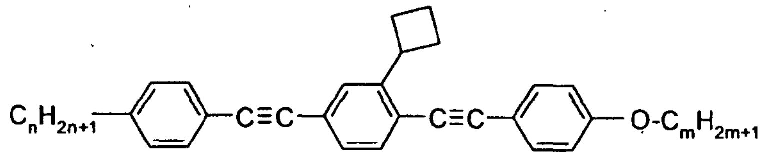

**PTP(c4)TP-n-Om**

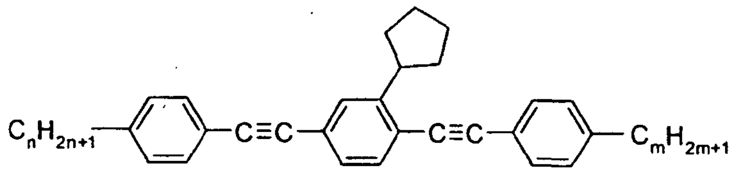

**PTP(c5)TP-n-Om**

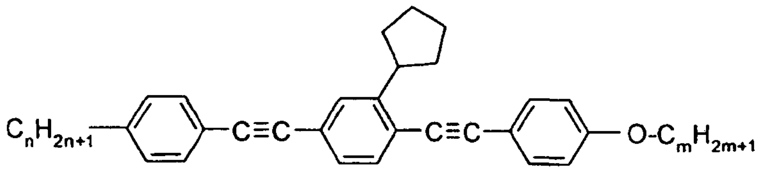

**PTP(c5)TP-n-Om**

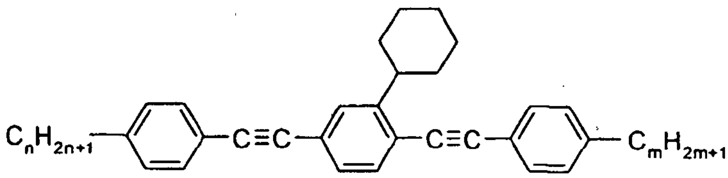

**PTP(c6)TP-n-Om**

Phasensequenz: n=m= 3: $T_g$ -23°C I

(fortgesetzt)

Beispiele für Verbindungen der *Komponente B*

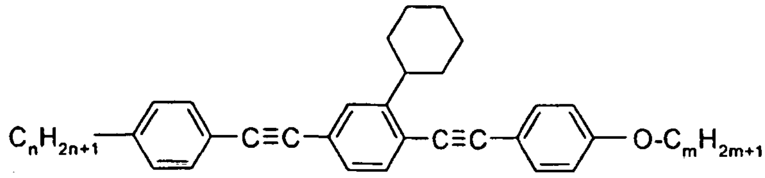

**PTP(c6)TP-n-Om**

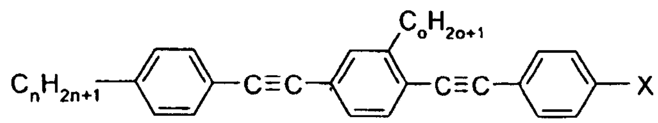

**PTP(o)TP-n-X, X=F, Cl**

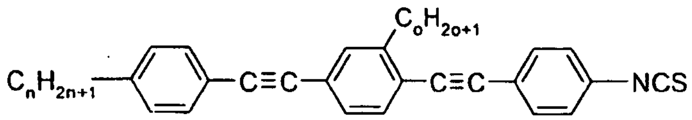

**PTP(o)TP-n-S**

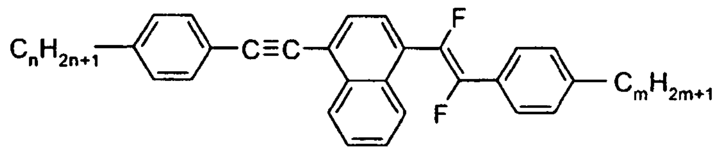

**PT(1,4N)BP-n-m**

Phasensequez: n=3, m=4: $T_g$ -34°C K 67°C N 180,6°C I; n=m=4: Tg -37 °C K 65°C N 162,0°C I

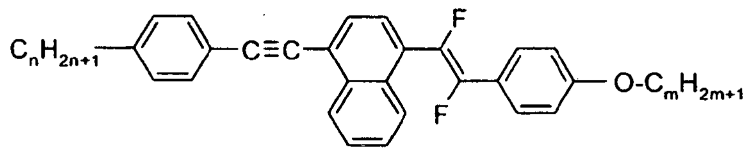

**PT(1,4N)BP-n-Om**

Beispiele für Verbindungen der *Komponente C*

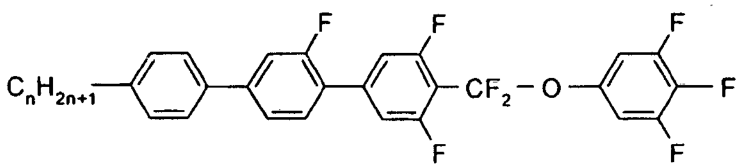

**PGUQU-n-F**

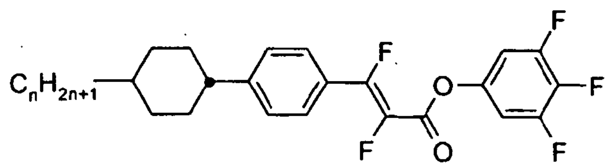

**CPBZU-u-F**

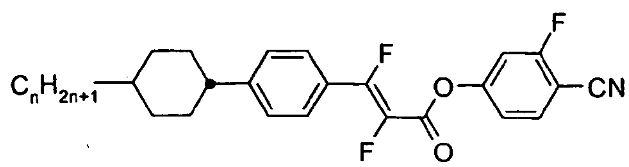

**CPBZG-n-N**

(fortgesetzt)

Beispiele für Verbindungen der *Komponente C*

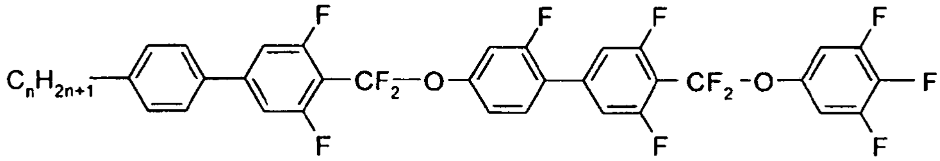

**PUQGUQU-n-F**

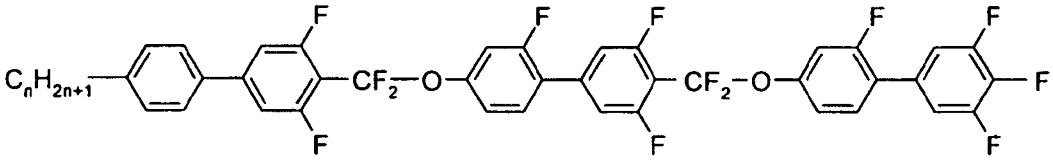

**PUQGUQGU-n-F = PU[QGU]$_2$-n-F**

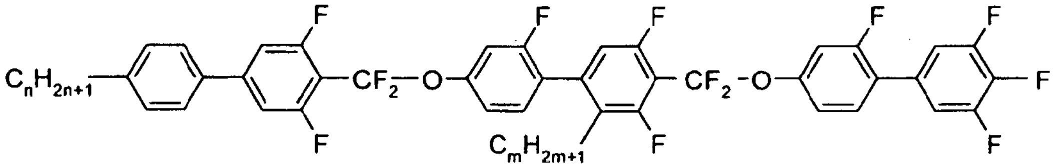

**PUQGU(m)QGU-n-f = PU[QGU]$_2^{(m)}$-n-F**

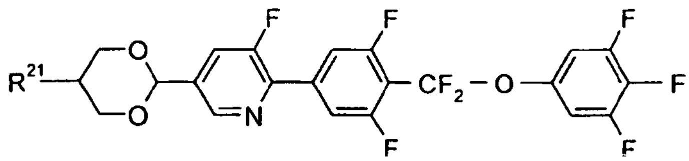

**D(fN)UQU-n-F**

Beispiele für Verbindungen der *Komponente D*

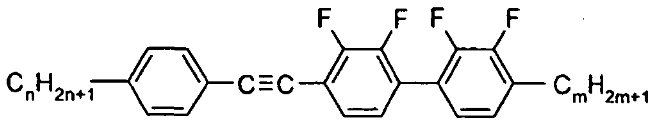

**PTYY-n-m**

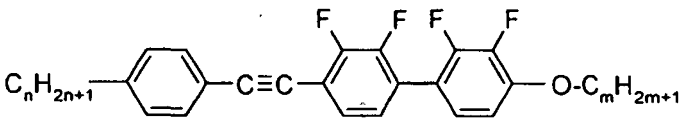

**PTYY-n-Om**

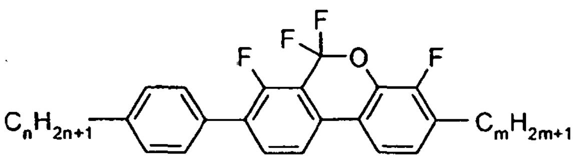

**PIX-n-m**

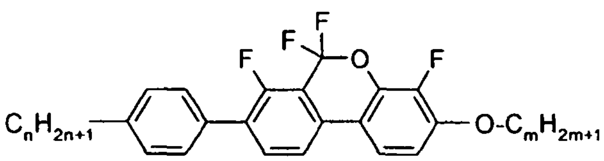

**PfX-n-Om**

Beispiele für Verbindungen der *Komponente E*

Verbindungen mit drei 6-gliedrigen Ringen

(fortgesetzt)

Beispiele für Verbindungen der *Komponente E*

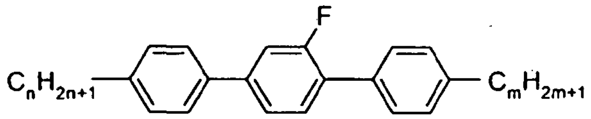

**PGP-n-m**

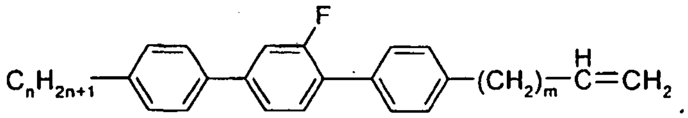

**PGP-n-mV**

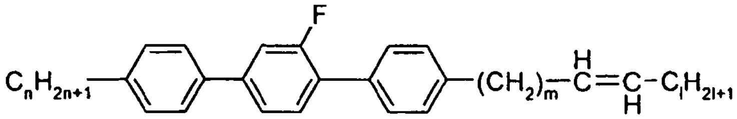

**PGP-n-mVl**

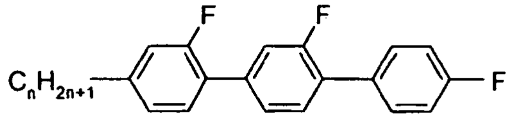

**GGP-n-F**

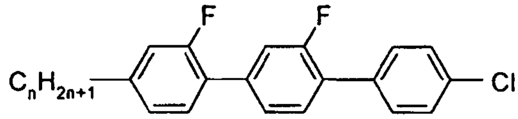

**GGP-n-CL**

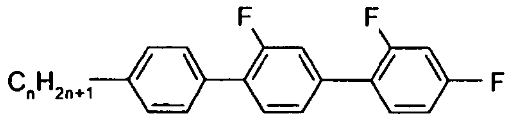

**PGIGI-n-F**

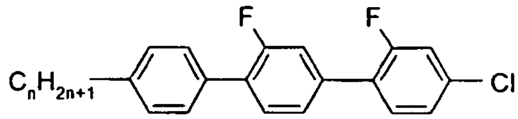

**PGIGI-n-CL**

Verbindungen mit vier 6-gliedrigen Ringen

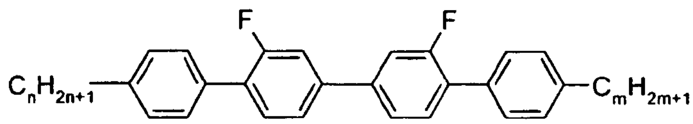

**PGIGP-n-m**

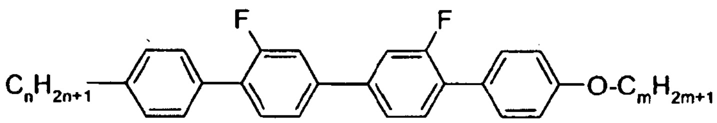

**PGIGP-n-Om**

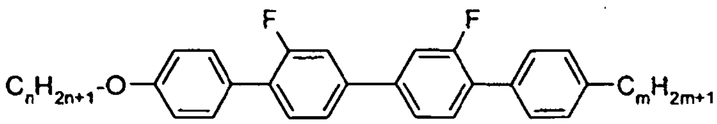

**PGIGP-nO-m**

(fortgesetzt)

Beispiele für Verbindungen der *Komponente E*

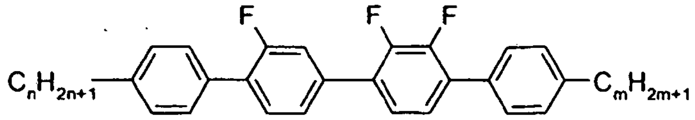

**PGIYP-n-m**

Beispielstrukturen eingesetzter polarer Verbindungen:

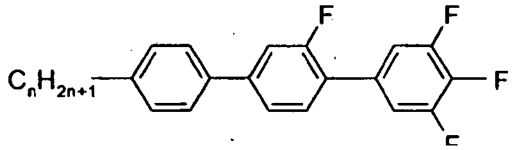

**PGU-n-F**

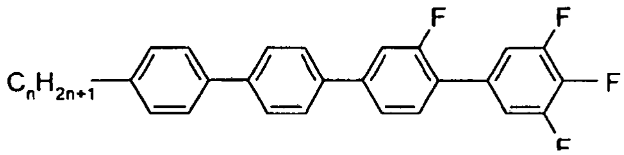

**PPGU-n-F**

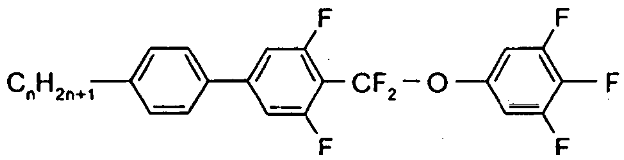

**PUQU-n-F**

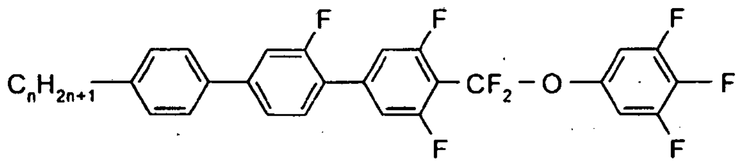

**PGUQU-n-F**

Beispielstrukturen weiterer bevorzugt eingesetzter neutraler Verbindungen:

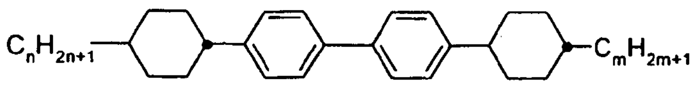

**CPPC-n-m**

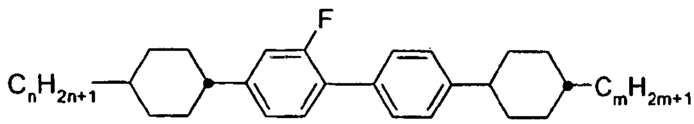

**CGPC-n-m**

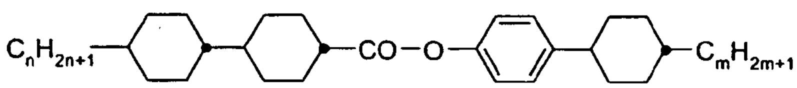

**CCZPC-n-m**

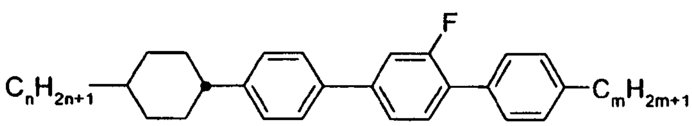

**CPGP-n-m**

(fortgesetzt)

Beispiele für Verbindungen der *Komponente E*

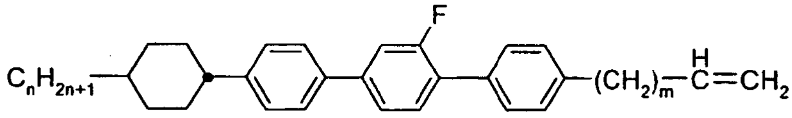

**CPGP-n-nV**

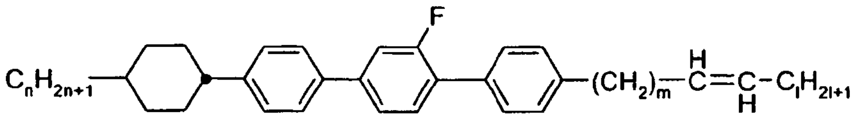

**CPGP-n-mVI**

Beispielstrukturen weiterer eingesetzter polarer Verbindungen:

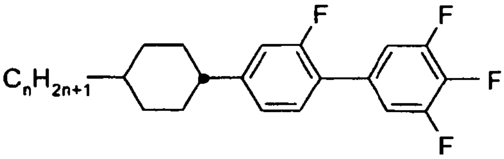

**CGU-n-F**

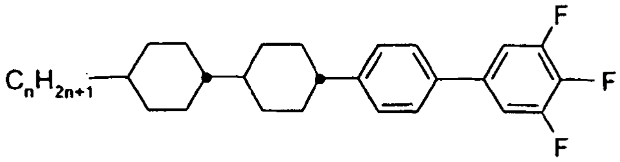

**CCPU-n-F**

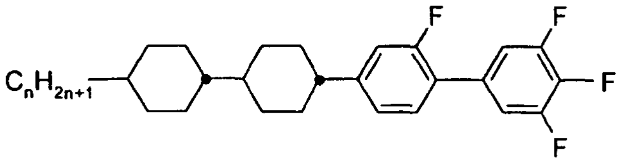

**CCGU-n-F**

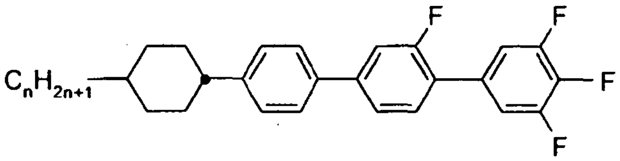

**CPGU-n-F**

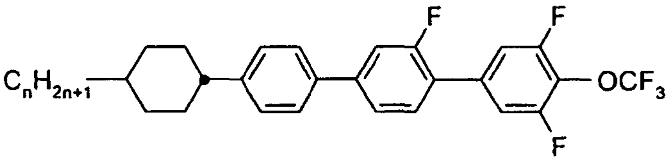

**CPGU-n-OT**

In der folgenden Tabelle, Tabelle E, sind Beispielverbindungen zusammengestellt, die in den mesogenen Medien gemäß der vorliegenden Erfindung als Stabilisator verwendet werden können. Die Gesamtkonzentration dieser bzw. ähnlicher Verbindungen in den Medien beträgt bevorzugt 5 % oder weniger.

**Tabelle E**

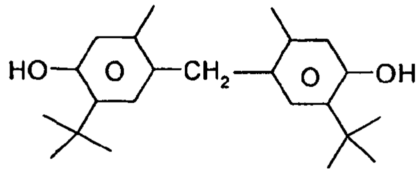

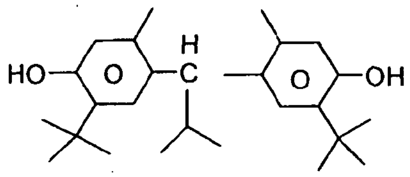

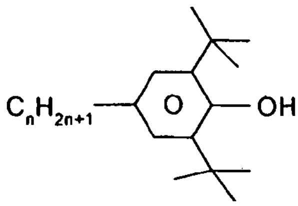

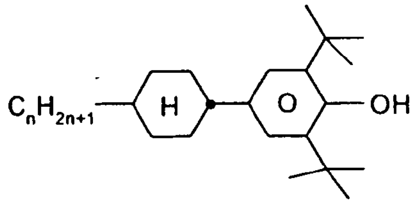

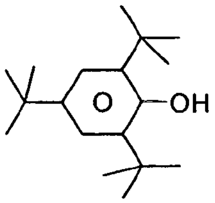

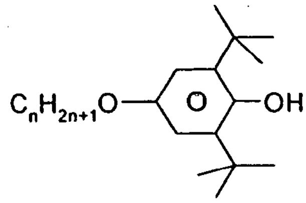

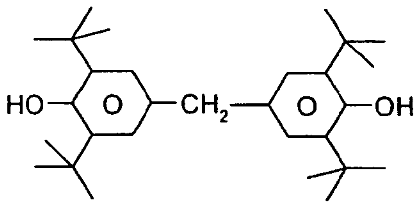

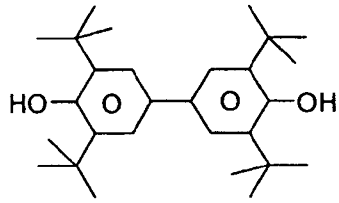

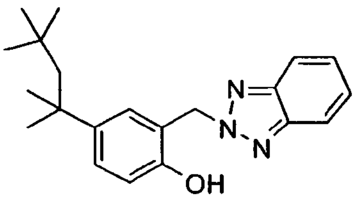

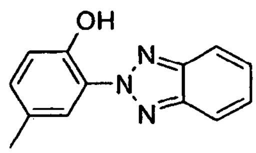

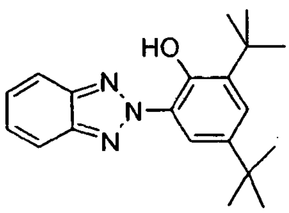

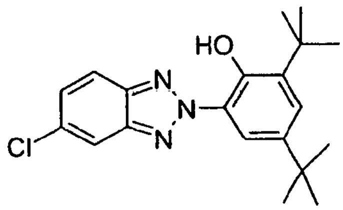

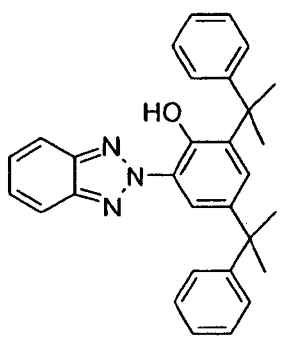

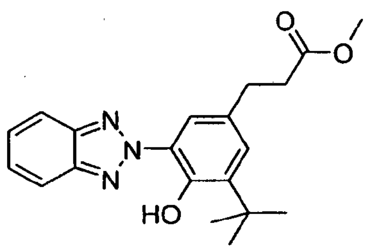

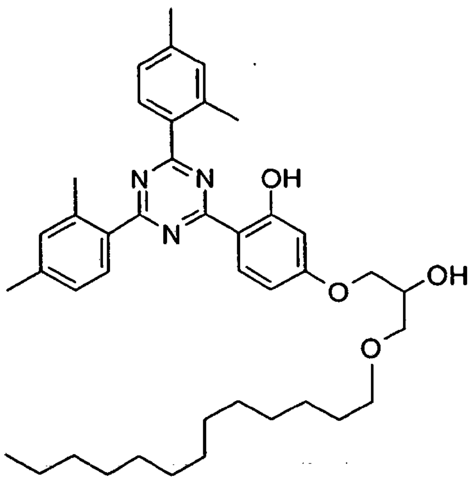

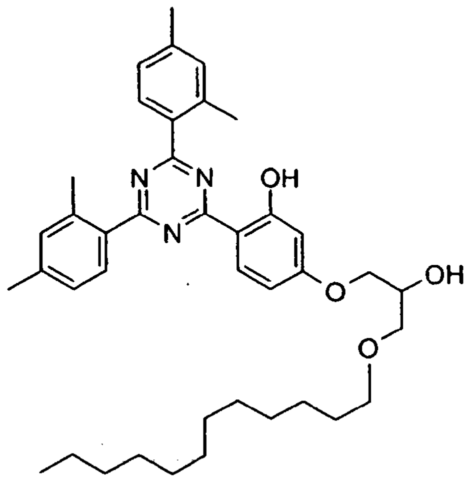

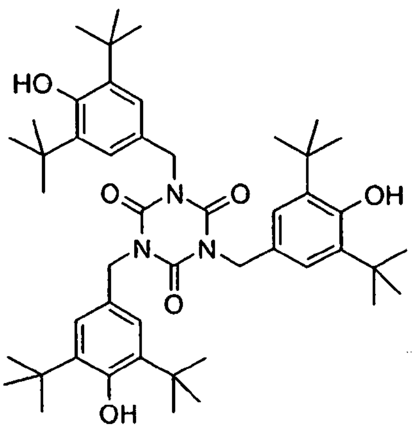

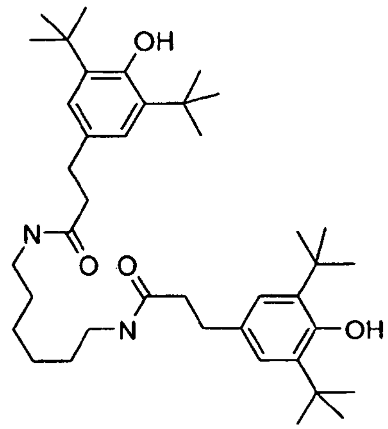

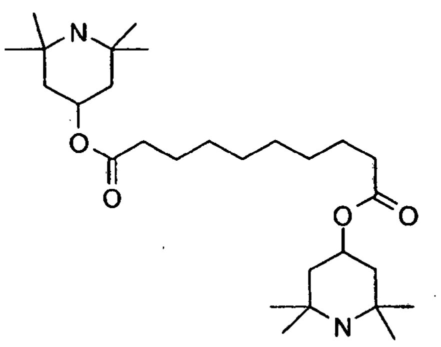

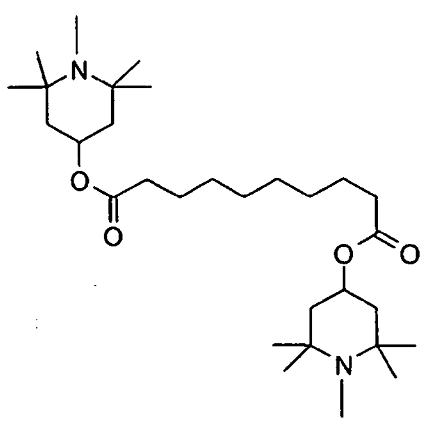

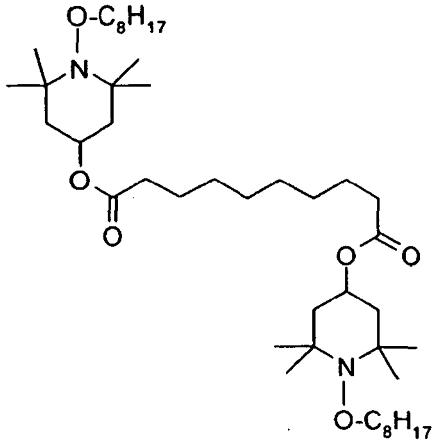

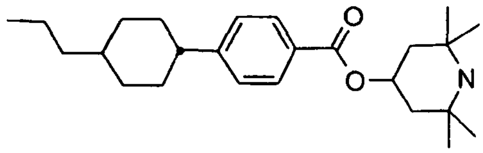

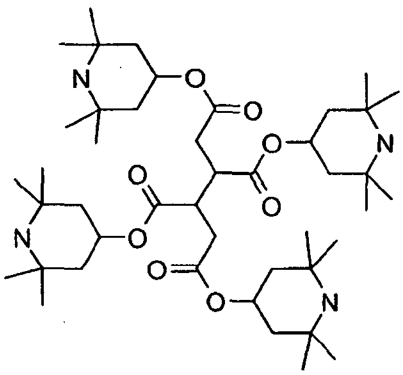

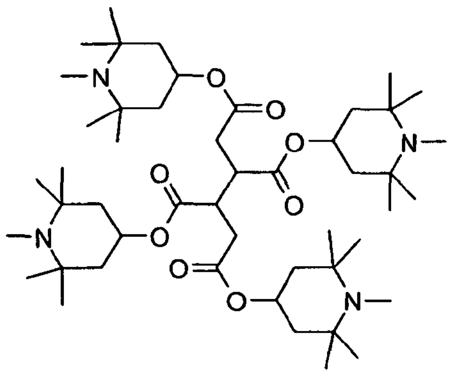

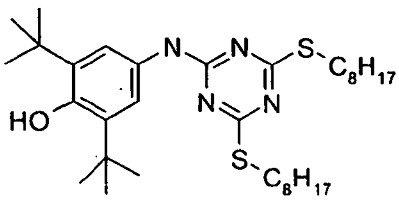

In einer bevorzugten Ausführungsform der vorliegenden Erfindung enthalten die mesogenen Medien eine oder mehrere Verbindungen ausgewählt aus der Gruppe der Verbindungen der Tabelle E.

In der folgenden Tabelle, Tabelle F, sind Beispielverbindungen zusammengestellt, die in den mesogenen Medien gemäß der vorliegenden Erfindung vorzugsweise als chirale Dotierstoffe verwendet werden können.

## Tabelle F

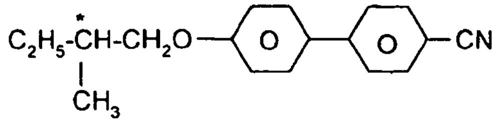

**C 15**

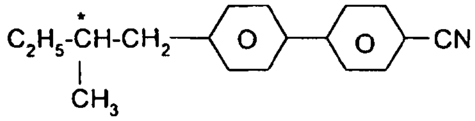

**CB 15**

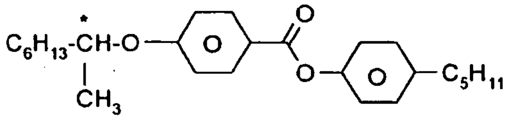

**CM 21**

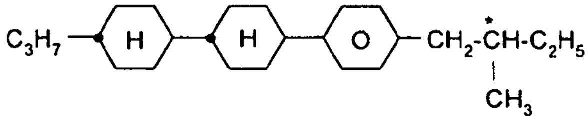

**CM 44**

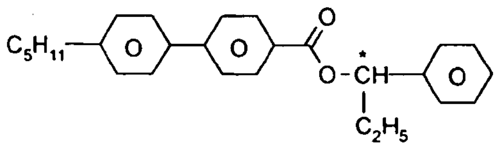

**CM 45**

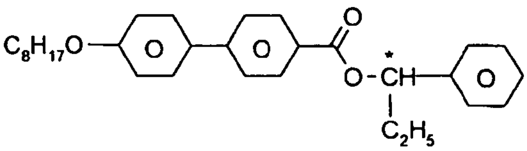

**CM 47**

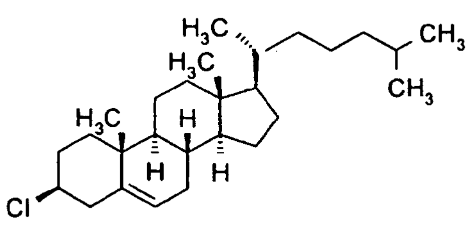

**CC**

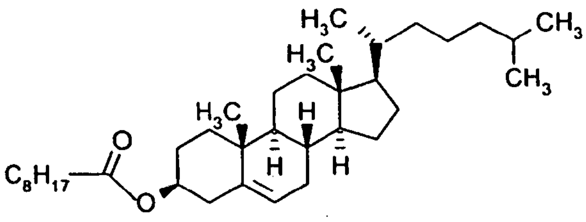

**CN**

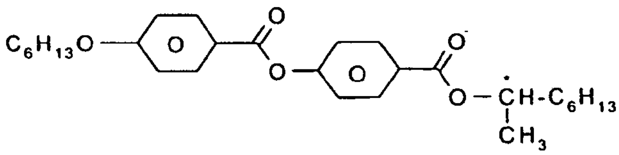

**R/S-811**

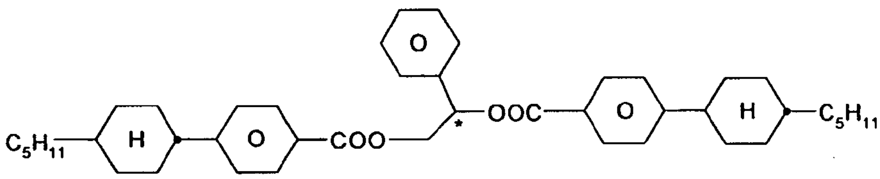

**R/S-1011**

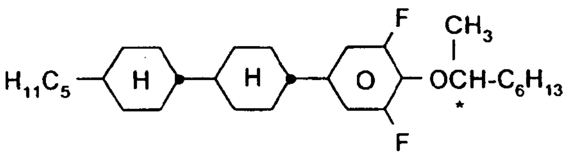

**R/S-2011**

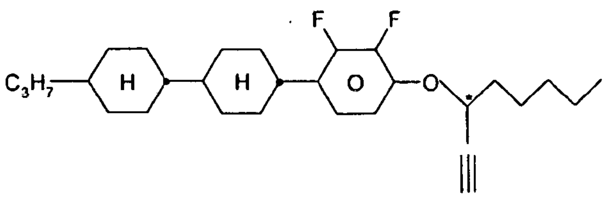

**R/S-3011**

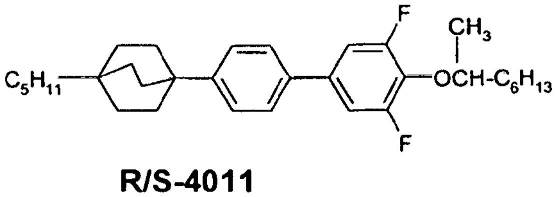

**R/S-4011**

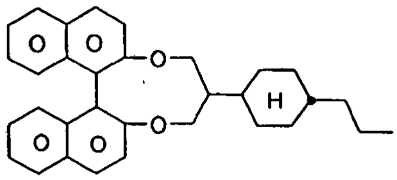

**R/S-5011**

In einer bevorzugten Ausführungsform der vorliegenden Erfindung enthalten die mesogenen Medien eine oder mehrere Verbindungen ausgewählt aus der Gruppe der Verbindungen der Tabelle F.

Vorzugsweise enthalten die mesogenen Medien gemäß der vorliegenden Anmeldung zwei oder mehr, vorzugsweise vier oder mehr, Verbindungen ausgewählt aus der Gruppe bestehend aus den Verbindungen der obigen Tabellen.

Die Flüssigkristallmedien gemäß der vorliegenden Erfindung enthalten vorzugsweise

- sieben oder mehr, vorzugsweise acht oder mehr Verbindungen, vorzugsweise Verbindungen mit drei oder mehr, vorzugsweise vier oder mehr unterschiedlichen Formeln, ausgewählt aus der Gruppe der Verbindungen der Tabelle D.

Beispiele

Die folgenden Beispiele erläutern die vorliegende Erfindung, ohne sie in irgendeiner Weise zu beschränken.

Aus den physikalischen Eigenschaften wird dem Fachmann jedoch deutlich, welche Eigenschaften zu erzielen sind und in welchen Bereichen sie modifizierbar sind. Insbesondere ist also die Kombination der verschiedenen Eigenschaften, die vorzugsweise erreicht werden können, für den Fachmann gut definiert.

Substanzbeispiele

Substanzbeispiel 1

Herstellung der Verbindung

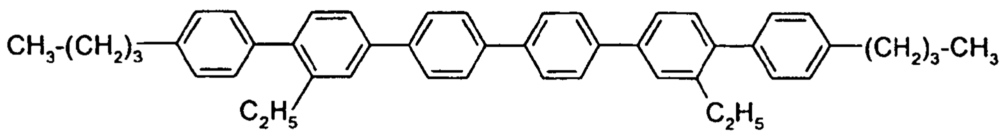

Schritt 1.1

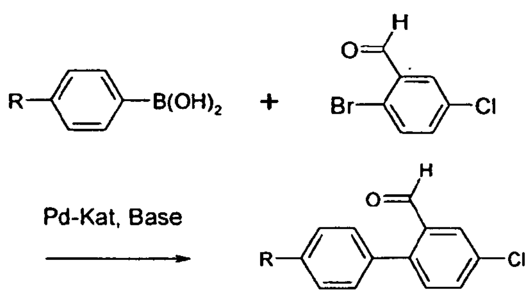


24,0 g Natriummetaborat wird in 60,0 ml vollentsalztem (VE-) Wasser gelöst. Dann werden nacheinander 1,50 g Bis(triphenylphosphin)-palladium(II)-chlorid (15,2 % Pd) zur Synthese, 0,100 ml Hydraziniumhydroxid (ca. 100 %-ig)

und 25,0 g 2-Brom-5-chlorbenzaldehyd zugegeben und 10 min bei Umgebungstemperatur gerührt. Dann werden 19,7 g des Borats gelöst in 120,0 ml Tetrahydrofuran tropfenweise zugegeben und das Gemisch zum Sieden erhitzt. Es wird für 16 h unter Rückfluß erhitzt. Das Produkt wird wie üblich aufgereinigt. Es wird als gelbes Öl erhalten.

Schritt 1.2

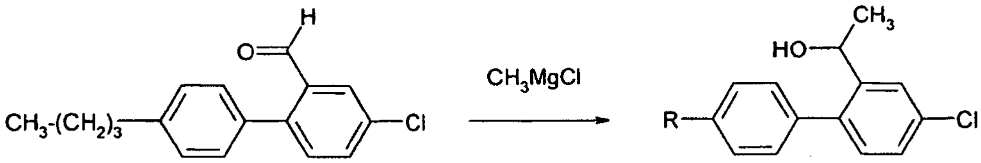


18,0 ml 20 %-ige Lösung von Methylmagnesiumchlorid in Tetrahydrofuran werden vorgelegt. 11,2 g des Produkts des letzten Schritts werden in 100 ml THF gelöst und unter Kühlung bei einer Temperatur von ca. 5°C tropfenweise zugegeben. Das Reaktionsgemisch wird anschließend 1 h lang bei einer Temperatur von 5°C gerührt. Das Produkt wird wie üblich aufgereinigt. Es wird als klares, hellgelbes, zähflüssiges Öl erhalten.

Schritt 1.3

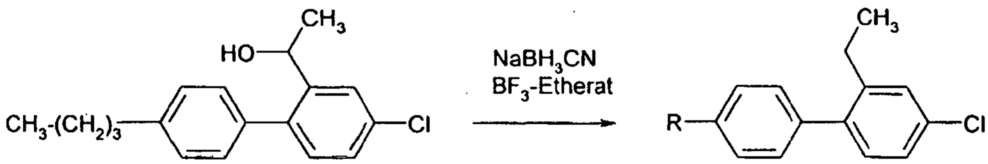


9.20 g des Produkts des letzten Schritts werden unter Stickstoffatmosphäre bei Umgebungstemperatur in 150,0 ml Tetrahydrofuran gelöst. Dann werden 49,0 ml einer Lösung von Bortrfluorid-Diehylether-Komplex (zur Synthese) tropfenweise zugefügt. Dabei erhöht sich die Temperatur des Reaktionsgemisches auf ca. 28°C. Sodann werden 17,40 g Natriumcyanoborhydrid portionsweise zugegeben. Dabei steigt die Temperatur bis auf ca. 35°C. Anschließend wird für 70 h unter Rückfluß erhitzt. Man erhält 60 g Rohprodukt als gelbes, viskoses Öl. Das Produkt wird wie üblich aufgereinigt.

Schritt 1.4

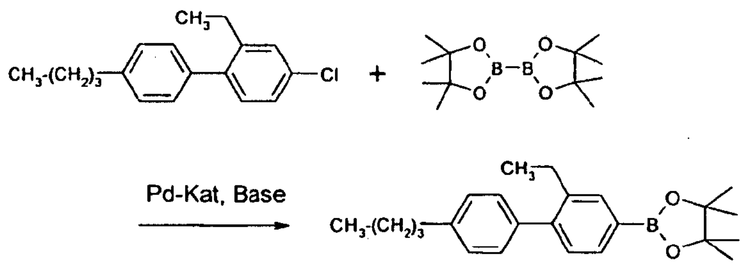


7,20 g des Produkts des letzten Schritts werden mit 13,3 g Bis-(pinacolato)-dibor, 490,0 mg Tris(dibenzylideneacetone)dipalladium(0), 510 mg 2-Dicyclohexylphosphino-2',4',6'-triisopropylbiphenyl und 7,70 g Kaliumacetat werden in 100,0 ml 1,4-Dioxan gelöst. Das Gemisch wird 16 h bei 100°C unter Rückfluß erhitzt. Das Produkt wird wie üblich aufgereinigt.

Schritt 1.5

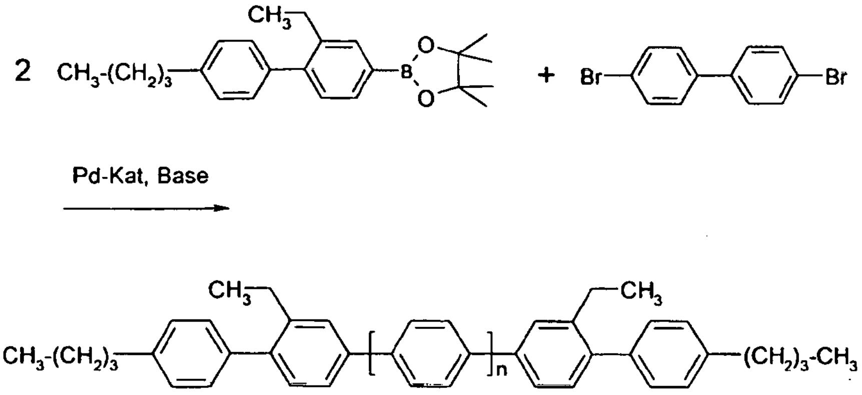


n = 2

3,00 g des Produkts des letzten Schritts werden mit 1,28 g 4,4'-Dibromphenyl zur Synthese, 300,0 mg Tris(dibenzylidene-acetone)dipalladium(0), 35,0 mg 2-Dicyclohexylphosphino-2',6'dimethoxybiphenyl und 4,80 g Kaliumphosphat monohydrat (aus Kaliumphosphat trihydrat nach 16 h bei 140°C hergestellt) in 50,0 ml 1,4-Dioxan gelöst. Das Reaktionsgemisch wird bei 100°C 16 h unter Rückfluß erhitzt. Das Rohprodukt (grünblaue Kristalle) wird isoliert. Das Produkt wird wie üblich aufgereinigt.

Das Produkt hat die Phasensequenz: K 197°C N 330,4°C I und, extrapoliert aus 5 %-iger Lösung in ZLI-4792, ein $\Delta\varepsilon$ von 2,8 und ein $\Delta n$ von 0,351.

Substanzbeispiel 2

Analog zu Substanzbeispiel 1 wird die folgende Verbindung hergestellt

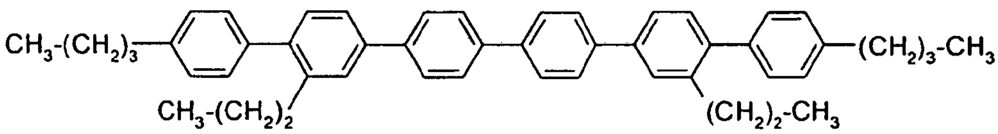

Der Einfachkeit halber wird hier nur der letzte Syntheseschritt im Detail beschrieben.

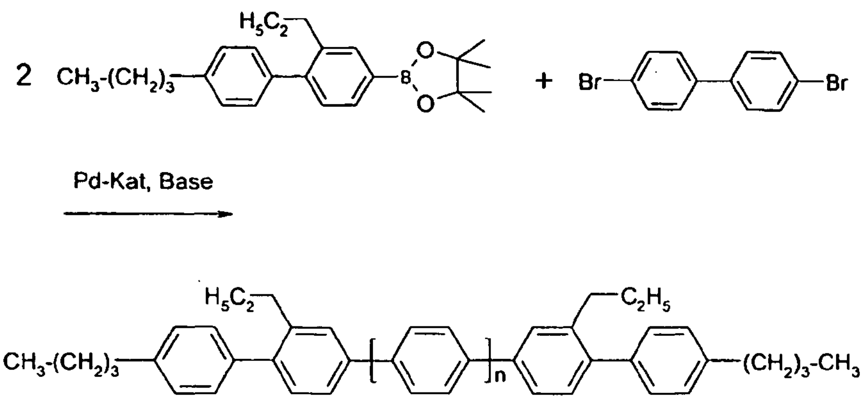


n = 2

3,80 g des Borats werden mit 1,560 g 4,4'-Dibrombiphenyl (zur Synthese), 90,0 mg Tris(dibenzylidene-acetone)dipalladium(0), 170 mg 2-Dicyclohexylphosphino-2',6'dimethoxybiphenyl und 5,80 g Kaliumphosphat monohydrat

(aus Kaliumphosphat trihydrat nach 16 h bei 140°C hergestellt)) in 50,0 ml 1,4-Dioxan gelöst. Das Reaktionsgemisch wird bei 100°C 16 h unter Rückfluß erhitzt. Das Rohprodukt wird isoliert. Das Produkt wird wie üblich aufgereinigt. Es werden hell beige Kristalle erhalten.

Das Produkt hat die Phasensequenz: K 174°C N 252,7°C I und, extrapoliert aus 5 %-iger Lösung in ZLI-4792, ein $\Delta\varepsilon$ von 1,6 und ein $\Delta n$ von 0,319.

Substanzbeispiel 3

Herstellung der Verbindung

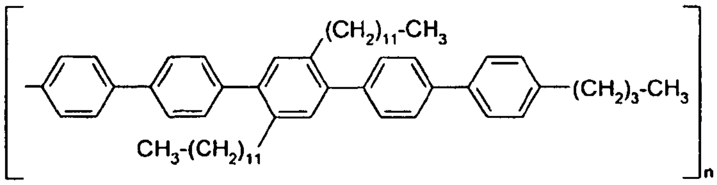

n = 2

Schritt 3.1

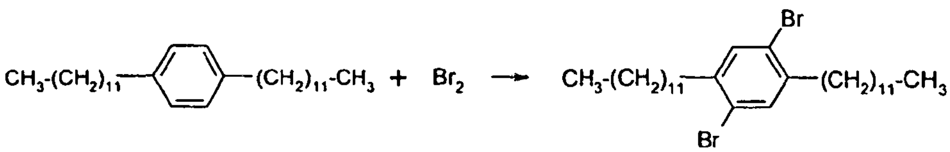

33,50 g 1,4-Di-n-dodecylbenzol, wird in 100 ml Dichlormethan gelöst. 200 mg Iod (doppelt sublimiert) werden als Katalysator zugfügt. Anschließend werden 8,5 ml Brom (reinst) zügig tropfenweise zugegeben. Das braune Reaktionsgemisch wird unter Ausschluß von Licht für 16 h bei Umgebungstemperatur gerührt. Dann werden noch einmal 100 ml Dichlormethan zugesetzt und es wird für weitere 50 h gerührt.
Das Rohprodukt (hellgelbe Kristalle) wird isoliert und wird wie üblich aufgereinigt. Es werden weiße Kristalle erhalten.

Schritt 3.2

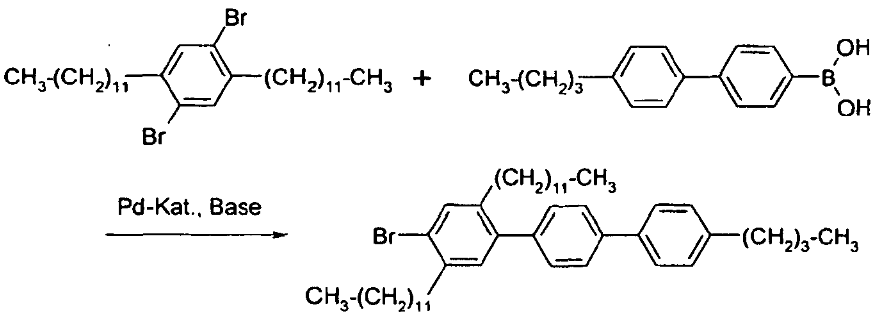


20,0 g des Produkts des letzten Schritts, 100,0 ml Toluol (reinst), 12,0 g wasserfreies Natriumcarbonat (reinst) und 50,0 ml vollentsalztem (VE-) Wasser werden vorgelegt und Unter kräftigem Rühren auf eine Temperatur im Bereich von ca. 75°C bis 80°C erwärmt. Anschließend werden 0,40 g Tetrakis(Triphenylphosphin) Pd(0) zugegeben und eine Lösung aus 9,40 g der Boronsäure in 50,0 ml Ethanol (absolut reinst) tropfenweise zugegeben. Die alkoholische Lösung der Boronsäure wird zweckmäßigerweise vorher leicht erwärmt um die Boronsäure vollständig zu lösen. Das Reaktionsgemisch wird 16h unter Rückfluß erhitzt.

Das Rohprodukt (ein braunes Öl) wird isoliert und wird wie üblich aufgereinigt. Es wird ein klares Öl erhalten.

Schritt 3.3

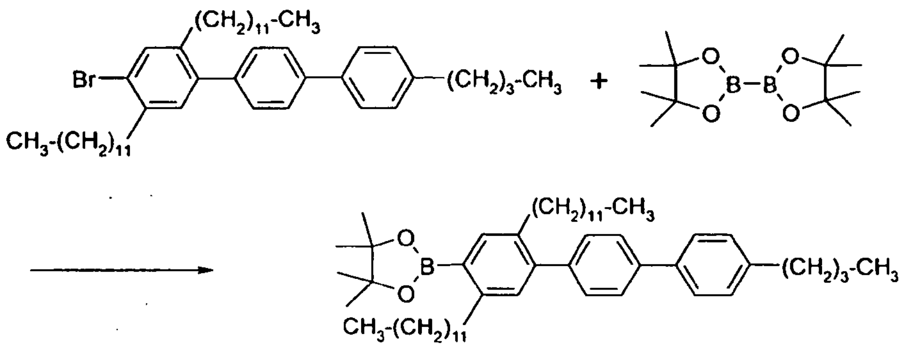

Pd-Kat., Base

13,60 g des Produkts des letzten Schritts werden in 100,0 ml 1,4-Dioxan (reinst) gelöst. Anschließend werden 0,40 g $PdCl_2$-dppf, (Bis-diphenylphosphino-Ferrocen-Palladiumdichlorid) 5,50 g Bis(pinacolato)diboron und 5,40 g Kaliumacetat (reinst) zugegeben und das Reaktionsgemisch für 4 h unter Rückfluß erhitzt. Das Rohprodukt wird isoliert (schwarzes Öl) und wird wie üblich aufgereinigt. Das Produkt wird als Öl erhalten.

Schritt 3.4

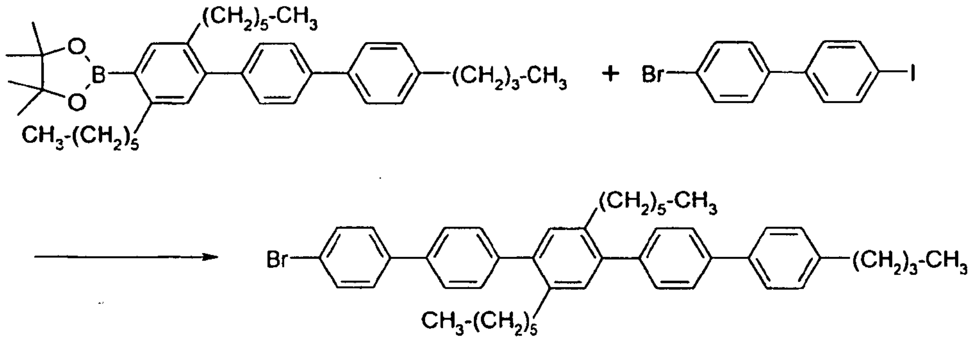

3,00 g 4-Brom-4'-iodbiphenyl werden in 80,0 ml Toluol (reinst) gelöst. Dann werden der Reihe nach 3,00 g wasserfreies Natriumcarbonat (reinst), 50,0 ml vollentsalztem (VE-) Wasser, 6,20 g des Produkts des letzten Schritts zugegeben. Das Rohprodukt (gelbbraune Kristalle) wird isoliert und wird wie üblich aufgereinigt. Es werden gelb-braune Kristalle erhalten.

Schritt 3.5

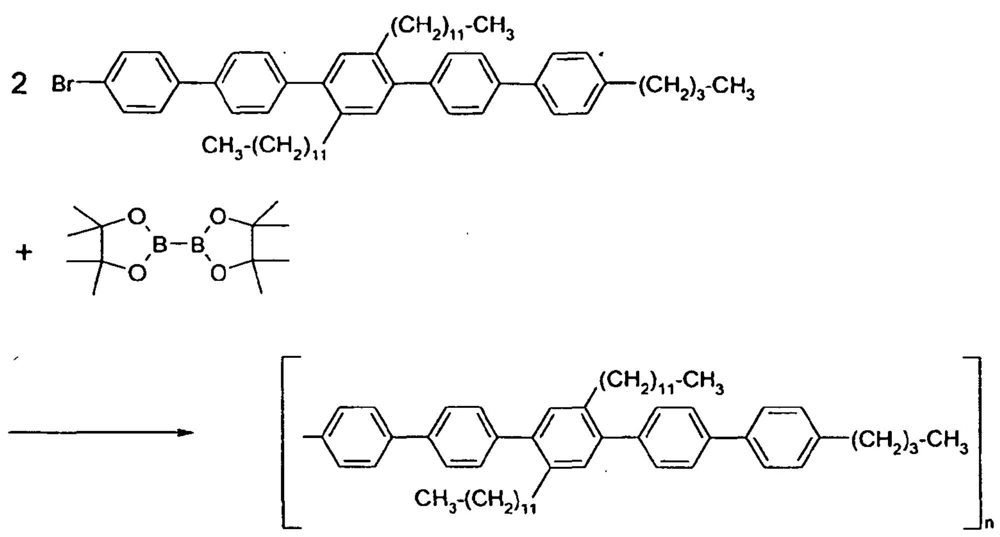

n = 2

4,70 g des Produkts des letzten Schritts und 0,680 g Bis-(pincalto)-diboron werden in 25,0 ml 1,4-Dioxan (reinst) unter leichtem Erwärmen gelöst. Dann werden zunächst 200,0 mg $PdCl_2(PCy_3)_2$ (Bis-Tricyclohexylphosphino-Palladuimdichlorid) und anschließend 3,50 g Cäsiumfluorid und ein Tropfen Wasser zugegeben. Das Reaktionsgemisch wird unter Stickstoffatmosphäre 16 h bei einer Temperatur von 100°C gerührt. Das Rohprodukt (gelbe Kristalle) wird isoliert. Das Produkt wird wie üblich aufgereinigt. Es werden gelbe Kristalle erhalten.

Das Produkt hat die Phasensequenz: K 103°C N 199,9°C I und, extrapoliert aus 5 %-iger Lösung in ZLI-4792, ein $\Delta\varepsilon$ von 0,3 und ein $\Delta n$ von 0,255.

Substanzbeispiel 4

Analog zu Substanzbeispiel 3 wird die folgende Verbindung hergestellt

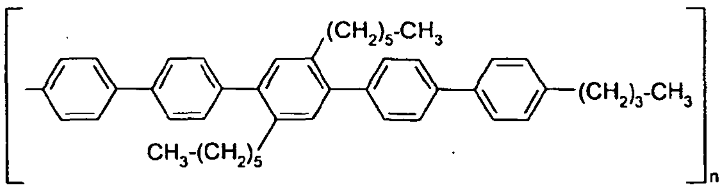

n = 2

Der Einfachkeit halber wird hier nur der letzte Syntheseschritt im Detail beschrieben.

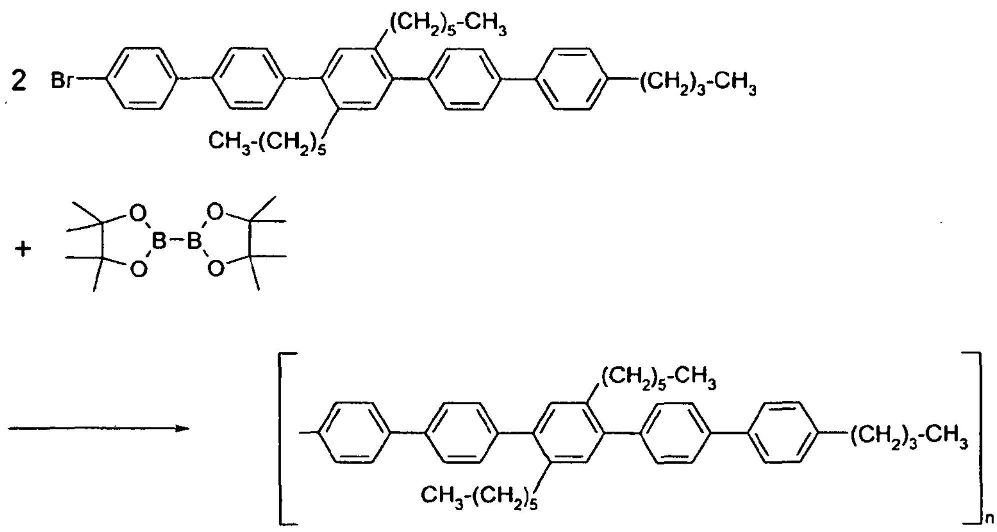

n = 2

1,70 g des Produkts des letzten Schritts und 0,280 g Bis-(pincalto)-diboron werden in 10,0 ml 1,4-Dioxan unter leichtem Erwärmen gelöst. Dann werden zunächst 80,0 mg $PdCl_2(PCy_3)_2$ (Bis-Tricyclohexylphosphino-Palladuimdichlorid) und anschließend 1,40 g Cäsiumfluorid zugegeben. Das Reaktionsgemisch wird unter Stickstoffatmosphäre 16 h unter Rückfluß erhitzt. Das Produkt wird wie üblich aufgereinigt. Es werden gelbe Kristalle erhalten.

Das Produkt hat die Phasensequenz: K 154°C N 283,9°C I und, extrapoliert aus 5 %-iger Lösung in ZLI-4792, ein $\Delta\varepsilon$ von 3,0 und ein $\Delta n$ von 0,308.

Substanzbeispiel 5

Analog zu Substanzbeispiel 3 wird die folgende Verbindung hergestellt

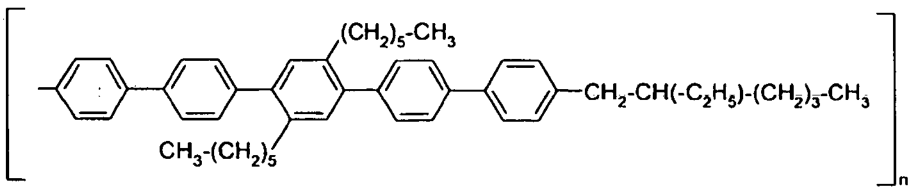

n = 2

Der Einfachkeit halber wird hier nur der letzte Syntheseschritt im Detail beschrieben.

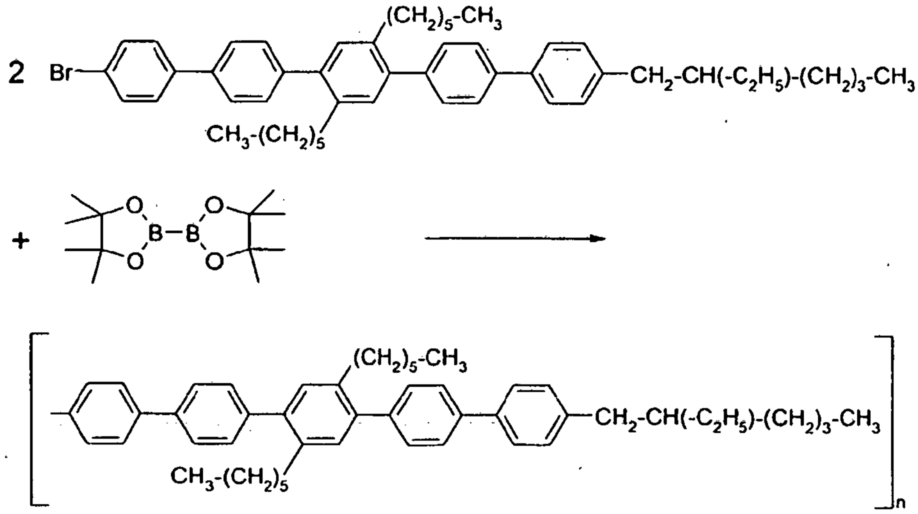

n = 2

5,0 g des Produkts des letzten Schritts und 0,863 g Bis-(pincalto)-diboron werden in 35,0 ml 1,4-Dioxan unter leichtem Erwärmen gelöst. Dann werden zunächst 250 mg $PdCl_2(PCy_3)_2$ (Bis-Tricyclohexylphosphino-Palladuimdichlorid) und anschließend 1,40 g Cäsiumfluorid und 1 Tropfen Wasser zugegeben. Das Reaktionsgemisch wird unter Stickstoffatmosphäre 16 h bei einer Temperatur von 100°C gerührt. Das Rohprodukt (eine amorphe, gelbliche/gelblich-orange Masse) wird isoliert. Das Produkt wird wie üblich aufgereinigt. Es werden gräuliche Kristalle erhalten.

Das Produkt hat die Phasensequenz: K 147°C N 238,4°C I und, extrapoliert aus 10 %-iger Lösung in ZLI-4792, ein $\Delta\varepsilon$ von 0,4 und ein $\Delta n$ von 0,267.

<u>Anwendungsbeispiele</u>

<u>Vergleichsbeispiel 1</u>

Es wird die bekannte flüssigkristalline Verbindung 4'-Pentyl-4-cyanobiphenyl (auch 5CB oder K15 genannt, Merck KGaA, Darmstadt, Deutschland) bezüglich ihrer physikalischen Eigenschaften, insbesondere im $\mu$-Wellenbereich, bei 20°C untersucht.

<u>Tabelle 1: Eigenschaften der Verbindung K15 bei 30 GHz</u>

| T/°C | $\varepsilon_{r,\parallel}$ | $\varepsilon_{r,\perp}$ | $\tau$ | tan $\delta_{\varepsilon,r,\parallel}$ | tan $\delta_{\varepsilon,r,\perp}$ | $\eta$ |
|---|---|---|---|---|---|---|
| 20 | 2,87 | 2,55 | 0,110 | 0,0114 | 0,026 | 4,3 |

Diese Verbindung hat eine Phasenfolge von: K 23°C N 35,1 °C und bei einer Temperatur von 26°C ein $\Delta\varepsilon$ von 11,0 und bei einer Temperatur von 29°C ein $\Delta\varepsilon$ von 9,9, sowie extrapoliert aus 10 %-iger Lösung in ZLI-4792, ein $\Delta\varepsilon$ von 20,1 und ein $\Delta n$ von 0,212.

Die Verbindung hat eine seht geringe Materialgüte und eignet sich nicht besonders gut für Anwendungen im Mikrowellenbereich, da sie einen sehr schmalen Phasenbereich und ein eher niedriges $\eta$ hat.

<u>Vergleichsbeispiel 2</u>

Es wird eine flüssigkristalline Substanz mit der Kurzbezeichnung PTP(2)TP-6-3 nach Hsu, C. S. Shyu, K. F., Chuang, Y. Y. and Wu, S.-T., Liq. Cryst., 27 (2), (2000), p. 283-287 hergestellt und bezüglich ihrer physikalischen Eigenschaften, insbesondere im $\mu$-Wellenbereich, untersucht. Die Verbindung hat eine nematische Phase und einen Klärpunkt von 108°C.

<u>Tabelle 2: Eigenschaften der Verbindung PTP(2)TP-6-3 bei 30 GHz</u>

| T/°C | $\varepsilon_{r,\parallel}$ | $\varepsilon_{r,\perp}$ | $\tau$ | tan $\delta_{\varepsilon,r,\parallel}$ | tan $\delta_{\varepsilon,r,\perp}$ | $\eta$ |
|---|---|---|---|---|---|---|
| 20 | 3,17 | 2,38 | 0,249 | 0,0018 | 0,0063 | 40 |

(fortgesetzt)

| T/°C | $\varepsilon_{r,\parallel}$ | $\varepsilon_{r,\perp}$ | $\tau$ | tan $\delta_{\varepsilon,r,\parallel}$ | tan $\delta_{\varepsilon,r,\perp}$ | $\eta$ |
|---|---|---|---|---|---|---|
| 22 | 3,22 | 2,44 | 0,242 | 0,0018 | 0,0064 | 38 |

Die Verbindung hat eine Phasenfolge von: $T_g$ -54°C N 119,2°C und, extrapoliert aus 10 %-iger Lösung in ZLI-4792, ein $\Delta\varepsilon$ von 1,8 und ein $\Delta n$ von 0,393.

Die Verbindung eignet sich für Anwendungen im Mikrowellenbereich, insbesondere für Phasenschieber allerdings hat sie ein geringes $\Delta\varepsilon$.

Tabelle 3: Vergleich der Eigenschaften der verschiedenen Beispiele bei 30 GHz bei 20°C

| Beispiel | Flüssiqkrst. | $\Delta\varepsilon$ | $\varepsilon_{r,\parallel}$ | $\varepsilon_{r,\perp}$ | $\tau$ | tan $\delta_{\varepsilon\ r,Max.}$ | $\eta$ |
|---|---|---|---|---|---|---|---|
| Vergl. 1 | K15 | 20 | 2,87 | 2,55 | 0,110 | 0,026 | 4,3 |
| Vergl. 2 | P2-6-3* | 0,4 | 3,17 | 2,38 | 0,249 | 0,0063 | 40 |
| Vergl. 2 | P2-6-3* | 0,4 | 3,22§ | 2,44§ | 0,242§ | 0,0064§ | 38§ |
| 1 | M-1 | 0,8 | 3,15 | 2,38 | 0,244 | 0,0057 | 45 |
| 2 | M-2 | n.z.b. | 3,18 | 2,40 | 0,245 | 0,0055 | 46 |
| 3 | M-3 | 0,8 | 3,14 | 2,37 | 0,245 | 0,0054 | 45 |
| 4 | M-4 | 2,4 | 3,18 | 2,39 | 0,248 | 0,0074 | 34 |
| 5 | M-5 | 0,9 | 3,13§ | 2,40§ | 0,233§ | 0,0052§ | 45§ |
| 6 | M-6 | 3,0 | 3,23 | 2,42 | 0,250 | 0,0085 | 30 |
| Bemerkungen: *): P2-6-3: PTP(2)TP-6-3,<br>§): bei T = 22°C und<br>n.z.b.: noch zu bestimmen. | | | | | | | |

Beispiel 1

Es wird eine Flüssigkristallmischung M-1 mit der Zusammensetzung und den Eigenschaften wie in der folgenden Tabelle angegeben hergestellt und bezüglich ihrer physikalischen Eigenschaften, insbesondere im $\mu$-Wellenbereich, untersucht.

| Zusammensetzung | | | Physikalische Eigenschaften | |
|---|---|---|---|---|
| Verbindung | | | T(N,I) | = 118,5 °C |
| Nr. | Abkürzung | | $n_e$ (20°C, 589,3 nm) | = n.z.b. |
| 1 | PTP(2)TP-6-3 | 95,0 | $n_o$ (20°C, 589,3 nm) | = n.z.b. |
| 2 | 6*P-1 | 5,0 | $\varepsilon_{\parallel}$ (20°C, 1 kHz) | = 3,4 |
| Σ | | 100,0 | $\Delta\varepsilon$ (20°C, 1 kHz) | = +0,8 |
| | | | $k_1$ (20°C) | = 11,7 pN |
| | | | $K_3$ (20°C) | = 52,2 pN |
| Bemerkungen: n.z.b.: noch zu bestimmen und | | | | |

Tabelle 4: Eigenschaften der Mischung M-1 bei 30 GHz

| T/°C | $\varepsilon_{r,\parallel}$ | $\varepsilon_{r,\perp}$ | $\tau$ | tan $\delta_{\varepsilon,r,\parallel}$ | tan $\delta_{\varepsilon,r,\perp}$ | $\eta$ |
|---|---|---|---|---|---|---|
| 20 | 3,15 | 2,38 | 0,244 | 0,0013 | 0,0057 | 45 |

Diese Mischung eignet sich sehr gut für Anwendungen im Mikrowellenbereich, insbesondere für Phasenschieber.

Beispiel 2

Es wird eine Flüssigkristallmischung M-2 mit der Zusammensetzung und den Eigenschaften wie in der folgenden Tabelle angegeben hergestellt und bezüglich ihrer physikalischen Eigenschaften, insbesondere im $\mu$-Wellenbereich, untersucht.

| Zusammensetzung | | | Physikalische Eigenschaften | |
|---|---|---|---|---|
| Verbindung | | | T(N,I) | = 115,5 °C |
| Nr. | Abkürzung | | $n_e$ (20°C, 589,3 nm) | = n.z.b. |
| 1 | PTP(2)TP-6-3 | 93,0 | $n_o$ (20°C, 589,3 nm) | = n.z.b. |
| 2 | 10*P-1 | 7,0 | $\varepsilon_{\|}$ (20°C, 1 kHz) | = n.z.b. |
| $\Sigma$ | | 100,0 | $\Delta\varepsilon$ (20°C, 1 kHz) | = n.z.b. |
| Bemerkung: n.z.b.: noch zu bestimmen. | | | | |

Tabelle 5: Eigenschaften der Mischung M-2 bei 30 GHz

| T/°C | $\varepsilon_{r,\|}$ | $\varepsilon_{r,\perp}$ | $\tau$ | tan $\delta_{\varepsilon,r,\|}$ | tan $\delta_{\varepsilon,r,\perp}$ | $\eta$ |
|---|---|---|---|---|---|---|
| 20 | 3,18 | 2,40 | 0,245 | 0,0015 | 0,0055 | 46 |

Diese Mischung eignet sich sehr gut für Anwendungen im Mikrowellenbereich, insbesondere für Phasenschieber.

Beispiel 3

Es wird eine Flüssigkristallmischung M-3 mit der Zusammensetzung und den Eigenschaften wie in der folgenden Tabelle angegeben hergestellt und bezüglich ihrer physikalischen Eigenschaften, insbesondere im $\mu$-Wellenbereich, untersucht.

| Zusammensetzung | | | Physikalische Eigenschaften | |
|---|---|---|---|---|
| Verbindung | | | T(N,I) | = 116,5 °C |
| Nr. | Abkürzung | | $n_e$ (20°C, 589,3 nm) | = n.z.b. |
| 1 | PTP(2)TP-6-3 | 90,0 | $n_o$ (20°C, 589,3 nm) | = n.z.b. |
| 2 | 10*P-3 | 10,0 | $\varepsilon_{\|}$ (20°C, 1 kHz) | = 3,4 |
| $\Sigma$ | | 100,0 | $\Delta\varepsilon$ (20°C, 1 kHz) | = +0,8 |
| | | | $k_1$ (20°C) | = 12,47 pN |
| | | | $K_3$ (20°C) | = 51,2 pN |
| Bemerkung: n.z.b.: noch zu bestimmen. | | | | |

Tabelle 6: Eigenschaften der Mischung M-3 bei 30 GHz

| T/°C | $\varepsilon_{r,\|}$ | $\varepsilon_{r,\perp}$ | $\tau$ | tan $\delta_{\varepsilon,r,\|}$ | tan $\delta_{\varepsilon,r,\perp}$ | $\eta$ |
|---|---|---|---|---|---|---|
| 20 | 3,14 | 2,37 | 0,245 | 0,0015 | 0,0054 | 45 |

Diese Mischung eignet sich sehr gut für Anwendungen im Mikrowellenbereich, insbesondere für Phasenschieber.

Beispiel 4

Es wird eine Flüssigkristallmischung M-4 mit der Zusammensetzung und den Eigenschaften wie in der folgenden Tabelle angegeben hergestellt und bezüglich ihrer physikalischen Eigenschaften, insbesondere im $\mu$-Wellenbereich, untersucht.

| Zusammensetzung | | | Physikalische Eigenschaften | |
|---|---|---|---|---|
| Verbindung | | | T(N,I) | = 123,5 °C |
| Nr. | Abkürzung | | $n_e$ (20°C, 589,3 nm) | = n.z.b. |
| 1 | PTP(2)TP-3-1 | 10,0 | $n_o$ (20°C, 589,3 nm) | = n.z.b. |
| 2 | PTP(2)TP-6-3 | 63,0 | $\varepsilon_\parallel$ (20°C, 1 kHz) | = 5,2 |
| 3 | PTP(2)TP-3-O5 | 10,0 | $\Delta\varepsilon$ (20°C, 1 kHz) | = 2,4 |
| 4 | PGUQU-3-F | 5,0 | $k_1$ (20°C) | = n.z.b. pN |
| 5 | $PU[QGU]_2$-5-F | 5,0 | $K_3$ (20°C) | = n.z.b. pN |
| 6 | 10*P-3 | 7,0 | | |
| Σ | | 100,0 | | |
| Bemerkungen: n.z.b.: noch zu bestimmen,<br>$PU[QGU]_2$-5-F: PUQGUQGU-5-F,<br>Phasensequenz: K 86°C N 236,4°C I. | | | | |

Tabelle 7: Eigenschaften der Mischung M-4 bei 30 GHz

| T/°C | $\varepsilon_{r,\parallel}$ | $\varepsilon_{r,\perp}$ | $\tau$ | $\tan \delta_{\varepsilon,r,\parallel}$ | $\tan \delta_{\varepsilon,r,\perp}$ | $\eta$ |
|---|---|---|---|---|---|---|
| 20 | 3,18 | 2,39 | 0,248 | 0,0021 | 0,0074 | 34 |

Diese Mischung eignet sich sehr gut für Anwendungen im Mikrowellenbereich, insbesondere für Phasenschieber. Sie hat insbesondere ein relativ hohes $\Delta\varepsilon$.

Beispiel 5

Es wird eine Flüssigkristallmischung M-5 mit der Zusammensetzung und den Eigenschaften wie in der folgenden Tabelle angegeben hergestellt und bezüglich ihrer physikalischen Eigenschaften, insbesondere im $\mu$-Wellenbereich, untersucht.

| Zusammensetzung | | | Physikalische Eigenschaften | |
|---|---|---|---|---|
| Verbindung | | | T(N,I) | = 119,0 °C |
| Nr. | Abkürzung | | $n_e$ (20°C, 589,3 nm) | = n.z.b. |
| 1 | PTP(2)TP-6-3 | 70,0 | $n_o$ (20°C, 589,3 nm) | = n.z.b. |
| 2 | PTP(c3)TP-4-4 | 10,0 | $\varepsilon_\parallel$ (20°C, 1 kHz) | = 3,6 |
| 3 | PT(1,4N)BP-3-4 | 10,0 | $\Delta\varepsilon$ (20°C, 1 kHz) | = +0,9 |
| 4 | 10*P-3 | 10,0 | $k_1$ (20°C) | = 10,9 pN |
| Σ | | 100,0 | $K_3$ (20°C) | = 46,4 pN |
| Bemerkungen: n.z.b.: noch zu bestimmen. | | | | |

Tabelle 8: Eigenschaften der Mischung M-5 bei 30 GHz

| T/°C | $\varepsilon_{r,\parallel}$ | $\varepsilon_{r,\perp}$ | $\tau$ | $\tan \delta_{\varepsilon,r,\parallel}$ | $\tan \delta_{\varepsilon,r,\perp}$ | $\eta$ |
|---|---|---|---|---|---|---|
| 22 | 3,13 | 2,40 | 0,233 | 0,0015 | 0,0052 | 45 |

Diese Mischung eignet sich sehr gut für Anwendungen im Mikrowellenbereich, insbesondere für Phasenschieber.

Beispiel 6

Es wird eine Flüssigkristallmischung M-6 mit der Zusammensetzung und den Eigenschaften wie in der folgenden Tabelle angegeben hergestellt.

| Zusammensetzung | | | Physikalische Eigenschaften | |
|---|---|---|---|---|
| Verbindung | | | T(N,I) | = 129,5 °C |
| Nr. | Abkürzung | | $n_e$ (20°C, 589,3 nm) | = n.z.b. |
| 1 | PTP(2)TP-3-1 | 15,0 | $n_o$ (20°C, 589,3 nm) | = n.z.b. |
| 2 | PTP(2)TP-3-3 | 15,0 | $\varepsilon_{\|}$ (20°C, 1 kHz) | = 6,5 |
| 3 | PTP(2)TP-3-O5 | 15,0 | $\Delta\varepsilon$ (20°C, 1 kHz) | = 3,0 |
| 4 | PTP(2)TP-6-3 | 42,0 | $k_1$ (20°C) | = n.z.b. |
| 5 | D(fN)UQU-3-F | 8,0 | $K_3$ (20°C) | = n.z.b. |
| 6 | 10*P-3 | 5,0 | | |
| Σ | | 100,0 | | |
| Bemerkungen: n.z.b.: noch zu bestimmen und D(fN)UQU-3-F, Phasensequenz: K 80°C N 120°C I. | | | | |

Tabelle 9: Eigenschaften der Mischung M-6 bei 30 GHz

| T/°C | $\varepsilon_{r,\|}$ | $\varepsilon_{r,\perp}$ | $\tau$ | tan $\delta_{\varepsilon,r,\|}$ | tan $\delta_{\varepsilon,r,\perp}$ | $\eta$ |
|---|---|---|---|---|---|---|
| 20 | 3,23 | 2,42 | 0,250 | 0,0023 | 0,0085 | 30 |

Diese Mischung eignet sich sehr gut für Anwendungen im Mikrowellenbereich, insbesondere für Phasenschieber. Sie hat insbesondere ein relativ hohes $\Delta\varepsilon$.

## Patentansprüche

1. Bauteil für die Hochfrequenztechnik, bzw. für den Mikrowellenbereich und Millimeterwellenbereich des elektromagnetischen Spektrums, **dadurch gekennzeichnet, dass** es ein Flüssigkristallmedium enthält, das seinerseits eine *Komponente A* enthält, die ihrerseits aus einer oder mehreren Verbindungen der Formel I

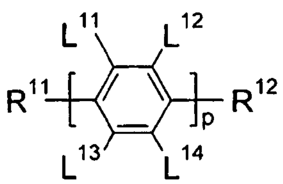

I

worin

$R^{11}$ und $R^{12}$ unabhängig voneinander Halogen, unfluoriertes Alkyl oder fluoriertes Alkyl oder unfluoriertes Alkoxy oder fluoriertes Alkoxy jeweils mit 1 bis 15 C-Atomen oder unfluoriertes Alkenyl oder fluoriertes Alkenyl, unfluoriertes Alkenyloxy oder unfluoriertes Alkoxyalkyl oder fluoriertes Alkoxyalkyl jeweils mit 2 bis 15 C-Atomen, worin unabhängig voneinander auch eine oder mehrere "-$CH_2$-"-Gruppen durch Cycloalkyl mit 3 bis 6 C-Atomen, bevorzugt mit 4 oder 6 C-Atomen, ersetzt sein können, und alternativ auch einer von $R^{11}$ und $R^{12}$ oder $R^{11}$ und $R^{12}$ beide H,

$L^{11}$ bis $L^{14}$ bei jedem Erscheinen, jeweils unabhängig voneinander H, Alkyl mit 1 bis 15 C-Atomen, F oder Cl und

p eine ganze Zahl im Bereich von 6 bis 15

bedeuten, besteht.

2. Bauteil nach Anspruch 1, **dadurch gekennzeichnet, dass** es ein Flüssigkristallmedium enthält, dass eine *Komponente B* enthält, die aus einer oder mehreren Verbindungen der Formel IV

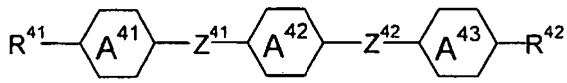

IV

worin

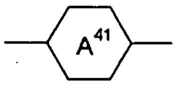 und 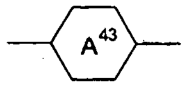

beide

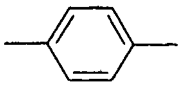

und

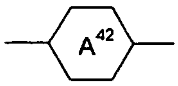 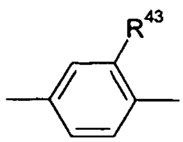

oder
einer oder mehrere von

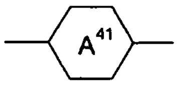 bis 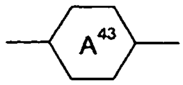

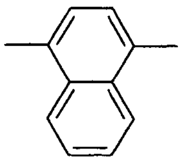

und die anderen

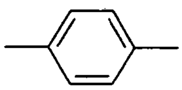,

$R^{41}$ bis $R^{43}$ unabhängig voneinander eine der in Anspruch 1 für $R^{11}$ gegeben Bedeutungen haben, und

$Z^{41}$ und $Z^{42}$ unabhängig voneinander -C≡C-, -CF=CF-, -CF=CH-,- CH=CF oder -CH=CH-,

bedeuten, besteht.

**3.** Bauteil nach einem der Ansprüche 1 und 2, **dadurch gekennzeichnet, dass** das Flüssigkristallmedium neben einer Komponente A zusätzlich eine oder mehrere Komponenten ausgewählt aus der Gruppe der folgenden Komponenten, *Komponenten B* bis *E*, enthält:

- eine Komponente, *Komponente B*, die eine dielektrische Anisotropie im Bereich von mehr als -5,0 und von weniger als 10,0 aufweist und bevorzugt aus einer oder mehreren der Verbindungen der in Anspruch 2 gegebenen Formel IV besteht,
- einer stark dielektrisch positiven Komponente, *Komponente C*, die eine dielektrische Anisotropie von 10 oder mehr aufweist,
- einer stark dielektrisch negativen Komponente, *Komponente D*, die eine dielektrische Anisotropie mit einem Betrag von 5 oder mehr aufweist,
- eine Komponente, *Komponente E,* die ebenfalls eine dielektrische Anisotropie im Bereich von mehr als -5,0 und von weniger als 10,0 aufweist und aus Verbindungen mit bis zu fünf fünf-, sechs- oder siebengliedrigen Ringen besteht.

**4.** Bauteil nach Anspruch 3, **dadurch gekennzeichnet, dass** das Flüssigkristallmedium eine *Komponente* C enthält.

**5.** Bauteil nach einem der Ansprüche 3 und 4, **dadurch gekennzeichnet, dass** das Flüssigkristallmedium eine *Komponente D* enthält.

**6.** Bauteil nach einem der Ansprüche 3 und 4, **dadurch gekennzeichnet, dass** das Flüssigkristallmedium eine *Komponente E* enthält.

**7.** Flüssigkristallmedium, **dadurch gekennzeichnet, dass** es

• eine *Komponente A*, die aus einer oder mehreren Verbindungen der in Anspruch 1 angegebenen Formel I besteht, und
• zusätzlich eine oder mehrere Komponenten ausgewählt aus der Gruppe der folgenden Komponenten, *Komponenten B* bis *E*:

  - eine Komponente, *Komponente B*, die eine dielektrische Anisotropie im Bereich von mehr als -5,0 und von weniger als 10,0 aufweist und bevorzugt aus Verbindungen der in Anspruch 2 gegebenen Formel IV besteht,
  - eine stark dielektrisch positive Komponente, *Komponente C*, die eine dielektrische Anisotropie von 10 oder mehr aufweist,
  - eine stark dielektrisch negative Komponente, *Komponente D,* die eine dielektrische Anisotropie mit einem Betrag von 5 oder mehr aufweist,
  - eine Komponente, *Komponente E,* die ebenfalls eine dielektrische Anisotropie im Bereich von mehr als -5,0 und von weniger als 10,0 aufweist und aus Verbindungen mit bis zu fünf fünf-, sechs- oder siebengliedrigen Ringen besteht,

enthält.

**8.** Flüssigkristallmedium nach Anspruch 7, **dadurch gekennzeichnet, dass** das Flüssigkristallmedium eine *Komponente B* enthält.

**9.** Flüssigkristallmedium nach einem der Ansprüche 7 und 8, **dadurch gekennzeichnet, dass** das Flüssigkristallmedium eine *Komponente C* enthält.

**10.** Flüssigkristallmedium nach einem der Ansprüche 7 und 8, **dadurch gekennzeichnet, dass** das Flüssigkristallmedium eine *Komponente D* enthält.

**11.** Verwendung eines Flüssigkristallmediums nach einem oder mehreren der Ansprüche 7 bis 10 in einem Bauteil für die Hochfrequenztechnik.

**12.** Verfahren zur Herstellung eines Flüssigkristallmediums, **dadurch gekennzeichnet, dass** eine oder mehrere Verbindungen der Formel I, wie in Anspruch 1 angegeben, mit einer oder mehreren weiteren Verbindungen und/oder mit einem oder mehreren Additiven gemischt werden.

**13.** Verfahren zur Herstellung eines Bauteils nach einem oder mehreren der Ansprüche 1 bis 6, **dadurch gekennzeichnet, dass** ein Flüssigkristallmedium nach einem oder mehreren der Ansprüche 7 bis 10 verwendet wird.

**14.** Phasenschieber, **dadurch gekennzeichnet**, das er ein oder mehrere Bauteile nach einem oder mehreren der Ansprüche 1 bis 6 enthält.

**15.** Mikrowellenantennenarray, **dadurch gekennzeichnet**, das es ein oder mehrere Bauteile nach einem oder mehreren der Ansprüche 1 bis 6 enthält.

**16.** Verfahren zum Abstimmen eines Mikrowellenantennenarrays,
**dadurch gekennzeichnet, dass** ein oder mehrere Bauteile nach einem oder mehreren der Ansprüche 1 bis 6 elektrisch angesteuert werden bzw. elektrisch angesteuert werden können.

**17.** Verbindung der Formel I

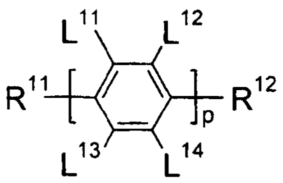

I

worin

$R^{11}$ und $R^{12}$ unabhängig voneinander Halogen, unfluoriertes Alkyl oder fluoriertes Alkyl oder unfluoriertes Alkoxy oder fluoriertes Alkoxy jeweils mit 1 bis 15 C-Atomen oder unfluoriertes Alkenyl oder fluoriertes Alkenyl, unfluoriertes Alkenyloxy oder unfluoriertes Alkoxyalkyl oder fluoriertes Alkoxyalkyl jeweils mit 2 bis 15 C-Atomen, worin unabhängig voneinander auch eine oder mehrere "-$CH_2$-"-Gruppen durch Cycloalkyl mit 3 bis 6 C-Atomen, bevorzugt mit 4 oder 6 C-Atomen, ersetzt sein können,
$L^{11}$ bis $L^{14}$ bei jedem Erscheinen, jeweils unabhängig voneinander H, Alkyl mit 1 bis 15 C-Atomen, F oder Cl und
p eine ganze Zahl im Bereich von 6 bis 15

bedeuten,
wobei mindestens zwei der vorhandenen Substituenten $L^{11}$ bis $L^{14}$ eine von H verschiedene Bedeutung haben.

**18.** Verbindung der Formel I nach Anspruche 17, **dadurch gekennzeichnet, dass** mindestens zwei der vorhandenen Substituenten

$L^{11}$ bis $L^{14}$ Alkyl

bedeuten.

**19.** Verbindung der Formel I nach Anspruche 17 oder 18, **dadurch gekennzeichnet, dass**

$R^{11}$ $C_nH_{2n+1}$ oder $CH_2$=CH-$(CH_2)_Z$, und
$R^{12}$ $C_mH_{2m+1}$ oder O-$C_mH_{2m+1}$ oder $(CH_2)_Z$-CH=$CH_2$,

bedeutet, worin

n und m unabhängig voneinander eine ganze Zahl im Bereich von 1 bis 7 bedeuten, und
z 0, 1, 2, 3 oder 4 bedeutet.

**20.** Verfahren zur Herstellung einer Verbindung der Formel I nach einem oder mehreren der Ansprüche 17 bis 19,

**dadurch gekennzeichnet, dass** das Produkt oder eine oder mehrere Zwischenprodukte aus einer Arylboratverbindung mit einer Arylhalogenverbindung oder mit einer Aryltriflatverbindung durch Palladium-katalysierte Homokopplung und/oder Kreuzkopplung verknüpft wird.

## Claims

**1.** Device for high-frequency technology or for the microwave region and millimetre wave region of the electromagnetic spectrum, **characterised in that** it contains a liquid-crystal medium which itself comprises a *component A* which itself consists of one or more compounds of the formula I

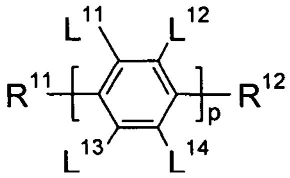

I

in which

$R^{11}$ and $R^{12}$, independently of one another, denote halogen, unfluorinated alkyl or fluorinated alkyl or unfluorinated alkoxy or fluorinated alkoxy, each having 1 to 15 C atoms, or unfluorinated alkenyl or fluorinated alkenyl, unfluorinated alkenyloxy or unfluorinated alkoxyalkyl or fluorinated alkoxyalkyl, each having 2 to 15 C atoms, in which, independently of one another, one or more "-$CH_2$-" groups may, in addition, be replaced by cycloalkyl having 3 to 6 C atoms, preferably having 4 or 6 C atoms, and alternatively, in addition, one of $R^{11}$ and $R^{12}$ or both $R^{11}$ and $R^{12}$ denote H,
$L^{11}$ to $L^{14}$ on each appearance, in each case independently of one another, denote H, alkyl having 1 to 15 C atoms, F or Cl, and
p denotes an integer in the range from 6 to 15.

**2.** Device according to Claim 1, **characterised in that** it contains a liquid-crystal medium which comprises a *component B* which consists of one or more compounds of the formula IV

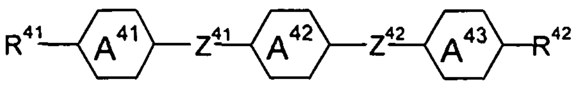

IV

in which

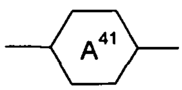

and

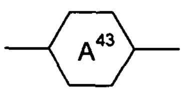

both denote

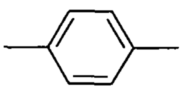

and

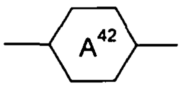

denotes 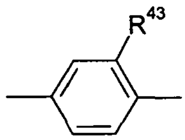

or

one or more of

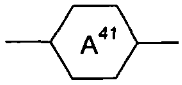 to 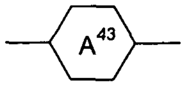

denote(s)

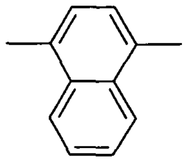

and the others denote

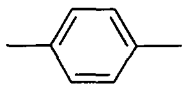 ,

$R^{41}$ to $R^{43}$, independently of one another, have one of the meanings given for $R^{11}$ in Claim 1, and
$Z^{41}$ and $Z^{42}$, independently of one another, denote -C≡C-, -CF=CF-, -CF=CH-, -CH=CF- or -CH=CH-.

**3.** Device according to one of Claims 1 and 2, **characterised in that** the liquid-crystal medium, besides a component A, additionally comprises one or more components selected from the group of the following components, *components B* to *E*:

- a component, *component B,* which has a dielectric anisotropy in the range from more than -5.0 to less than 10.0 and preferably consists of one or more of the compounds of the formula IV given in Claim 2,
- a strongly dielectrically positive component, *component C*, which has a dielectric anisotropy of 10 or more,
- a strongly dielectrically negative component, *component D,* which has a dielectric anisotropy having a value of 5 or more,
- a component, *component E,* which likewise has a dielectric anisotropy in the range from more than -5.0 to less than 10.0 and consists of compounds having up to five five-, six- or seven-membered rings.

**4.** Device according to Claim 3, **characterised in that** the liquid-crystal medium comprises a *component C.*

**5.** Device according to one of Claims 3 and 4, **characterised in that** the liquid-crystal medium comprises a *component D.*

**6.** Device according to one of Claims 3 and 4, **characterised in that** the liquid-crystal medium comprises a *component E.*

**7.** Liquid-crystal medium, **characterised in that** it comprises

• a *component A* which consists of one or more compounds of the formula I indicated in Claim 1, and
• additionally one or more components selected from the group of the following components, *components B* to *E:*

- a component, *component B,* which has a dielectric anisotropy in the range from more than -5.0 to less

than 10.0 and preferably consists of compounds of the formula IV given in Claim 2,
- a strongly dielectrically positive component, *component C*, which has a dielectric anisotropy of 10 or more,
- a strongly dielectrically negative component, *component D,* which has a dielectric anisotropy having a value of 5 or more,
- a component, *component E,* which likewise has a dielectric anisotropy in the range from more than -5.0 to less than 10.0 and consists of compounds having up to five five-, six- or seven-membered rings.

**8.** Liquid-crystal medium according to Claim 7, **characterised in that** the liquid-crystal medium comprises a *component B.*

**9.** Liquid-crystal medium according to one of Claims 7 and 8, **characterised in that** the liquid-crystal medium comprises a *component C.*

**10.** Liquid-crystal medium according to one of Claims 7 and 8, **characterised in that** the liquid-crystal medium comprises a *component D.*

**11.** Use of a liquid-crystal medium according to one or more of Claims 7 to 10 in a device for high-frequency technology.

**12.** Process for the preparation of a liquid-crystal medium, **characterised in that** one or more compounds of the formula I as indicated in Claim 1 is (are) mixed with one or more further compounds and/or with one or more additives.

**13.** Process for the production of a device according to one or more of Claims 1 to 6, **characterised in that** a liquid-crystal medium according to one or more of Claims 7 to 10 is used.

**14.** Phase shifter, **characterised in that** it comprises one or more devices according to one or more of Claims 1 to 6.

**15.** Microwave antenna array, **characterised in that** it comprises one or more devices according to one or more of Claims 1 to 6.

**16.** Process for tuning a microwave antenna array, **characterised in that** one or more devices according to one or more of Claims 1 to 6 is (are) electrically addressed or can be electrically addressed.

**17.** Compound of the formula I

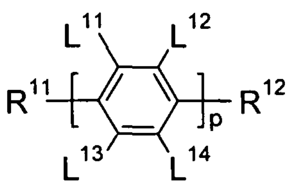

I

in which

$R^{11}$ and $R^{12}$, independently of one another, denote halogen, unfluorinated alkyl or fluorinated alkyl or unfluorinated alkoxy or fluorinated alkoxy, each having 1 to 15 C atoms, or unfluorinated alkenyl or fluorinated alkenyl, unfluorinated alkenyloxy or unfluorinated alkoxyalkyl or fluorinated alkoxyalkyl, each having 2 to 15 C atoms, in which, independently of one another, one or more "-$CH_2$-" groups may, in addition, be replaced by cycloalkyl having 3 to 6 C atoms, preferably having 4 or 6 C atoms,
$L^{11}$ to $L^{14}$ on each appearance, in each case independently of one another, denote H, alkyl having 1 to 15 C atoms, F or Cl, and
p denotes an integer in the range from 6 to 15,

where at least two of the substituents $L^{11}$ to $L^{14}$ present have a meaning other than H.

**18.** Compound of the formula I according to Claim 17, **characterised in that** at least two of the substituents

$L^{11}$ to $L^{14}$ present denote alkyl.

**19.** Compound of the formula I according to Claim 17 or 18, **characterised in that**

$R^{11}$ denotes $C_nH_{2n+1}$ or $CH_2=CH-(CH_2)_Z$, and
$R^{12}$ denotes $C_mH_{2m+1}$ or $O-C_mH_{2m+1}$ or $(CH_2)_Z-CH=CH_2$,

in which

n and m, independently of one another, denote an integer in the range from 1 to 7, and
z denotes 0, 1, 2, 3 or 4.

**20.** Process for the preparation of a compound of the formula I according to one or more of Claims 17 to 19, **characterised in that** the product or one or more intermediates from an aryl borate compound are linked to an aryl-halogen compound or to an aryl triflate compound by palladium-catalysed homocoupling and/or cross-coupling.

## Revendications

**1.** Dispositif pour la technologie hautes fréquences ou pour la région des micro-ondes et la région des ondes millimétriques du spectre électromagnétique, **caractérisé en ce qu'**il contient un milieu cristallin liquide qui comprend lui-même un *composant A* qui est constitué lui-même par un ou plusieurs composé(s) de la formule I :

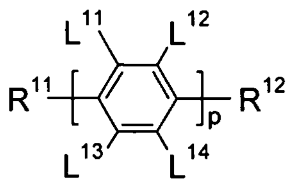

I

dans laquelle :

$R^{11}$ et $R^{12}$ représentent, indépendamment l'un de l'autre, halogène, alkyle non fluoré ou alkyle fluoré, alcoxy non fluoré ou alcoxy fluoré, chacun comportant de 1 à 15 atomes de C, ou alkényle non fluoré ou alkényle fluoré, alkényloxy non fluoré ou alkényloxy fluoré ou alcoxyalkyle non fluoré ou alcoxyalkyle fluoré, chacun comportant de 2 à 15 atomes de C, où, indépendamment les uns des autres, un ou plusieurs groupe(s) "$-CH_2-$" peut/peuvent, en outre, être remplacé(s) par cycloalkyle comportant de 3 à 6 atomes de C, de façon préférable comportant 4 ou 6 atomes de C, et à titre d'alternative, en outre, l'un de $R^{11}$ et $R^{12}$ ou à la fois $R^{11}$ et $R^{12}$ représente(nt) H,
$L^{11}$ à $L^{14}$ représentent, pour chaque occurrence dans chaque cas indépendamment les unes des autres, H, alkyle comportant de 1 à 15 atomes de C, F ou Cl, et
p représente un entier dans la plage de 6 à 15.

**2.** Dispositif selon la revendication 1, **caractérisé en ce qu'**il contient un milieu cristallin liquide qui comprend un *composant B* qui est constitué par un ou plusieurs composé(s) de la formule IV :

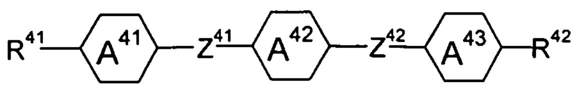

IV

dans laquelle :

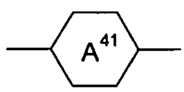
et
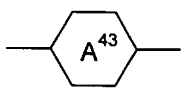

représentent tous deux

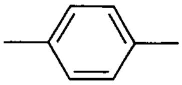

et

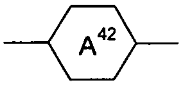 représente 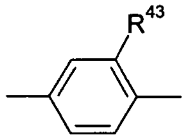

ou un ou plusieurs de

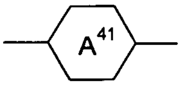 à 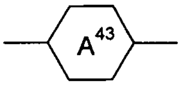

représente(nt)

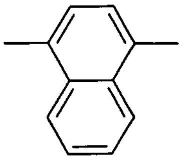

et les autres représentent

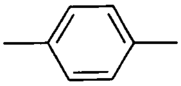,

$R^{41}$ à $R^{43}$ présentent, indépendamment les uns des autres, l'une des significations données pour $R^{11}$ selon la revendication 1, et
$Z^{41}$ et $Z^{42}$ représentent, indépendamment l'un de l'autre, -C≡C-, - CF=CF-, -CF=CH-, -CH=CF- ou -CH=CH-.

3. Dispositif selon l'une des revendications 1 et 2, **caractérisé en ce que** le milieu cristallin liquide, en plus d'un composant A, comprend de façon additionnelle un ou plusieurs composant(s) choisi(s) parmi le groupe des composants qui suivent, à savoir les *composants B* à *E* :

- un composant, soit le *composant B*, lequel présente une anisotropie diélectrique dans la plage qui va de plus de -5,0 à moins de 10,0 et lequel, de façon préférable, est constitué par un ou plusieurs des composés de la formule IV donnés selon la revendication 2,
- un composant fortement diélectriquement positif, soit le *composant C*, lequel présente une anisotropie diélectrique de 10 ou plus,
- un composant fortement diélectriquement négatif, soit le *composant D*, lequel présente une anisotropie diélectrique présentant une valeur de 5 ou plus,
- un composant, soit le *component E,* lequel présente pareillement une anisotropie diélectrique dans la plage de plus de -5,0 à moins de 10,0 et est constitué par des composés comportant jusqu'à cinq cycles à cinq, six ou sept éléments.

4. Dispositif selon la revendication 3, **caractérisé en ce que** le milieu cristallin liquide comprend le *composant C.*

**5.** Dispositif selon l'une des revendications 3 et 4, **caractérisé en ce que** le milieu cristallin liquide comprend le *composant D.*

**6.** Dispositif selon une ou plusieurs des revendications 3 et 4, **caractérisé en ce que** le milieu cristallin liquide comprend le *composant E.*

**7.** Milieu cristallin liquide, **caractérisé en ce qu'**il comprend :

- un *composant A,* lequel est constitué par un ou plusieurs composé(s) de la formule I indiquée selon la revendication 1, et
- de façon additionnelle, un ou plusieurs composant(s) choisi(s) parmi le groupe des composants qui suivent, soit les *composants BàE:*

  - un composant, soit le *composant B,* lequel présente une anisotropie diélectrique dans la plage qui va de plus de -5,0 à moins de 10,0 et lequel est constitué, de façon préférable, par des composés de la formule IV donnée selon la revendication 2,
  - un composant fortement diélectriquement positif, soit le *composant C*, lequel présente une anisotropie diélectrique de 10 ou plus,
  - un composant fortement diélectriquement négatif, soit le *composant D,* lequel présente une anisotropie diélectrique présentant une valeur de 5 ou plus,
  - un composant, soit le *composant E,* lequel présente pareillement une anisotropie diélectrique dans la plage qui va de plus de -5,0 à moins de 10,0 et est constitué par des composés comportant jusqu'à cinq cycles à cinq, six ou sept éléments.

**8.** Milieu cristallin liquide selon la revendication 7, **caractérisé en ce que** le milieu cristallin liquide comprend le *composant B.*

**9.** Milieu cristallin liquide selon une ou plusieurs des revendications 7 et 8, **caractérisé en ce que** le milieu cristallin liquide comprend le *composant C.*

**10.** Milieu cristallin liquide selon une ou plusieurs des revendications 7 et 8, **caractérisé en ce que** le milieu cristallin liquide comprend le *composant D.*

**11.** Utilisation d'un milieu cristallin liquide selon une ou plusieurs des revendications 7 à 10 dans un dispositif pour la technologie hautes fréquences.

**12.** Procédé pour la préparation d'un milieu cristallin liquide, **caractérisé en ce qu'**un ou plusieurs composé(s) de la formule I comme indiqué selon la revendication 1 est (sont) mélangé(s) avec un ou plusieurs autre(s) composé(s) et/ou avec un ou plusieurs additif(s).

**13.** Procédé pour la production d'un dispositif selon une ou plusieurs des revendications 1 à 6, **caractérisé en ce qu'**un milieu cristallin liquide selon une ou plusieurs des revendications 7 à 10 est utilisé.

**14.** Déphaseur, **caractérisé en ce qu'**il comprend un ou plusieurs dispositif(s) selon une ou plusieurs des revendications 1 à 6.

**15.** Antenne réseau microondes, **caractérisée en ce qu'**elle comprend un ou plusieurs dispositif(s) selon une ou plusieurs des revendications 1 à 6.

**16.** Procédé pour accorder une antenne réseau microondes, **caractérisé en ce qu'**un ou plusieurs dispositif(s) selon une ou plusieurs des revendications 1 à 6 est (sont) adressé(s) électriquement ou peut/peuvent être adressé(s) électriquement.

**17.** Composé de la formule I :

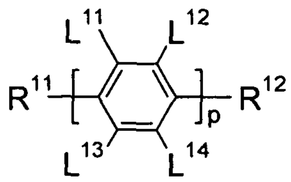

I

dans laquelle :

$R^{11}$ et $R^{12}$ représentent, indépendamment l'un de l'autre, halogène, alkyle non fluoré ou alkyle fluoré, alcoxy non fluoré ou alcoxy fluoré, chacun comportant de 1 à 15 atomes de C, ou alkényle non fluoré ou alkényle fluoré, alkényloxy non fluoré ou alkényloxy fluoré ou alcoxyalkyle non fluoré ou alcoxyalkyle fluoré, chacun comportant de 2 à 15 atomes de C, où, indépendamment les uns des autres, un ou plusieurs groupe(s) "$-CH_2-$" peut/peuvent, en outre, être remplacé(s) par cycloalkyle comportant de 3 à 6 atomes de C, de façon préférable comportant 4 ou 6 atomes de C,
$L^{11}$ à $L^{14}$ représentent, pour chaque occurrence dans chaque cas indépendamment les unes des autres, H, alkyle comportant de 1 à 15 atomes de C, F ou Cl, et
p représente un entier dans la plage de 6 à 15,

où au moins deux des substituants $L^{11}$ à $L^{14}$ présents présentent une signification autre que H.

**18.** Composé de la formule I selon la revendication 17, **caractérisé en ce qu'**au moins deux des substituants

$L^{11}$ à $L^{14}$ présents représentent alkyle.

**19.** Composé de la formule I selon la revendication 17 ou 18, **caractérisé en ce que**:

$R^{11}$ représente $C_nH_{2n+1}$ ou $CH_2=CH-(CH_2)_Z$, et
$R^{12}$ représente $C_mH_{2m+1}$ ou $O-C_mH_{2m+1}$ ou $(CH_2)_Z-CH=CH_2$,

où :

n et m représentent, indépendamment l'un de l'autre, un entier dans la plage de 1 à 7, et
z représente 0, 1, 2, 3 ou 4.

**20.** Procédé pour la préparation d'un composé de la formule I selon une ou plusieurs des revendications 17 à 19, **caractérisé en ce que** le produit ou un ou plusieurs produit(s) intermédiaire(s) dérivé(s) à partir d'un composé de borate d'aryle est/sont lié(s) à un composé aryle-halogène ou à un composé triflate d'aryle par homocouplage et/ou couplage par réticulation catalysé par palladium.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0364185 A2
- DE 102004029429 A
- JP 2005120208 A


### In der Beschreibung aufgeführte Nicht-Patentliteratur


- **SUBRAMANIAM, G. ; GILPIN, R. K.** Mesogenic Properties or 2',3''''-dimethyl-p-sexiphenyl. *MCLC,* 1989, vol. 166, 173-179
- **GAEBLER, A. ; MOESSINGER, A. ; GOELDEN, F. et al.** Liquid Crystal-Reconfigurable Antenna Concepts for Space Applications at Microwave and Millimeter Waves. *International Journal of Antennas and Propagation,* 2009, vol. 2009 (876989), 1-7
- **PENIRSCHKE, A. ; MÜLLER, S. ; SCHEELE, P. ; WEIL, C. ; WITTEK, M. ; HOCK, C. ; JAKOBY, R.** Cavity Perturbation Method for Characterization of Liquid Crystals up to 35 GHz. *34th European Microwave Conference-Amsterdam,* 545-548
- **LARIOS-LÓPEZ, L. ; NAVARRO-RODRIGUEZ, D. ; ARIAS-MARIN, E. M. ; MOGGIO, I. ; REYES-CASTENEDA, C. V.** *Liquid Crystals,* 2003, vol. 30 (4), 423-433
- **BANERJEE M. ; SHUKLA, R. ; RATHORE, R.** *J. Am. Chem. Soc.,* 2009, vol. 131, 1780-1786
- **REHAHN, M. ; GALDA, P.** *Synthesis,* 1996, 614-620
- **WU, S.-T. ; HSU, C.-S. ; SHYU, K.-F.** *Appl. Phys. Lett.,* 1999, vol. 74 (3), 344-346
- **HSU, C. S. ; SHYU, K. F. ; CHUANG, Y. Y. ; WU, S.-T.** *Liq. Cryst.,* 2000, vol. 27 (2), 283-287
- **DABROWSKI, R. ; KULA, P. ; GAUZA, S. ; DZIADISZEK, J. ; URBAN, S. ; WU, S.-T.** *IDRC,* 2008, vol. 08, 35-38
- **A. GAEBLER ; F. GOELDEN ; S. MÜLLER ; A. PENIRSCHKE ; R. JAKOBY.** Direct Simulation of Material Permittivites using an Eigen-Susceptibility Formulation of the Vector Variational Approach. *12MTC 2009-International Instrumentation and Measurement Technology Conference, Singapur,* 2009, 463-467
- Merck Liquid Crystals, Physical Properties of Liquid Crystals. Merck KGaA, November 1997
- **A. PENIRSCHKE ; S. MÜLLER ; P. SCHEELE ; C. WEIL ; M. WITTEK ; C. HOCK ; R. JAKOBY.** Cavity Perturbation Method for Characterization of Liquid Crystals up to 35GHz. *34th European Microwave Conference - Amsterdam,* 545-548
- **A. GAEBLER ; F. GOELDEN ; S. MÜLLER ; A. PENIRSCHKE ; R. JAKOBY.** Direct Simulation of Material Permittivites ... *12MTC 2009 - International Instrumentation and Measurement Technology Conference,* 2009, 463-467
- **A. PENIRSCHKE ; S. MÜLLER ; P. SCHEELE ; C. WEIL ; M. WITTEK ; C. HOCK ; R. JAKOBY.** Cavity Perturbation Method for Characterization of Liquid Crystals up to 35GHz. *34th European Microwave Conference,* 545-548